(19)

Europäisches
Patentamt
European
Patent Office
Office européen
des brevets

(11) **EP 4 309 685 A1**

(12) # EUROPEAN PATENT APPLICATION
published in accordance with Art. 153(4) EPC

(43) Date of publication:
**24.01.2024 Bulletin 2024/04**

(21) Application number: **22771767.5**

(22) Date of filing: **16.03.2022**

(51) International Patent Classification (IPC):
**A61L 27/46** *(2006.01)*     **A61L 27/58** *(2006.01)*
**A61F 2/28** *(2006.01)*      **A61F 2/30** *(2006.01)*

(52) Cooperative Patent Classification (CPC):
**A61F 2/28; A61F 2/30; A61L 27/46; A61L 27/58;
C08J 3/09; C08J 3/21; C08K 3/32; C08L 67/04**

(86) International application number:
**PCT/KR2022/003694**

(87) International publication number:
**WO 2022/197101 (22.09.2022 Gazette 2022/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.03.2021  KR 20210034059**

(71) Applicant: H&Bio Co., Ltd.
**Seongnam-si, Gyeonggi-do 13605 (KR)**

(72) Inventors:
• **SHIM, Jung Hee
Seoul 06003 (KR)**
• **JO, Ha Hyeon
Seongnam-si Gyeonggi-do 13601 (KR)**
• **KIM, Tae Ho
Bucheon-si Gyeonggi-do 14597 (KR)**
• **LEE, Gye Wook
Uiwang-si Gyeonggi-do 16004 (KR)**

(74) Representative: **Schön, Christoph
Dr. Schön, Neymeyr & Partner mbB
Bavariaring 26
80336 München (DE)**

(54) **COMPOSITE OF CALCIUM PHOSPHATE-BASED BIOCERAMIC AND BIODEGRADABLE POLYMER AND MANUFACTURING METHOD THEREFOR**

(57)     The present application relates to a biocompatible composite and a manufacturing method therefor. Since the biocompatible composite is prepared by mixing a dispersion in which calcium phosphate particles are sufficiently dispersed and a biodegradable polymer dispersion, the calcium phosphate particles are more uniformly dispersed in a biodegradable polymer matrix, so that the calcium phosphate particles are uniformly released when composite is applied to the body.

Fig. 3

```
┌─────────────────────────────────────────────┐
│ Preparing separately a second calcium         │
│ phosphate dispersion and a biodegradable       │
│ polymer dispersion (S1000)                     │
└─────────────────────────────────────────────┘
                      │
                      ▼
┌─────────────────────────────────────────────┐
│ Preparing a calcium phosphate-biodegradable    │
│ polymer dispersion by mixing the second        │
│ calcium phosphate dispersion and the           │
│ biodegradable polymer dispersion (S2000)       │
└─────────────────────────────────────────────┘
                      │
                      ▼
┌─────────────────────────────────────────────┐
│ Removing a liquid continuous phase of the      │
│ calcium phosphate-biodegradable polymer        │
│ dispersion to prepare a biocompatible          │
│ composite (S3000)                              │
└─────────────────────────────────────────────┘
```

EP 4 309 685 A1

## Description

### Technical Field

According to the present application, a biocompatible composite and a preparation method of the same are disclosed. More specifically, a biocompatible composite comprising a biodegradable polymer and calcium phosphate particles dispersed on the biodegradable polymer and a preparation method of the same are disclosed.

### Background Art

Calcium phosphate ceramics are currently being widely used as biocompatible inorganic materials. Calcium phosphate ceramics are used as raw materials for products such as dental restorative materials, bone cement, oral compositions, fillers, and artificial bone inserted in vivo for regeneration or treatment of human tissue. Representative examples of calcium phosphate ceramics include whitlockite (WH), hydroxyapatite (Hap, $Ca_{10}(PO_4)_6(OH)_2$), and β-tricalcium phosphate (TCP, $Ca_3(PO_4)_2$).

In addition, calcium phosphate ceramics and biodegradable polymers are used to prepare biocompatible composites so that a certain degree of mechanical strength can be obtained in vivo when applied in vivo.

Specifically, a biocompatible composite is prepared by dispersing calcium phosphate particles in a biodegradable polymer matrix. Representative examples of biodegradable polymers include poly(caprolactone), poly(L-lactic acid), poly(lactic-co-glycolic acid), and the like.

Existing biocompatible composites are prepared by directly mixing calcium phosphate powder with a polymer dispersion. However, in the case of powder-form calcium phosphate particles, aggregation between particles occurs unavoidably. Therefore, existing preparation methods of biocompatible composites further require a grinding process of calcium phosphate powder. Accordingly, the existing preparation methods of biocompatible composites require a longer preparation time due to drying and grinding processes. In addition, even when performing the grinding process, aggregation between calcium phosphate powders is not entirely prevented, which is inefficient. Furthermore, there is a problem in that aggregation between calcium phosphate powders leads to lack of uniformity in the degree of dispersion of calcium phosphate particles in each region in the prepared biocompatible composite.

Therefore, there is a growing need for research on a preparation method of a biocompatible composite, in which the degree of dispersion of calcium phosphate particles in each region in the prepared biocompatible composite is uniform while preparation time is shortened.

### Disclosure

### Technical Problem

According to a preparation method of a biocompatible composite according to one embodiment of the present application, a biocompatible composite in which calcium phosphate particles are dispersed further uniformly in a polymer matrix may be prepared.

In one embodiment of the present application, a biocompatible composite in which calcium phosphate particles are dispersed further uniformly in a polymer matrix is provided.

### Technical Solution

In one embodiment of the present application, a biocompatible composite in which calcium phosphate particles are dispersed further uniformly in a polymer matrix is prepared by sufficiently dispersing calcium phosphate crystals in a dispersion and then mixing the resulting dispersion with a biodegradable polymer dispersion.

### Advantageous Effects

According to one embodiment of the present application, aggregation between calcium phosphate particles can be prevented.

According to one embodiment of the present application, calcium phosphate particles can be uniformly distributed region by region in a biodegradable polymer matrix.

According to one embodiment of the present application, calcium phosphate particles can be contained in a biocompatible composite in a high weight ratio.

According to one embodiment of the present application, a biocompatible composite with varying weight ratios of calcium phosphate can be prepared.

Effects according to one embodiment of the present application are not limited to the effects described above. In addition, effects not mentioned herein will be clearly understood by those skilled in the art from this specification and the accompanying drawings.

**Description of Drawings**

FIG. 1 is a flowchart illustrating a preparation method of an existing calcium phosphate-biodegradable polymer composite;

FIG. 2 shows SEM images of a biocompatible composite prepared according to an existing preparation method;

FIG. 3 is a flowchart illustrating a preparation method of a biocompatible composite according to one embodiment of the present application;

FIG. 4 is a flowchart illustrating a preparation method of a second calcium phosphate dispersion according to one embodiment of the present application;

FIG. 5 is a flowchart illustrating a preparation method of a second calcium phosphate dispersion according to another embodiment of the present application;

FIGS. 6A, 6B, and 6C show SEM images of specimen No. 1 prepared in Preparation Example 1, and FIG. 6D shows an image displaying the portion occupied by calcium atoms in specimen No. 1 prepared in Preparation Example 1;

FIGS. 7A, 7B, and 7C show SEM images of specimen No. 2 prepared in Preparation Example 2, and FIG. 7D shows an image displaying the portion occupied by calcium atoms in the SEM image of specimen No. 2;

FIGS. 8A, 8B, and 8C show SEM images of specimen No. 3 prepared in Preparation Example 3;

FIG. 9 shows images displaying the portions occupied by each atom contained in a biocompatible composite in the SEM image of specimen No. 3;

FIGS. 10A and 10B show SEM images of specimen No. 4 prepared in Preparation Example 4, and FIG. 10C shows images displaying the portions occupied by each atom contained in a biocompatible composite in the SEM image of specimen No. 4;

FIG. 11A shows an SEM image of specimen No. 5 prepared in Preparation Example 5, and FIG. 11B shows an image displaying the portion occupied by calcium atoms in the SEM image of specimen No. 5;

FIG. 12A shows an SEM image of specimen No. 6-1 prepared in Preparation Example 6, FIG. 12B shows an image displaying the portion occupied by calcium atoms in the SEM image of specimen No. 6-1, FIG. 12C shows an SEM image of specimen No. 6-2 prepared in Preparation Example 6, and FIG. 12D shows an image displaying the portion occupied by calcium atoms in the SEM image of specimen No. 6-2;

FIGS. 13A, 13B, and 13C show SEM images of specimen No. 7-1 prepared in Preparation Example 7, and FIG. 13D shows an image displaying the portion occupied by magnesium atoms in the SEM image of specimen No. 7-1;

FIGS. 14A, 14B, and 14C show SEM images of specimen No. 7-2 prepared in Preparation Example 7, and FIG. 14D shows an image displaying the portion occupied by magnesium atoms in the SEM image of specimen No. 8-1;

FIG. 15 shows SEM images of specimens Nos. 8-1 and 8-2 prepared in Preparation Example 8 and images displaying the portions occupied by calcium atoms in the respective SEM images;

FIG. 16 shows SEM images of specimens Nos. 8-3 and 8-4 prepared in Preparation Example 8 and images displaying the portions occupied by calcium atoms in each SEM image;

FIG. 17 illustrates one aspect of defining a cross section to be analyzed using Image J software, for test specimens and control specimens;

FIG. 18 is a view illustrating one aspect of dividing an SEM image corresponding to each cross section into a plurality of regions for quantitative analysis using Image J software of test specimens and control specimens;

FIG. 19 is a table for explaining one aspect of calculating the average and standard deviation of quantitative values measured according to an analysis method using Image J software;

FIG. 20 is a table for explaining another aspect of calculating the average and standard deviation of quantitative values measured according to an analysis method using Image J software;

FIG. 21A shows an SEM image of specimen No. 1 prepared according to Preparation Example 1 and an image displaying the portion occupied by magnesium atoms in the SEM image of specimen No. 1, and FIG. 21B shows an SEM image of comparative specimen No. 1 prepared according to Comparative Example 1 and an image displaying the portion occupied by magnesium atoms in the SEM image of comparative specimen No. 1;

FIG. 22 is a comparison table of quantitative data on specimen No. 1 and quantitative data on comparative specimen No. 1;

FIG. 23 shows graphs comparing quantitative data on specimen No. 1 and quantitative data on comparative specimen No. 1;

FIG. 24 shows SEM images of specimen No. 2 prepared according to Preparation Example 2 and quantitative results obtained by analysis using Image J;

FIG. 25 shows SEM images of comparative specimen No. 2 prepared according to Comparative Example 2 and quantitative results obtained by analysis using Image J;

FIG. 26 is a graph comparing quantitative data on specimen No. 2 and quantitative data on comparative specimen No. 2;

FIG. 27 shows an SEM image (at 500x magnification) of specimen No. 4 prepared according to Preparation Example 4 and images displaying each of the portions occupied by calcium and phosphorus atoms in the SEM image of specimen No. 4;

FIG. 28 shows an SEM image (at 1000x magnification) of specimen No. 4 prepared according to Preparation Example 4 and images displaying each of the portions occupied by calcium and phosphorus atoms in the SEM image of specimen No. 4;

FIG. 29 is a table showing SEM image analysis results of specimen No. 4;

FIG. 30 shows an SEM image (at 500x magnification) of comparative specimen No. 4 prepared according to Comparative Example 4 and images displaying each of the portions occupied by calcium and phosphorus atoms in the SEM image of comparative specimen No. 4;

FIG. 31 shows an SEM image (at 1000x magnification) of comparative specimen No. 4 prepared according to Comparative Example 4 and images displaying each of the portions occupied by calcium and phosphorus atoms in the SEM image of comparative specimen No. 4;

FIG. 32 is a table showing SEM image analysis results of comparative specimen No. 4;

FIG. 33 shows graphs comparing quantitative data on specimen No. 4 and quantitative data on comparative specimen No. 4;

FIG. 34 is a graph comparing quantitative data on specimen No. 7-1, specimen No. 7-2, and comparative specimen No. 1;

FIG. 35 shows SEM images of specimens Nos. 8-1 to 8-3 prepared according to Preparation Example 8 and images displaying the portions occupied by calcium atoms in the respective SEM images;

FIG. 36 shows SEM images of specimen No. 8-1 prepared according to Preparation Example 8 and comparative specimen No. 2, and images displaying the portions occupied by a calcium atom in the respective SEM images;

FIG. 37 shows SEM images of specimen No. 9 prepared according to Preparation Example 9; and

FIG. 38 shows images displaying each of the portions occupied by calcium, phosphorus, and magnesium atoms in the SEM image of specimen No. 9.


**Best Mode**

In one embodiment of the present application, provided is a preparation method of a biocompatible composite.

In one embodiment, provided is a preparation method of a biocompatible composite, the method including:

separately preparing a second calcium phosphate dispersion and a biodegradable polymer solution, in which the biodegradable polymer solution is prepared by dissolving a biodegradable polymer in a solvent;

preparing a calcium phosphate-biodegradable polymer dispersion by mixing the second calcium phosphate dispersion and the biodegradable polymer solution; and

removing a liquid continuous phase of the calcium phosphate-biodegradable polymer dispersion.

Here, in one embodiment, the second calcium phosphate dispersion may be prepared by a method including adding calcium phosphate crystals to a second continuous phase.

Here, in one embodiment, the calcium phosphate crystal may be any one selected from among a whitlockite crystal, a hydroxyapatite crystal, a $\beta$-TCP crystal, or combinations thereof.

Here, in one embodiment, the calcium phosphate crystal may be a whitlockite crystal.

Here, in one embodiment, the calcium phosphate crystal may be a hydroxyapatite crystal.

Here, in one embodiment, the second continuous phase may be selected in consideration of the solubility for the solvent of the biodegradable polymer solution.

Here, in one embodiment, the second calcium phosphate dispersion may be prepared by a method including: adding the calcium phosphate crystals to the second continuous phase; and dispersing the calcium phosphate crystals added to the second continuous phase.

Here, in one embodiment, the dispersing of the calcium phosphate crystals added to the second continuous phase may be performed by one or more methods of sonication and stirring.

Here, in one embodiment, the second calcium phosphate dispersion may be prepared by a method including: preparing a first calcium phosphate dispersion; and preparing the second calcium phosphate dispersion by substituting a continuous phase of the first calcium phosphate dispersion with the second continuous phase.

Here, in one embodiment, the first calcium phosphate dispersion may be prepared by a method including: preparing a calcium ion aqueous solution in which calcium ions are dissolved; and adding phosphate ions to the calcium ion aqueous solution while providing conditions of pH and temperature at which calcium phosphate particles are enabled to be formed for a predetermined time, whereby the first calcium phosphate dispersion in which water serves as a continuous phase and the calcium phosphate particle serves as a dispersed phase is prepared.

Here, in one embodiment, the substituting of the continuous phase of the first calcium phosphate dispersion with the second continuous phase may include:

adding the second continuous phase to the first calcium phosphate dispersion; and removing the water by separating a layer of water, serving as the continuous phase of the first calcium phosphate dispersion, and a layer of the second continuous phase using a centrifuge.

Here, in one embodiment, the second continuous phase may be selected in consideration of the solubility in the solvent of the biodegradable polymer solution.

Here, in one embodiment, the second continuous phase may be ethanol.

Here, in one embodiment, the biodegradable polymer may be at least one of PCL, PLA, and PLGA, or combination thereof.

Here, in one embodiment, the biodegradable polymer may be PCL.

Here, in one embodiment, the solvent may be at least one of chloroform and dichloromethane (DCM).

Here, in one embodiment, the solvent may be chloroform.

Here, in one embodiment, the liquid continuous phase of the calcium phosphate-biodegradable polymer dispersion may contain the second continuous phase of the second calcium phosphate dispersion and the solvent of the polymer solution.

Here, in one embodiment, the removing of the liquid continuous phase of the calcium phosphate-biodegradable polymer dispersion may include drying the calcium phosphate-biodegradable polymer dispersion at a drying temperature.

Here, in one embodiment, the drying temperature may be determined in consideration of whether the biodegradable polymer is degraded and whether the calcium phosphates contained in the calcium phosphate-biodegradable polymer dispersion is precipitated.

Here, in one embodiment, the removing of the liquid continuous phase of the calcium phosphate-biodegradable polymer dispersion may include firstly drying the calcium phosphate-biodegradable polymer dispersion at a first temperature for a first time period; and secondly drying the resulting calcium phosphate-biodegradable polymer dispersion at a second temperature for a second time period.

Here, in one embodiment, the first temperature and the first time period may be determined in consideration of whether the biodegradable polymer is degraded and whether the calcium phosphates contained in the calcium phosphate-biodegradable polymer dispersion is precipitated.

Here, in one embodiment, the first temperature, the first time period, the second temperature, and the second time period may be determined in consideration of whether the biodegradable polymer is degraded and whether the calcium phosphates contained in the biodegradable polymer dispersion is precipitated.

Here, in one embodiment, the preparation method of the biocompatible composite may further include molding a calcium phosphate-biodegradable polymer composite.

In one embodiment of the present application, a biocompatible composite is provided.

In one embodiment, provided is a biocompatible composite comprising the following:

a biodegradable polymer matrix; and
calcium phosphate particles dispersed in the biodegradable polymer matrix with uniformity of a first degree of uniformity.

Here, in one embodiment, the biodegradable polymer matrix may be provided from any one or more of PCL, PLA, PLLA, and PLGA.

Here, in one embodiment, the biodegradable polymer matrix may be provided from PCL.

Here, in one embodiment, the calcium phosphate particle may be any one or combination of two or more selected from among a whitlockite particle, a hydroxyapatite particle, and a beta-tricalcium phosphate particle.

Here, in one embodiment, the calcium phosphate particle may be a whitlockite particle.

Here, in one embodiment, a weight ratio of the calcium phosphate particles to the biocompatible composite may be in a range of 10 wt% or more and 50 wt% or less.

Here, in one embodiment, the first degree of uniformity may be measured by a method including: measuring a plurality of first ratios (%) with respect to a plurality of regions in the biocompatible composite, wherein each of the plurality of first ratios is a ratio of an area occupied by a first atom in each of the plurality of regions, wherein the first atom is any one selected from among calcium, phosphorus, and magnesium atom; calculating a standard deviation of the plurality of first ratios measured from the plurality of regions to obtain a first standard deviation; and calculating the first degree of uniform ity based on the first standard deviation.

Here, in one embodiment, the first atom may be a calcium atom.

Here, in one embodiment, the respective regions may have the same reference area.

Here, in one embodiment, the first degree of uniformity may be calculated by dividing the first standard deviation by an average of the first ratio measured from the plurality of regions.

Here, in one embodiment, the first degree of uniformity may be 1 or less.

In one embodiment, provided is a biocompatible composite comprising the following:

a biodegradable polymer matrix; and

calcium phosphate particles dispersed in the biodegradable polymer matrix.

Here, in a cross section of the biocompatible composite, an average of a ratio of an area occupied by a first atom contained in calcium phosphate particle per unit area is 53% to 85%, and a standard deviation is 0.8 to 8.7.

Here, in one embodiment, the first atom may be any one selected from calcium atom, phosphorus atom, and magnesium atom.

Here, in one embodiment, the first atom may be a calcium atom.

Here, in one embodiment, a weight ratio of the calcium phosphate particles to the biocompatible composite may be 10 wt% or more and 50 wt% or less.

Here, in one embodiment, the biodegradable polymer matrix may be provided from any one or more of PCL, PLA, PLLA, and PLGA.

Here, in one embodiment, the biodegradable polymer matrix may be provided from PCL.

Here, in one embodiment, the calcium phosphate particle may be any one or combination of two or more selected from among a whitlockite particle, a hydroxyapatite particle, and a beta-tricalcium phosphate particle.

Here, in one embodiment, the calcium phosphate particle may be a whitlockite particle.

Here, in one embodiment, the weight ratio of the calcium phosphate particles to the biocompatible composite may be 10 wt% or more and 50 wt% or less.

Here, in one embodiment, the ratio of the area occupied by the first atom contained in the calcium phosphate particle per unit area may be calculated using Image J.

## Mode for Invention

The foregoing objectives, features, and advantages of the present disclosure will become more apparent from the following detailed description taken in conjunction with the accompanying drawings. However, the present disclosure may be variously modified and have various embodiments. Hereinafter, specific embodiments will be illustrated in the drawings and described in detail.

Like reference numerals throughout the specification in principle refer to the same elements. In the following description, like reference numerals are used to designate elements that have the same function within the same idea illustrated in the drawings of each embodiment.

In addition, in the following description of the present disclosure, detailed descriptions of known functions and components incorporated herein will be omitted when it may make the subject matter of the present disclosure unclear. Furthermore, numeral figures (for example, first, second, and the like) used during describing the present specification are just identification symbols for distinguishing one element from another element.

A ceramic used herein includes the meaning of solid inorganic non-metallic materials having a crystalline or partially crystalline structure.

A calcium phosphate ceramic used herein includes the meaning of ceramics containing calcium and phosphorus elements. In addition, the calcium phosphate ceramic used herein includes the meaning of ceramics having a form in which at least a portion of calcium and phosphorus elements are substituted with metal elements other than calcium. For example, the calcium phosphate ceramic may include: hydroxyapatite (Hap, $Ca_{10}(PO_4)_6(OH)_2$) containing calcium and phosphorus elements; beta-tricalcium phosphate (TCP, $Ca_3(PO_4)_2$); a whitlockite (WH) material containing calcium, magnesium, and phosphorus elements; and the like. However, the above descriptions are disclosed only for illustrative purposes, and the calcium phosphate ceramic used herein includes the meaning of all types of ceramics containing calcium and phosphorus elements.

As used herein, X element means X atom, and "including X element" may be used in the same sense as "including X atom". For example, "including Ca element" may be used in the same sense as "including Ca atom".

As used herein, a biodegradable polymer means a polymer that is a generic term for polymeric materials in which a compound having a high molecular weight is modified into a compound having a low molecular weight because the metabolism of a living organism (for example, enzymatic degradation or hydrolysis) is involved in at least one process of degradation. For example, the biodegradable polymer may include polymeric materials such as polycaprolactone (PCL), polylactide (PLA), poly(D,L-lactic-co-glycolic acid) (PLGA), and the like, which are degradable with the involvement of in vivo metabolism of living organisms. However, the above descriptions are disclosed only for illustrative purposes, and the biodegradable polymer includes any suitable polymers that are degradable with the involvement of in vivo metabolism of living organisms.

A dispersion used herein means a generic term for all types of mixtures having a form in which a discontinuous phase (dispersed phase) is dispersed in a continuous phase. In other words, the dispersion used herein includes the meaning of a mixture containing a dispersed phase and a continuous phase.

As used herein, a solution, which means any type of mixture having a form in which two or more materials are homogeneously present, refers to a mixture in which a first material and a second material are practically homogeneously distributed in the mixture, regardless of whether each "phase" of the two or more materials is a gas, liquid, or solid.

However, in the case where a plurality of materials is present, the solution and the dispersion may not be clearly distinguishable. Therefore, the solution and the dispersion used herein may be interchangeable.

The term "about" used herein means an amount, temperature, level, value, number, frequency, percentage, dimension, size, amount, weight, or length that varies by the degree of 30%, 25%, 20%, 25%, 10%, 9%, 8%, 7%, 6%, 5%, 4%, 3%, 2%, or 1%, with respect to a reference amount, temperature, level, value, number, frequency, percentage, dimension, size, amount, weight, or length.

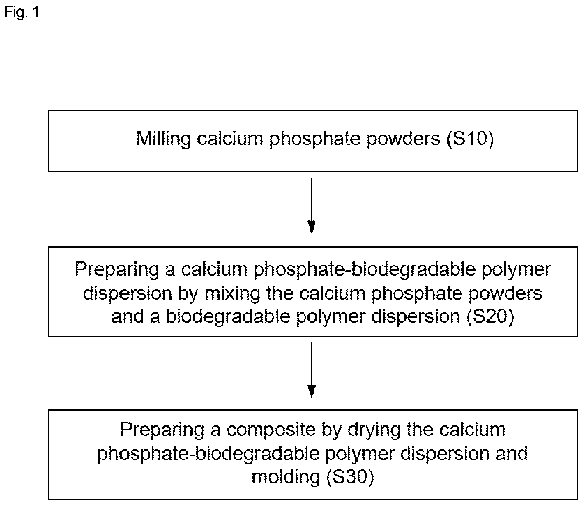

FIG. 1 is a flowchart illustrating a preparation method of an existing calcium phosphate-biodegradable polymer composite. The existing preparation method of the calcium phosphate-biodegradable polymer bone graft composite includes: step S10 of grinding calcium phosphate powder, step S20 of preparing a calcium phosphate-biodegradable polymer dispersion by mixing the calcium phosphate powder and a biodegradable polymer dispersion, and step S30 of preparing a calcium phosphate-biodegradable polymer composite by drying and molding the calcium phosphate-biodegradable polymer dispersion. In particular, in the existing preparation method of the calcium phosphate-biodegradable polymer composite, the calcium phosphate powder is subjected to grinding step S10 and then mixed with the biodegradable polymer dispersion.

The powder used herein includes the meaning of solid-phase fine particles.

Typically, a force with which powders aggregate to each other results in aggregation between the powders. In particular, such a tendency appears more dominantly in the case of particles in nanometer (nm) units. In the existing preparation method of the calcium phosphate-biodegradable polymer composite, the calcium phosphate powder and the biodegradable polymer dispersion are mixed. Thus, the preparation process of the calcium phosphate-biodegradable polymer composite inevitably involves a moment when the calcium phosphate particles are present in the powderform. Therefore, according to the existing preparation method of the composite, aggregation between calcium phosphate powders inevitably occurs.

For this reason, the existing preparation method of the composite includes step S10 of the grinding the aggregated calcium phosphate powders. For example, an operation of classifying the calcium phosphate powders having a predetermined or smaller size by filtering after grinding the calcium phosphate powders using a ball milling method may be repeatedly performed.

Nevertheless, the problem of aggregation between the calcium phosphate particles still exists in the existing preparation method of the composite.

Specifically, even when grinding the aggregated calcium phosphate powders, the calcium phosphate is maintained to be present in the powder form, so aggregation between the ground and filtered calcium phosphate powders occurs again. That is to say, aggregation occurs between the calcium phosphate powders even after the grinding. Therefore, the existing preparation method of the composite is inefficient because aggregation between calcium phosphate particles continues to occur as long as the calcium phosphate particles are present in the powder form. In addition, step S10 may be repeatedly performed multiple times depending on the degree of aggregation between the calcium phosphate particles, so there is a problem of being time-consuming.

In addition, according to the existing preparation method of the composite, aggregation between the calcium phosphate powders leads to lack of uniformity in the size of the ceramic material. Specifically, aggregation between the calcium phosphate powders occurs randomly between the calcium phosphate particles, leading to lack of uniformity in the particle size of the ceramic material, with the occurrence of aggregation.

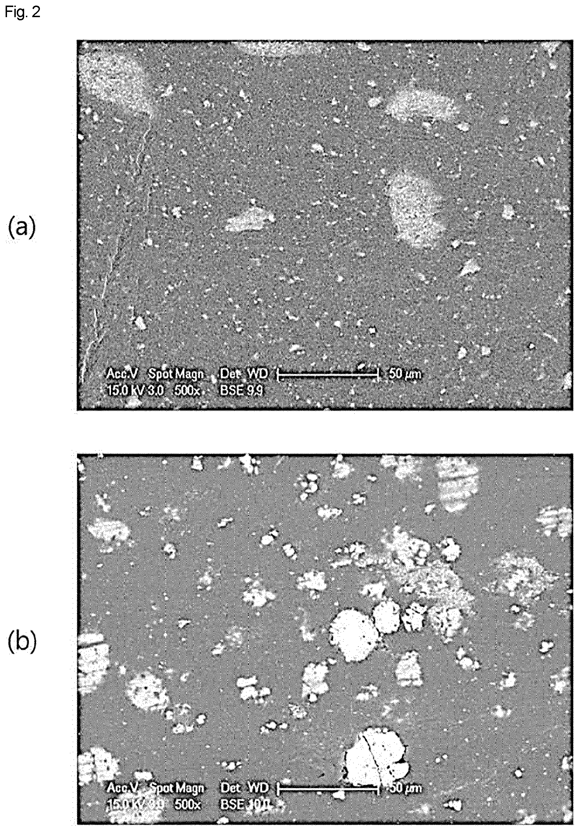

Referring to FIG. 2, FIG. 2 shows SEM images of a biocompatible composite prepared according to an existing preparation method.

The lack of uniformity in the particle size of the ceramic material may lead to lack of uniformity in the distribution of the ceramic material in the biocompatible composite, as shown in FIG. 2.

For example, FIG. 2A shows SEM data on a biocompatible composite prepared by the existing preparation method in which whitlockite powder is mixed with a PCL polymer dispersion. Referring to FIG. 2A, it is visually confirmed that whitlockite particles are non-uniformly distributed in the PCL polymer matrix.

For example, FIG. 2B shows SEM data on a biocompatible composite prepared by the existing preparation method in which hydroxyapatite powder is mixed with a PCL polymer dispersion. Referring to FIG. 2B, it is visually confirmed that hydroxyapatite particles are non-uniformly distributed in the PCL polymer matrix.

Therefore, to solve the problems occurring in the composite preparation method of the composite described above, in a preparation method of a biocompatible composite according to one embodiment of the present application, calcium phosphate is dispersed in a continuous phase in an environment free of a biodegradable polymer. Then, the resulting calcium phosphate dispersion is mixed with a biodegradable polymer dispersion. In other words, in the preparation method of the biocompatible composite according to one embodiment of the present application, the dispersion having a form in which the calcium phosphate particles are dispersed in a continuous liquid phase is mixed with the biodegradable polymer dispersion (or biodegradable polymer solution).

However, there are additional matters to be taken into account when mixing the calcium phosphate dispersion with the biodegradable polymer dispersion (or biodegradable polymer solution) while maintaining the dispersion form. For example, the calcium phosphate dispersion may contain a first liquid continuous phase in which the calcium phosphate particles are dispersed. In addition, the biodegradable polymer dispersion (or biodegradable polymer solution) may contain a second liquid continuous phase different from the first liquid continuous phase. Here, when mixing the calcium phosphate dispersion containing the first liquid continuous phase with the biodegradable polymer dispersion (or biodegradable polymer solution) containing the second liquid continuous phase (or solvent), the resulting mixture may be separated into a plurality of phases depending on a difference in chemical properties (for example, polarity and non - polarity) of the first and second liquid continuous phases (or solvents), or solubility between the first and second liquid continuous phases (or solvents). In this case, the calcium phosphate particles may be heterogeneously dispersed in the plurality of separated phases, where aggregation between the calcium phosphate particles may occur more severely. Therefore, there is a need for appropriately selecting the first liquid continuous phase, in which the calcium phosphate is dispersed, and the second liquid continuous phase (or solvent used for preparing the biodegradable polymer solution), in which the biodegradable polymer dispersion is dispersed, in consideration of the relation between the calcium phosphate, the biodegradable polymer dispersion, the first liquid continuous phase, and/or the second liquid continuous phase (or solvent).

Hereinafter, a biocompatible composite and a preparation method of the same, according to one embodiment of the present application, will be described in detail.


**1. Preparation method of biocompatible composite**

Hereinafter, a preparation method of a biocompatible composite, according to one embodiment of the present application, will be disclosed in more detail.

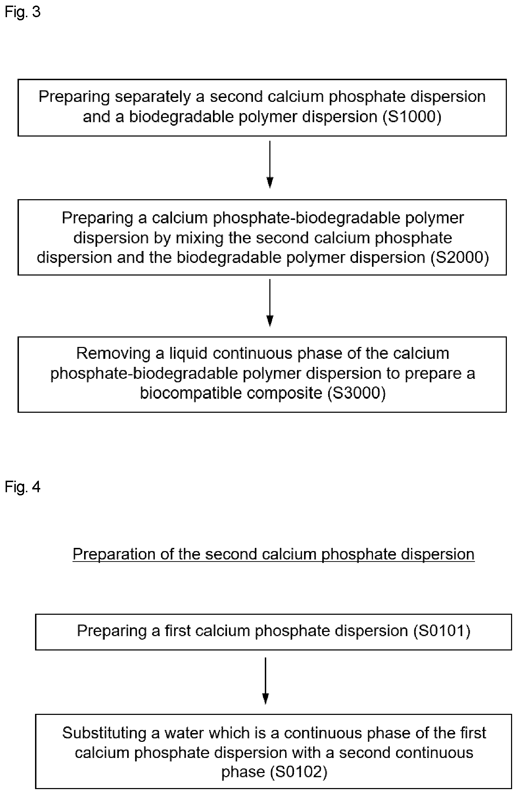

Referring to FIG. 3, FIG. 3 is a flowchart illustrating a preparation method of a biocompatible composite according to one embodiment disclosed herein.

The preparation method of the biocompatible composite of the present application may include:

preparing a second calcium phosphate dispersion and a biodegradable polymer dispersion (or biodegradable polymer solution) respectively (S1000); preparing a calcium phosphate-biodegradable polymer dispersion by mixing the second calcium phosphate dispersion and the biodegradable polymer dispersion (or biodegradable polymer solution) (S2000); and removing a liquid continuous phase of the calcium phosphate-biodegradable polymer dispersion to prepare a biocompatible composite (S3000).

In one embodiment, the preparation method of the biocompatible composite may further include molding the biocompatible composite after preparing the biocompatible composite (S4000).

According to the preparation method of the biocompatible composite of the present application, the biocompatible composite in which the calcium phosphate is further uniformly distributed may be prepared. Such an effect may be achieved by the following feature of the preparation method of the biocompatible composite of the present application: Before the mixing of the calcium phosphate and the biodegradable polymer, calcium phosphate crystals are sufficiently dispersed in an appropriate continuous phase or solvent in an environment free of the biodegradable polymer. Then, the calcium phosphate crystals in the continuous phase or solvent are mixed with the biodegradable polymer.

That is to say, the present disclosure is characterized by sufficiently dispersing the calcium phosphate crystals in the appropriate continuous phase or solvent in the environment free of the biodegradable polymer before the calcium phosphate crystals come into contact with the biodegradable polymer, and then bringing the calcium phosphate crystals into contact with the biodegradable polymer by a suitable method.

For example, the biodegradable polymer may be added to the calcium phosphate dispersion containing the sufficiently dispersed calcium phosphate crystals to achieve the feature of the present method.

For a more preferred example, the calcium phosphate dispersion containing the sufficiently dispersed calcium phosphate crystals and the biodegradable polymer dispersion (or biodegradable polymer solution) containing the biodegradable polymer may be separately prepared. Then, the calcium phosphate dispersion and the biodegradable polymer dispersion (or biodegradable polymer solution) may be mixed to achieve the feature of the present disclosure. This may be a further preferable preparation method of the biocompatible composite in which the calcium phosphate is further uniformly distributed because the calcium phosphate crystals and the biodegradable polymer are brought into contact after dispersing not only the calcium phosphate crystals but also the biodegradable polymer in the respective dispersions.

According to the preparation method of the biocompatible composite provided by the present application, the calcium phosphate particles are mixed with the biodegradable polymer dispersion or biodegradable polymer solution (hereinafter, the biodegradable polymer dispersion or biodegradable polymer solution is referred to as a biodegradable polymer dispersion or the like) while maintaining to be present in the dispersion form, not in the "powder" form. In this case, the interaction between the calcium phosphate particles and the continuous liquid phase exists in addition to the interaction between the calcium phosphate particles. Due to the additional interaction between the calcium phosphate particles and the continuous liquid phase, the force with which the calcium phosphate powders aggregate to each other is suppressed, thereby preventing aggregation between calcium phosphate particles from occurring.

In addition, aggregation between the calcium phosphate particles may occur less, so the size of the calcium phosphate particles may be minutely maintained. Accordingly, the calcium phosphate particles are less affected by gravity based on the size of the calcium phosphate particles when being dispersed in the biodegradable polymer. Therefore, a phenomenon in which the calcium phosphate particles gravitate in the polymer may occur less.

In addition, aggregation between the calcium phosphate particles may be reduced, so the composite may be prepared such that the calcium phosphate particles, dispersed in the biodegradable polymer, are uniformly distributed in each region. Specifically, aggregation between the calcium phosphate powders may inevitably and randomly occur in any particles. According to the preparation method of the biocompatible composite according to one embodiment, aggregation between the calcium phosphate particles may be prevented. For this reason, the calcium phosphate particles may be dispersed in the biodegradable polymer while regularly maintaining the size distribution of the calcium phosphate particles. Therefore, the composite in which the calcium phosphate particles, dispersed in the biodegradable polymer, are uniformly distributed in each region may be prepared.

Furthermore, as the calcium phosphate particles are dispersed in the biodegradable polymer while uniformly maintaining the size distribution thereof, the composite with consistent quality, such as mechanical strength or bioactivity, may be prepared.

Furthermore, the composite may be prepared such that the calcium phosphate particles, dispersed in the biodegradable polymer, are uniformly distributed in each region. Thus, when the composite is applied in vivo, the degree to which the calcium phosphate particles are released may be uniform. This may further facilitate control of the properties of the composite so that the calcium phosphate particles are released to a target location in a desired amount.

Hereinafter, each step of the preparation method of the biocompatible composite is specifically disclosed.


**1.1. Step S1000 of preparing each of second calcium phosphate dispersion and biodegradable polymer dispersion (or biodegradable polymer solution)**

As described above, the preparation method of the biocompatible composite of the present application may include step of separately preparing the second calcium phosphate dispersion and the biodegradable polymer dispersion (or biodegradable polymer solution) (S1000). In this case, the second calcium phosphate dispersion and the biodegradable polymer dispersion (or biodegradable polymer solution) may be prepared in the respective containers.

Step S1000 includes preparing the second calcium phosphate dispersion.

Hereinafter, the step of preparing the second calcium phosphate dispersion is specifically disclosed.


*Preparation of second calcium phosphate dispersion*

In one embodiment, the second calcium phosphate dispersion may be (1) prepared from a first calcium phosphate dispersion (where a continuous phase of the first calcium phosphate dispersion is water).

In another embodiment, the first calcium phosphate dispersion may be (2) prepared by dispersing the calcium phosphate crystals in the liquid continuous phase.

In one embodiment, the second calcium phosphate dispersion may be a dispersion in which the calcium phosphate crystals are dispersed in a second continuous phase.

In one embodiment, the calcium phosphate crystal may be any one or combination of two or more selected from among a hydroxyapatite crystal, a whitlockite crystal, and a beta-tricalcium phosphate crystal.

In one embodiment, the second continuous phase may be selected on the basis of the following criteria but is not limited thereto: solubility in or affinity for the liquid continuous phase (or solvent) of the biodegradable polymer dispersion or the like to be described later, properties of the biodegradable polymer of the biodegradable polymer dispersion or the like, and/or the properties of the calcium phosphate crystals. In a specific embodiment, the second continuous phase may be ethanol, but not limited thereto, and an appropriate material may be selected in consideration of the criteria described above. The specific embodiment regarding the second continuous phase is disclosed in detail in the relevant paragraphs.

Each embodiment of preparing the second calcium phosphate dispersion is specifically disclosed below.

*(1) Prepared from first calcium phosphate dispersion*

In one embodiment, the second calcium phosphate dispersion may be prepared from the first calcium phosphate dispersion in which water serves as the continuous phase. More specifically, the second calcium phosphate dispersion may be prepared by replacing the water, serving as the continuous phase of the first calcium phosphate dispersion, with the second continuous phase.

In other words, in one embodiment, the second calcium phosphate dispersion may be prepared by a method including the following:

preparing the first calcium phosphate dispersion (S0101), and
substituting the water which is the continuous phase of the first calcium phosphate dispersion with the second continuous phase (S0102).

A flowchart illustrating one embodiment of the second calcium phosphate dispersion is disclosed in FIG. 4.

*Preparation of first calcium phosphate dispersion*

In one embodiment, the first calcium phosphate dispersion may be prepared by a method including the following: preparing a calcium ion aqueous solution in which calcium ions are dissolved; and adding phosphate ions to the calcium ion aqueous solution while providing conditions of pH and temperature capable of producing the calcium phosphate particles for a predetermined time period, whereby the first calcium phosphate dispersion (also referred to as dispersion) in which water is the continuous phase and the calcium phosphate particles is the dispersed phase is prepared.

For example, the first calcium phosphate dispersion may be a first whitlockite dispersion. In this case, the first whitlockite dispersion may be prepared by a method including: preparing a cationic aqueous solution in which calcium and magnesium ions are dissolved, and adding phosphate ions to the cationic aqueous solution while providing conditions of pH, temperature, and time disclosed in the preparation method of whitlockite disclosed in Korean Patent Nos. 2084896 and 2116206.

In the Korean Patent No. 2084896, a method for preparing whitlockite is disclosed, which is characterized in that a step of reacting calcium ions and phosphate ions and a step of reacting phosphate ions and cations other than calcium ions (for example, magnesium ions) are separated in time.

In the Korean Patent No. 2116206, a method for preparing whitlockite is disclosed, which is characterized in that a step of reacting calcium ions and phosphate ions and a step of reacting phosphate ions and cations other than calcium ions (for example, magnesium ions) are spatially separated.

However, the preparation methods of the first whitlockite dispersion described above are disclosed only for illustrative purposes. The first whitlockite dispersion may be prepared by various preparation methods, including dispersing a whitlockite solid crystals in water, produced by precipitating whitlockite particles in an aqueous solution.

For another example, the first calcium phosphate dispersion may be a first hydroxyapatite dispersion.

In this case, the first hydroxyapatite dispersion may be prepared by a method including: preparing a cationic aqueous solution in which calcium ions are dissolved, and adding phosphate ions to the cationic aqueous solution while providing conditions of pH and temperature at which hydroxyapatite particles are enabled to be formed.

For example, when aging an aqueous solution containing calcium ions and phosphate ions in a neutral condition and a basic condition, hydroxyapatite crystals are known to be prepared. Therefore, the first hydroxyapatite crystal may be prepared by adding the phosphate ions to the calcium ion aqueous solution while providing conditions of pH corresponding to neutrality and basicity.

Alternatively, any materials (for example, Urea, Na(OH), and the like) suitable for providing an appropriate pH may be additionally mixed.

However, the preparation method of the first hydroxyapatite dispersion described above is disclosed only for illustrative purposes. The first hydroxyapatite dispersions may be prepared using various preparation methods, including dispersing hydroxyapatite solid crystals prepared by precipitating hydroxyapatite particles in an aqueous solution, in water.

In one embodiment, the calcium phosphate contained in the first calcium phosphate dispersion may be any one selected from among hydroxyapatite (HAp), whitlockite (WH), $\beta$-tricalcium phosphate ($\beta$-TCP, $\beta$-Ca$_3$(PO$_4$)$_2$), or combinations thereof. According to the foregoing, although the preparation methods of the whitlockite dispersion and the hydroxyapatite dispersion have been mainly described, it is disclosed that a first $\beta$-TCP dispersion or the like may be prepared by any suitable preparation method of the calcium phosphate dispersion.

In addition, the calcium phosphate described above is disclosed only for illustrative purposes, and the first calcium phosphate dispersion containing any calcium phosphate ceramic may be prepared depending on the properties of the composite without being limited thereto.

*Substituting water, serving as continuous phase of first calcium phosphate dispersion, with second continuous phase*

As described above, in one embodiment, the water, serving as the continuous phase of the first calcium phosphate dispersion, may be replaced with the second continuous phase to prepare the second calcium phosphate dispersion.

Here, the meaning of substitution used herein may include the meaning that the liquid continuous phase is changed such that a continuous phase of the calcium phosphate dispersion is a first material at a first time point and a liquid continuous phase of the calcium phosphate dispersion is a second material at a second time point different from the first time point.

In this case, when mixing the first calcium phosphate dispersion with the biodegradable polymer dispersion or the like to be described later without substituting the water contained in the first calcium phosphate dispersion, phase separation may occur, which causes the calcium phosphate particles to be non-uniformly distributed within the biodegradable polymer matrix. Specifically, when the water of the first calcium phosphate dispersion is mixed with a biodegradable polymer dispersion to be described later without being substituted with an appropriate liquid continuous phase, the liquid continuous phases of the biodegradable polymer, the calcium phosphate particles, water, and the biodegradable polymer (or solvents) may coexist in some cases. In such cases, with a plurality of materials coexisting, aggregation between the calcium phosphate particles is likely to occur by being separated into a plurality of phases. In addition, the calcium phosphate particles may be non-uniformly distributed in the biodegradable polymer matrix because the calcium phosphate particles become irregular due to the aggregation. To avoid the problems described above, a process of substituting the water of the first calcium phosphate dispersion with an appropriate liquid continuous phase is required.

In this case, selection of the appropriate liquid continuous phase to be contained in the second calcium phosphate dispersion so that phase separation is prevented in the calcium phosphate-biodegradable polymer dispersion to be described later is an important factor in the quality of the biocompatible composite to be prepared. Selection of the appropriate second continuous phase may be one of the important factors in the method of the present disclosure, which improves the dispersion stability of the calcium phosphate particles in the biodegradable polymer matrix, thereby allowing the calcium phosphate particles to be uniformly distributed in the biocompatible composite.

The second continuous phase may be selected through various criteria to be described later.

For example, the second continuous phase with which the water of the first calcium phosphate dispersion is replaced may be selected in consideration of solubility in the liquid continuous phase (or solvent) of the biodegradable polymer dispersion or the like to be described later.

For example, when the solvent of the biodegradable polymer dispersion or the like is chloroform or DCM, a material having a relatively higher solubility in chloroform or DCM than water may be selected as a temporary continuous phase. For example, ethanol exhibits a relatively higher solubility in chloroform or DCM than water. Therefore, when the solvent of the biodegradable polymer dispersion or the like is chloroform or DCM, ethanol characterized by having high solubility in the solvent of the biodegradable polymer dispersion or the like may be selected as the second continuous phase of the second calcium phosphate dispersion.

Furthermore, the second continuous phase may be selected in consideration of various properties, including not only solubility but also polarity and non-polarity of the liquid continuous phase (or solvent) of the biodegradable polymer dispersion or the like.

For another example, the second continuous phase with which the water of the first calcium phosphate dispersion is replaced may be selected in consideration of the properties of the biodegradable polymer (for example, the melting point of the biodegradable polymer or the like) of the biodegradable polymer dispersion or the like to be described later.

For example, a liquid having an evaporation temperature higher than the melting point of the biodegradable polymer may be selected as the second continuous phase. Through this, the calcium phosphate-biodegradable polymer dispersion containing the liquid continuous phase having an evaporation temperature higher than the melting point of the biodegradable polymer may be prepared, thereby softening or partially me lting the biodegradable polymer using the liquid continuous phase as a heat medium. This may facilitate the calcium phosphate-biodegradable polymer dispersion to be well-mixed, thereby preparing the composite in which the calcium phosphate particles are dispersed uniformly in the biodegradable polymer.

For example, when the biodegradable polymer contained in the biodegradable polymer dispersion or the like is a polymer having a melting point in a range of about 55°C to 60°C (for example, a PCL polymer), ethanol having an evaporation temperature higher than the melting point of the polymer may be selected as the second continuous phase contained in the second calcium phosphate dispersion.

For another example, the same material as the liquid continuous phase (or solvent) of the biodegradable polymer dispersion or the like to be described later may be selected as the second continuous phase with which the water of the first calcium phosphate dispersion is substituted. For example, when the liquid continuous phase (or solvent) of the biodegradable polymer dispersion or the like is ethanol, the same liquid as the liquid continuous phase (or solvent) of the biodegradable polymer may be selected as the second continuous phase to reduce the possibility of phase separation occurring with a plurality of materials coexisting in step S2000 to be described later.

However, the above description is disclosed only for illustrative purposes, and any suitable material for improving the dispersion stability of calcium phosphate in the biodegradable polymer matrix may be selected as the second continuous phase in consideration of the criteria described above.

On the other hand, the step of preparing the second calcium phosphate dispersion by substituting the water of the first calcium phosphate dispersion with the second continuous phase may further include a step of removing at least a portion of the second continuous phase contained in the second calcium phosphate dispersion. However, the step of removing at least a portion of the second continuous phase contained in the second calcium phosphate dispersion is clearly different from preparing the completely dried calcium phosphate particles in powder form by removing at least a portion of the second continuous phase. In other words, even when the step of removing at least a portion of the second continuous phase is included, the second calcium phosphate dispersion being mixed with the biodegradable polymer dispersion is not present in the powder form but in the dispersion form.

As described above, the characteristics of the method of the present disclosure may be achieved by sufficiently dispersing the calcium phosphate (or calcium phosphate crystals) in the appropriate continuous phase or solvent in an environment free of the biodegradable polymer. Hereinafter, another embodiment of preparing the second calcium phosphate dispersion is to be disclosed.

(2) *Preparing by dispersing calcium phosphate crystals in liquid continuous phase*

In another embodiment, the second calcium phosphate dispersion may be prepared by dispersing the calcium phosphate particles in the second continuous phase.

That is, in one embodiment, preparing the second calcium phosphate dispersion may include:
adding the calcium phosphate crystals to the second continuous phase (S0201).

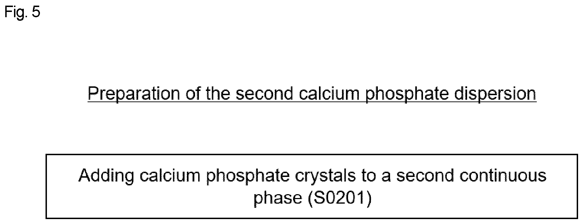

A flowchart illustrating one embodiment of the second calcium phosphate dispersion is disclosed in FIG. 5.

That is, the second calcium phosphate dispersion may be prepared by adding the calcium phosphate crystals to the second continuous phase. For example, the second calcium phosphate dispersion may be prepared by adding the powder-form calcium phosphate crystals to the second continuous phase. In this case, the calcium phosphate crystal added to the second continuous phase may be any one selected from among a hydroxyapatite crystal, a whitlockite crystal, a β-tricalcium phosphate crystal, or combinations thereof. In a specific embodiment, the calcium phosphate crystal may be a whitlockite or hydroxyapatite crystal.

In one embodiment, the second calcium phosphate dispersion may contain the second continuous phase and the calcium phosphate. In this case, the calcium phosphate contained in the second calcium phosphate dispersion may be any one selected from among hydroxyapatite, whitlockite, β-tricalcium phosphate, or combinations thereof. In a specific embodiment, the calcium phosphate contained in the second calcium phosphate dispersion may be hydroxyapatite or whitlockite.

The second continuous phase contained in the second calcium phosphate dispersion is as described above. For example, as described above, the second continuous phase may be selected through various criteria described above.

For example, the second continuous phase may be selected in consideration of solubility in or affinity for the liquid continuous phase (or solvent) of the biodegradable polymer dispersion or the like to be described later.

For another example, the second continuous phase may be selected in consideration of the properties of the biodegradable polymer of the biodegradable polymer dispersion or the like to be described later.

For another example, the second continuous phase may be selected in consideration of properties of the added calcium phosphate crystals, (for example, the relation between the calcium phosphate crystals and the second continuous phase).

For another example, the same material as the liquid continuous phase (or solvent) of the biodegradable polymer dispersion or the like to be described later may be selected as the second continuous phase.

In a specific embodiment, the second continuous phase may be ethanol, including but not limited thereto, and any suitable material for improving the dispersion stability of the calcium phosphate in the biodegradable polymer matrix may be selected as the second continuous phase in consideration of the criteria described above.

In one embodiment, preparing the second calcium phosphate dispersion may further include dispersing the added calcium phosphate crystals in addition to the adding of the calcium phosphate crystals to the second continuous phase.

The step of dispersing the added calcium phosphate crystals means a process of sufficiently dispersing the calcium phosphate (or calcium phosphate crystals) in the second continuous phase. In this case, a method such as sonication and/or stirring may be used to disperse the calcium phosphate in the second continuous phase, including but not limited thereto. Any method commonly used in the art, which allows a material to be dispersed in a solvent (for example, adding a suitable dispersant to the second calcium phosphate dispersion or the like) may be used.

*Preparation of biodegradable polymer dispersion (or biodegradable polymer solution)*

Step S1000 includes a step of preparing the biodegradable polymer dispersion (or biodegradable polymer solution). The biodegradable polymer dispersion may contain the biodegradable polymer and the liquid continuous phase. Alternatively, the biodegradable polymer solution may be prepared by dissolving the biodegradable polymer in a solvent.

As used herein, the solvent contained in the biodegradable polymer solution (that is, the biodegradable polymer dispersion or the like) means a solvent used to prepare the biodegradable polymer solution. In addition, the biodegradable polymer contained in the biodegradable polymer solution (that is, the biodegradable polymer dispersion or the like) means a biodegradable polymer used to prepare the biodegradable polymer solution. For example, when the biodegradable polymer dispersion or the like is prepared by dissolving the PCL polymer in a chloroform solvent, the solvent contained in the biodegradable polymer solution is chloroform, and the biodegradable polymer contained in the biodegradable polymer solution is the PCL polymer. In addition, the solvent used to prepare the biodegradable polymer dispersion or the like may be referred to as the liquid continuous phase of the biodegradable polymer dispersion or the like. For example, when using chloroform to prepare the biodegradable polymer dispersion or the like, the continuous phase of the biodegradable polymer dispersion or the like is chloroform.

For example, the biodegradable polymer of the biodegradable polymer dispersion or biodegradable polymer solution (hereinafter referred to as biodegradable polymer dispersion or the like) may be polycaprolactone (hereinafter referred to as PCL), poly(lactic-co-glycolic acid (hereinafter referred to as PLGA), polylactic acid (hereinafter referred to as PLLA), or combinations thereof. However, these examples are disclosed only for illustrative purposes, and the biodegradable polymer dispersion or biodegradable polymer solution may be prepared using various biodegradable polymers.

The solvent or liquid continuous phase of the biodegradable polymer dispersion or the like may be selected in consideration of the solubility in the biodegradable polymer. In one embodiment, as the solvent or liquid continuous phase of the biodegradable polymer dispersion, chloroform, dichloromethane (DCM), dimethylformamide (DMF), ethanol, or combinations thereof may be used, including but not limited thereto. The liquid continuous phase of the biodegradable polymer dispersion may be suitably selected in consideration of the relation with the biodegradable polymer (for example, solubility, affinity, or the like), and/or the relation with the calcium phosphate particles contained in the calcium phosphate-biodegradable polymer dispersion to be described later or the like.

For example, when using the PCL polymer as the biodegradable polymer, chloroform or dichloromethane (DCM), which has a relatively high solubility in the PCL polymer, may be used as the solvent or liquid continuous phase of the PCL polymer, enabling the biodegradable polymer dispersion or biodegradable polymer solution to be prepared.

However, these examples are disclosed only for illustrative purposes, and dimethylformamide (DMF), which may be advantageous in dispersing the calcium phosphate particles contained in the calcium phosphate-biodegradable polymer dispersion to be described later, may be used as the solvent or liquid continuous phase of the biodegradable polymer.

Alternatively, the solvent or liquid continuous phase of the biodegradable polymer dispersion may be selected in consideration of the potential thereof to serve as the heat medium for the biodegradable polymer.

For example, when using the PCL polymer as the biodegradable polymer, ethanol or chloroform capable of serving as a medium for transferring heat to the PCL polymer may be selected as the solvent or liquid continuous phase of the biodegradable polymer dispersion or the like.

For example, the PCL polymer has a melting point in a range of 55°C to 60°C. In this case, ethanol has an evaporation temperature of about 78°C, so by using ethanol as the liquid continuous phase, ethanol may serve as the heat medium for the PCL polymer. In this case, with the heat transferred through ethanol, the PCL polymer dispersion or the like in which the PCL polymer is melted or partially melted may be prepared. Alternatively, the heat transferred through ethanol may help the PCL polymer dissolve in ethanol, thereby preparing the PCL polymer dispersion or the like in which the PCL polymer is dissolved. Here, the term partially melted means that while a portion of the PCL polymer in ethanol is melted, the remainder is not melted.

For another example, the PCL polymer is characterized by being well-soluble in organic solvents. Therefore, chloroform, one of the organic solvents, may be selected as the liquid continuous phase. In addition, like ethanol described above, chloroform has an evaporation temperature of about 61.2°C, which is higher than the melting point of PCL (in a range of about 55°C to 60°C). For this reason, by using chloroform as the liquid continuous phase, chloroform may serve as the heat medium for the PCL polymer. In addition, with the heat transferred through chloroform, a PCL polymer dispersion in which the PCL polymer is melted may be prepared. Alternatively, the heat transferred through chloroform may help the dissolution of the PCL polymer in chloroform, thereby preparing a PCL polymer dispersion or the like in which the PCL polymer is dissolved.

Alternatively, the solvent or liquid continuous phase of the biodegradable polymer dispersion or the like may be selected in consideration of the relation with the second continuous phase of the second calcium phosphate dispersion (for example, solubility, affinity, and the like) described above.

For example, chloroform or dichloromethane (hereinafter referred to as DCM) is characterized by having a relatively high solubility in ethanol which may be the second continuous phase of the second calcium phosphate dispersion described above. Here, chloroform or DCM, which has a relatively high solubility in ethanol, may be selected as the solvent or liquid continuous phase of the biodegradable polymer dispersion or the like. This may help materials (for example, a combination of chloroform and ethanol or of DCM and ethanol) contained in the calcium phosphate-biodegradable polymer dispersion which will be described later, to be dissolved with each other without forming separable phases. In addition, environmental conditions under which the aggregation of the calcium phosphate particles occurs less may be provided.

Alternatively, the solvent or liquid continuous phase of the biodegradable polymer dispersion may be selected in consideration of volatility. Specifically, the preparation method of the biocompatible composite of the present application may include step S3000 of removing the liquid continuous phase of the calcium phosphate-biodegradable polymer dispersion to prepare the biocompatible composite to be described later. In this case, when using a highly volatile material as the solvent or liquid continuous phase of the biodegradable polymer dispersion or the like, the solvent or liquid continuous phase of the calcium phosphate-biodegradable polymer dispersion may be removed at a relatively low temperature. For example, DCM is characterized by having a low boiling point (about 39.6°C) and high volatility, and thus may be used as the solvent or liquid continuous phase of the PCL polymer dispersion, the PLLA polymer dispersion, or the like.

In one embodiment, the biodegradable polymer dispersion or biodegradable polymer solution may be mixed under mixing conditions determined in consideration of the properties of the biodegradable polymer and the liquid continuous phase (or solvent). Here, the mixing conditions may include the meaning of various conditions related to mixing, including mixing temperature, mixing time, mixing speed, and the like.

In one embodiment, the biodegradable polymer and the liquid continuous phase may be mixed at room temperature.

In one embodiment, the biodegradable polymer and the liquid continuous phase may be mixed at a temperature other than room temperature.

For example, when the biodegradable polymer is the PCL polymer and the liquid continuous phase is ethanol (EtOH), a temperature at which the biodegradable polymer and the liquid continuous phase are mixed may be determined to be a temperature in a range of 78°C or higher and 100°C or lower and preferably in the range of 78°C or higher and 90°C or lower. In particular, considering the melting point of the PCL polymer and the evaporation temperature of ethanol, the mixing temperature in the range of 78°C or higher and 90°C or lower may be a temperature that allows the PCL polymer to be melted or partially melted by applying heat to the PCL polymer while using ethanol as the heat medium. Therefore, the PCL polymer dispersion in which PCL polymer particles are dispersed in the ethanol continuous phase may be prepared by being heated to a temperature in a range of 78°C or higher and 90°C or lower.

For another example, when the biodegradable polymer is the PCL polymer and the solvent is chloroform, a temperature at which the biodegradable polymer and the liquid continuous phase are mixed may be determined to be a temperature in a range of 15°C or higher and 60°C or lower. For another example, the temperature may be determined to be a temperature in a range of 40°C or higher and 60°C or lower. Here, the temperature in a range of 40°C or higher and 60°C or lower is a temperature lower than 61.5°C which is the evaporation temperature of chloroform, and may be a temperature capable of shortening the time of the process. Here, the PCL polymer solution in which the PCL polymer particles are melted or dissolved in the chloroform solvent may be prepared by being heated to a temperature in a range of 40°C or higher and 60°C or lower.

For another example, when the biodegradable polymer is the PCL polymer and the solvent is DCM, a temperature at which the biodegradable polymer and the liquid continuous phase are mixed may be determined to be a temperature in a range of 15°C or higher and 39°C or lower and preferably in the range of 30°C or higher and 35°C or lower. In particular, the temperature in the range of 30°C or higher and 35°C or lower is a temperature lower than 39°C which is the evaporation temperature of DCM, and may be a temperature suitable for facilitating shortening the time of the process and stirring. Here, the PCL polymer solution in which PCL polymer particles are melted or dissolved in the DCM solvent may be prepared by being heated to the temperature conditions described above.

Although the liquid continuous phase (or solvent) of the biodegradable polymer dispersion or the like, the mixing conditions, and the like have been described above, the liquid continuous phase (or solvent) and the mixing temperature described above are disclosed only for illustrative purposes. Any suitable material may be selected as the liquid continuous phase (or solvent) of the biodegradable polymer dispersion. In this case, it is apparent that the biodegradable polymer dispersion may be mixed under any suitable mixing conditions in consideration of the properties of the selected liquid continuous phase (or solvent) and the properties of the biodegradable polymer. In addition, the descriptions related to the criteria for selecting the liquid continuous phase (or solvent) and the mixing conditions described above may be applied similarly to the step of mixing the second calcium phosphate dispersion and the biodeg radable polymer dispersion in step S2000 which will be described later.

On the other hand, the form (state) of the biodegradable polymer dispersion or biodegradable polymer solution, prepared in step S1000, may depend on the type of selected liquid continuous phase (or solvent) and the biodegradable polymer.

For example, when the biodegradable polymer is the PCL polymer and the selected liquid continuous phase is ethanol (EtOH), the biodegradable polymer dispersion prepared in step S1000 may be present in dispersion form in which solid-phase biodegradable polymer particles are dispersed in ethanol as the liquid continuous phase. The PCL polymer tends to be relatively less soluble in ethanol (EtOH) than in organic solvents, including chloroform, which will be described later. Thus, when the biodegradable polymer is the PCL polymer and the selected liquid continuous phase is ethanol (EtOH) in step S1000, the PCL polymer dispersion in which the PCL polymer is not completely melted or dissolved may be prepared.

For another example, when the biodegradable polymer is PCL polymer and the solvent selected in step S1000 is chloroform or DCM, the resulting biodegradable polymer mixture prepared in step S1000 may be present in solution form. For example, as described above, the PCL polymer tends to be relatively more soluble in organic solvents, including chloroform or DCM, than in ethanol (EtOH). Thus, in the case where a mixture contains the PCL polymer and chloroform or DCM serving as the solvent, the bonding between units constituting the PCL polymer may be broken to a relatively greater extent than in the case where ethanol is used as the liquid continuous phase. Accordingly, when the biodegradable polymer is the PCL polymer and the selected solvent is chloroform or DCM in step S1000, the PCL polymer solution having a form in which the PCL polymer is practically completely dissolved may be prepared.

Although the PCL polymer among the biodegradable polymers has been mainly described above, the above descriptions may be similarly applied to any biodegradable polymer (for example, a PLLA polymer, a PLGA polymer, and the like) having similar properties for ethanol, chloroform, or DCM (for example, a PLLA polymer, a PLGA polymer, and the like).

The second calcium phosphate dispersion and the biodegradable polymer dispersion prepared through the embodiment described above are mixed in step S2000. Hereinafter, step S2000 of preparing the calcium phosphate-biodegradable polymer dispersion by mixing the second calcium phosphate dispersion and the biodegradable polymer dispersion (or biodegradable polymer solution) is to be described in detail.

## 1.2. Step S2000 of preparing calcium phosphate-biodegradable polymer dispersion by mixing second calcium phosphate dispersion and biodegradable polymer dispersion (or biodegradable polymer solution)

The preparation method of the biocompatible composite of the present application includes step S2000 of preparing the calcium phosphate-biodegradable polymer dispersion by mixing the second calcium phosphate dispersion and the biodegradable polymer dispersion or biodegradable polymer solution (hereinafter referred to as biodegradable polymer dispersion or the like).

In one embodiment, in step S2000, the second calcium phosphate dispersion and the biodegradable polymer dispersion or the like prepared in the respective containers may be combined into one container and then mixed, including but not limited thereto. It is apparent that any suitable method may be applied such that the second calcium phosphate dispersion and the biodegradable polymer dispersion or the like coexist.

On the other hand, the calcium phosphate-biodegradable polymer dispersion prepared in step S2000 is mixed using a sonicator, an impeller, or a magnetic bar, thereby dispersing the calcium phosphate particles in the biodegradable polymer matrix.

In one embodiment, mixing conditions of the calcium phosphate-biodegradable polymer dispersion may be determined in consideration of the liquid continuous phase of the biodegradable polymer or the like. Furthermore, the mixing conditions may be determined in consideration of the properties of the second continuous phase of the second calcium phosphate dispersion.

For example, the liquid continuous phase of the biodegradable polymer and the liquid continuous phase of the second calcium phosphate dispersion may be the same. In this case, the mixing conditions (for example, mixing temperature) of the liquid continuous phase (or solvent) and the biodegradable polymer disclosed in step S1000 may be similarly applied.

For example, the second calcium phosphate dispersion and the biodegradable polymer dispersion or the like may be mixed at room temperature. For another example, the second calcium phosphate dispersion and the biodegradable polymer dispersion or the like may be mixed at a temperature other than room temperature.

For example, when the biodegradable polymer is the PCL polymer and the temporarily substituted continuous phase is ethanol (EtOH), in consideration of the melting point of the PCL polymer (in a range of about 55°C to 60°C) and the evaporation temperature of ethanol (about 78°C), a temperature at which the calcium phosphate-biodegradable polymer dispersion is mixed may be a temperature suitable for melting or partially melting the PCL polymer while using ethanol as the heat medium. For example, in this case, the mixing temperature in step S2000 may be determined to be a temperature in a range of 78°C or higher and 100°C or lower. For example, the mixing temperature in step S2000 may be determined to be a temperature in a range of 78°C or higher and 90°C or lower.

For another example, when the biodegradable polymer is the PCL polymer and the solvent of the biodegradable polymer solution is chloroform, in consideration of the solubility of the PCL polymer in chloroform, the melting point of the PCL polymer, the evaporation temperature of chloroform, and/or the solubility of ethanol in chloroform, a temperature at which the calcium phosphate-biodegradable polymer dispersion is mixed may be a temperature suitable for melting or dissolving the PCL polymer. Alternatively, when the biodegradable polymer is the PCL polymer and the solvent of the biodegradable polymer solution is chloroform, a temperature at which the calcium phosphate-biodegradable polymer dispersion is mixed may be determined to be a temperature relatively lower than the evaporation temperature of chloroform.

For example, in this case, the mixing temperature in step S2000 may be a temperature in a range of 15°C or higher and 60°C or lower. For example, the mixing temperature in step S2000 may be determined to be a temperature in a range of 40°C or higher and 60°C or lower.

For another example, when the biodegradable polymer is the PCL polymer and the solvent of the biodegradable polymer solution is DCM, in consideration of the solubility of the PCL polymer in DCM, the melting point of the PCL polymer, the evaporation temperature of DCM, and/or the solubility of ethanol in DCM, a temperature at which the calcium phosphate-biodegradable polymer dispersion is mixed may be a temperature suitable for melting or dissolving the PCL polymer. Alternatively, when the biodegradable polymer is the PCL polymer and the solvent of the biodegradable polymer solution is DCM, a temperature at which the calcium phosphate-biodegradable polymer dispersion is mixed may be determined to be a temperature relatively lower than the evaporation temperature of DCM.

For example, in this case, DCM is characterized by being soluble in ethanol and having a relatively lower boiling point (about 39°C) than chloroform. For this reason, the mixing temperature in step S2000 may be determined to be a temperature in a range of 15°C or higher and 39°C or lower, which encompasses relatively lower temperatures than in the case of using chloroform. For example, the mixing temperature in step S2000 may be determined to be a temperature in a range of 20°C or higher and 39°C or lower. For example, the mixing temperature in step S2000 may be determined to be a temperature in a range of 30°C or higher and 35°C or lower.

Step S2000 may enable the calcium phosphate particles and the biodegradable polymer to be mixed while coexisting, so the calcium phosphate particles may be dispersed in the biodegradable polymer.

Although the mixing conditions of the calcium phosphate-degradable polymer dispersion and the like have been described above, the mixing conditions, including the mixing temperature and the like, described above, are disclosed only for illustrative purposes. The calcium phosphate-degradable polymer dispersion may be prepared by mixing the second calcium phosphate dispersion and the biodegradable polymer dispersion under any suitable mixing condition in consideration of the form (state) of the calcium phosphate-biodegradable polymer dispersion, the properties of the biodegradable polymer, the properties of the liquid continuous phase, and/or the like.

### 1.3. Step S3000 of removing liquid continuous phase of calcium phosphate-biodegradable polymer dispersion to prepare biocompatible composite

The preparation method of the biocompatible composite of the present application includes step S3000 of removing the liquid continuous phase of the calcium phosphate-biodegradable polymer dispersion to prepare the biocompatible composite.

The polymer may be cured while removing the liquid continuous phase of the calcium phosphate-biodegradable polymer dispersion. The calcium phosphate particles, which were more uniformly dispersed in the liquid continuous phase, are uniformly dispersed and positioned in the polymer matrix during the process of removing the liquid continuous phase and curing the polymer to form the polymer matrix. Through such processes, the biocompatible composite containing the polymer matrix and the calcium phosphate particles may be prepared.

Step S3000 may include drying the calcium phosphate-biodegradable polymer dispersion so that the liquid continuous phase of the calcium phosphate-biodegradable polymer dispersion is removed. Hereinafter, the step of drying the calcium phosphate-biodegradable polymer dispersion is to be disclosed in detail.

In one embodiment, the calcium phosphate-biodegradable polymer dispersion may be dried by applying heat or dried at room temperature to remove the liquid continuous phase contained in the calcium phosphate-biodegradable polymer dispersion, thereby preparing the biocompatible composite.

In this case, the liquid continuous phase contained in the calcium phosphate-biodegradable polymer dispersion is prepared by mixing the second continuous phase, which is the continuous phase of the second calcium phosphate dispersion, and the liquid continuous phase (or solvent) of the biodegradable polymer dispersion or the like. In one embodiment, the liquid continuous phase of the calcium phosphate-biodegradable polymer dispersion may contain the second continuous phase and the liquid continuous phase (or solvent) of the biodegradable polymer dispersion or the like.

For example, the liquid continuous phase of the calcium phosphate-biodegradable polymer may be simply a mixture of the second continuous phase and the liquid continuous phase (or solvent) of the biodegradable polymer dispersion or the like. For another example, the liquid continuous phase of the calcium phosphate-biodegradable polymer may have a form in which either one of the second continuous phase and the liquid continuous phase (or solvent) of the biodegradable polymer dispersion or the like, serving as a solute, is dissolved in the other, serving as a solvent. For a specific example, the liquid continuous phase of the calcium phosphate-biodegradable polymer may have a form in which the liquid continuous phase (or solvent) of the biodegradable polymer dispersion or the like is dissolved in the second continuous phase. For another specific example, the liquid continuous phase of the calcium phosphate-biodegradable polymer may have a form in which the second continuous phase is dissolved in the liquid continuous phase (or solvent) of the biodegradable polymer dispersion or the like.

In one embodiment, the biocompatible composite may be prepared by drying the calcium phosphate-biodegradable polymer dispersion at room temperature and evaporating the liquid continuous phase contained in the calcium phosphate-biodegradable polymer dispersion.

For example, when the calcium phosphate-biodegradable polymer dispersion contains chloroform or DCM as the continuous phase, the calcium phosphate-biodegradable polymer dispersion may be thinly spread and dried at room temperature, enabling chloroform or DCM to be removed and thus preparing the biocompatible composite. In particular, when the calcium phosphate-biodegradable polymer dispersion contains DCM as the continuous phase, since DCM is characterized by having a lower boiling point and higher volatility than chloroform, the DCM contained in the calcium phosphate-biodegradable dispersion may be removed further easily and quickly even when dried at a relatively lower temperature than in the case of using chloroform.

In another embodiment, thermal energy may be applied to the calcium phosphate-biodegradable polymer dispersion in any manner such that the liquid continuous phase of the calcium phosphate-biodegradable polymer dispersion is removed. For example, the calcium phosphate-biodegradable polymer dispersion may be heated using a heating plate, a dry oven, and the like such that the liquid continuous phase of the calcium phosphate-biodegradable polymer dispersion is removed. As a result, the liquid continuous phase of the calcium phosphate-biodegradable polymer dispersion may be removed, thereby preparing the biocompatible composite.

In this case, the drying temperature may be determined in consideration of the properties of the liquid continuous phase contained in the calcium phosphate-biodegradable polymer dispersion, the properties of the calcium phosphate contained in the calcium phosphate-biodegradable polymer dispersion, the properties of the biodegradable polymer contained in the calcium phosphate-biodegradable polymer dispersion, and/or whether the calcium phosphate contained in the calcium phosphate-biodegradable polymer dispersion is precipitated. For example, the drying temperature may be a temperature higher than the boiling point of the liquid continuous phase contained in the calcium phosphate-biodegradable polymer dispersion. For example, the drying temperature may be a temperature lower than the sintering temperature of the calcium phosphate particles contained in the calcium phosphate-biodegradable polymer dispersion. For example, the drying temperature may be set to fall within a temperature condition under which the biodegradable polymer contained in the calcium phosphate-biodegradable polymer dispersion is prevented from being degraded.

In one embodiment, the drying temperature may be about 50°C, 55°C, 60°C, 65°C, 70°C, 75°C, 80°C, 85°C, 90°C, 95°C, 100°C, 110°C, 120°C, 130°C, 140°C, 150°C, 160°C, 170°C, 180°C, 190°C, 200°C, 220°C, 240°C, 260°C, 280°C, or 300°C, or may be set to a temperature value equal to or higher than the temperature value described above.

Hereinafter, the drying temperature is to be described through various examples.

The drying temperature may be determined in consideration of the boiling point of the liquid continuous phase of the calcium phosphate-biodegradable polymer dispersion. For example, when the liquid continuous phase of the calcium phosphate-biodegradable polymer dispersion contains ethanol (EtOH), the drying temperature may be determined in consideration of the boiling point of ethanol (EtOH), which is about 78°C. Specifically, the drying temperature may be determined to be a temperature higher than the boiling point of ethanol (EtOH), which is about 78°C. For example, the drying temperature may be determined to be a temperature in a range of higher than 78°C and lower than or equal to 200°C. Preferably, the drying temperature may be determined to be a temperature in the range of higher than 78°C and lower than or equal to 90°C.

For another example, when the liquid continuous phase of the calcium phosphate-biodegradable polymer dispersion contains ethanol (EtOH), the drying temperature may be determined to be a temperature at which the biodegradable polymer is prevented from being degraded. For example, the drying temperature may be determined to be a temperature in a range of higher than 55°C and lower than or equal to 90°C.

For example, when the liquid continuous phase of the calcium phosphate-biodegradable polymer dispersion is chloroform, the drying temperature may be determined in consideration of the boiling point of chloroform, which is about 61.2°C. Specifically, the drying temperature may be determined to be a temperature higher than the boiling point of chloroform, which is about 61.2°C. Preferably, the drying temperature may be determined to be a temperature in a range of 61.2°C or higher and 200°C or lower.

For another example, when the liquid continuous phase of the calcium phosphate-biodegradable polymer dispersion is chloroform, the drying temperature may be determined to be a temperature at which the biodegradable polymer is prevented from being degraded. For example, the drying temperature may be determined to be a temperature in a range of higher than 40°C and lower than or equal to 80°C.

In addition, the drying temperature may be determined in consideration of the sintering temperature between the calcium phosphate particles of the calcium phosphate-biodegradable polymer dispersion. Preferably, the drying temperature may be determined to be a temperature lower than the sintering temperature between the calcium phosphate particles. For example, while the sintering temperature of the calcium phosphate particles (for example, hydroxyapatite particles) may be about 900°C or higher, the drying temperature in this case may be determined to be about 900°C or lower.

In another embodiment, the calcium phosphate-biodegradable polymer dispersion may be dried at a first temperature for a first time period and then dried at a second temperature for a second time period. In this case, a second temperature value is lower than a first temperature value. The calcium phosphate-biodegradable polymer dispersion is firstly dried at the first temperature for the first time period. In the firstly drying, a portion of the liquid continuous phase of the calcium phosphate-biodegradable polymer dispersion is further rapidly removed through the short-term drying at the first temperature for the first time period, thereby preventing the calcium phosphate particles from being precipitated. The first temperature, which is a first drying temperature, may be higher than the temperature at which the biodegradable polymer is degraded. However, the first time period may be set to a time period before the biodegradable polymer is degraded, thereby preventing the biodegradable polymer from being degraded. In other words, the first temperature, which is the first drying temperature, is the temperature of the external environment (for example, the temperature set in a heating plate), not the temperature inside the calcium phosphate-biodegradable polymer dispersion. In this case, the first temperature and the first time period may be determined such that the heat transferred from the external environment into the calcium phosphate-biodegradable polymer dispersion for the first time period is insufficient to cause the biodegradable polymer to be degraded. The firstly drying is performed at the first temperature for the first time period to prevent the precipitation of the calcium phosphate particles. In addition, the secondly drying is performed at the second temperature for the second time period to completely remove the liquid continuous phase of the calcium phosphate-biodegradable polymer dispersion. In this case, the second temperature is set to be lower than the first temperature, thereby preventing the biodegradable polymer from being degraded due to the temperature.

In one embodiment, the first temperature may be about 50°C, 55°C, 60°C, 65°C, 70°C, 75°C, 80°C, 85°C, 90°C, 95°C, 100°C, 110°C, 120°C, 130°C, 140°C, 150°C, 160°C, 170°C, 180°C, 190°C, 200°C, 220°C, 240°C, 260°C, 280°C, or 300°C, or may be set to a temperature value greater than or equal to the temperature value described above.

In one embodiment, the first time period may be about 0.1, 0.2, 0.3, 0.4, 0.6, 0.8, 1.0, 1.2, 1.4, 1.6, 1.8, 2, 2.2, 2.4, 2.6, 2.8, 3, 3.2, 3.4, 3.6, 3.8, 4, 4.2, 4.4, 4.6, 4.8, 5, 5.5, 6, 6.5, 7, 7.5, 8, 8.5, 9, 9.5, or 10 hours, or may be set to a value greater than or equal to the value described above.

For example, when the calcium phosphate-biodegradable polymer dispersion contains ethanol and chloroform, the calcium phosphate-biodegradable polymer dispersion may be firstly dried at 150°C for 2 hours and then dried at 80°C for 24 hours to prepare the biocompatible composite.

However, the descriptions above are disclosed only for illustrative purposes, and any suitable method may be applied to prepare the biocompatible composite by removing the liquid continuous phase of the calcium phosphate-biodegradable polymer dispersion. In addition, the exemplified drying temperatures are disclosed only for illustrative purposes. The drying temperature may be determined to fall within any suitable temperature range in consideration of the properties of the continuous liquid phase of the calcium phosphate-biodegradable polymer dispersion or the quality of the biocompatible composite.

On the other hand, the biocompatible composite prepared in step S3000 may be shaped into various forms.

In one embodiment, the calcium phosphate-biodegradable polymer dispersion may be dried to prepare the biocompatible composite in a sheet form. For example, the calcium phosphate-biodegradable polymer dispersion prepared in step S2000 may be dried at room temperature while being thinly spread on a die or the like. Alternatively, in step S3000, heat may be applied to the thinly spread calcium phosphate-biodegradable polymer dispersion to dry the calcium phosphate-biodegradable polymer dispersion. In particular, when the liquid continuous phase of the calcium phosphate-biodegradable polymer dispersion contains chloroform, the drying of the calcium phosphate-biodegradable polymer dispersion in the sheet-like form may facilitate the liquid continuous phase to be removed. For example, the calcium phosphate-biodegradable polymer dispersion may be dried to prepare a biocompatible composite in a cubic-like form. For example, the calcium phosphate-biodegradable polymer dispersion prepared in step S2000 may be dried on a mold using a casting method. In this case, the calcium phosphate-biodegradable polymer dispersion prepared in step S2000 may be dried using a dry oven or the like while being poured into the mold. In particular, when the liquid continuous phase of the calcium phosphate-biodegradable polymer dispersion is ethanol (EtOH), the drying of the calcium phosphate-biodegradable polymer dispersion in the cubic-like form may facilitate the liquid continuous phase to be removed.

For example, the biocompatible composite in a form of pellet may be prepared by drying the calcium phosphate-biodegradable polymer dispersion. For example, the biocompatible composite in the sheet-like or cubic-like form described above may be cut into the biocompatible composite in the pellet-like form.

In this case, the biocompatible composite may be stored and distributed in the sheet-like, cubic-like, or pellet-like form described above.

However, the forms of the biocompatible composite described above are disclosed only for illustrative purposes, and the biocompatible composite may be transformed into various forms, including a plate-like form and the like.

**1.4. Step capable of being additionally included: step S4000 of molding biocompatible composite**

In one embodiment, the preparation method of the biocompatible composite of the present application may further include step S4000 of molding the biocompatible composite. This enables the prepared biocompatible composite to be molded into a specific form.

In one embodiment, step S4000 may include applying a predetermined pressure to the biocompatible composite to shape the same. This enables the biocompatible composite to be molded into a specific form. For example, the biocompatible composite may be shaped by applying a pressure in a range of 10 MPa or more and 50 MPa or less to the biocompatible composite. For example, through compression molding in which a pressure in a range of 10 MPa or more and 50 MPa or less is applied to the biocompatible composite in the pellet-like form prepared in step S4000, the biocompatible composite in the plate-like form may be prepared.

For another example, through thermal compression molding in which a predetermined pressure (for example, in a range of 10 MPa or more and 50 MPa or less) is applied to the biocompatible composite in the pellet-like form in step S3000 while heating the same to a predetermined temperature, the biocompatible composite in the plate-like form may be prepared. In this case, the heating temperature may be determined in consideration of the sintering temperature of the calcium phosphate particles described above. For example, the heating temperature may be a temperature of about 900°C or lower, which is known to be the sintering temperature of the calcium phosphate particles. For example, the heating temperature may be a temperature in a range of 180°C or higher and 220°C or lower. For example, the heating temperature may be a temperature of about 200°C.

For another example, the biocompatible composite prepared in step S3000 may be compressed using a mold to shape the biocompatible composite. Here, in the case of compressing the biocompatible composite using the mold, the amount of heat substantially applied to the biocompatible composite placed in the mold may be relatively smaller than that in the case of directly heating the biocompatible composite material to a temperature in a range of 180°C or higher and 220°C or lower, as described above.

However, the descriptions are disclosed only for illustrative purposes, and any suitable method may be applied to shape the biocompatible composite material into a predetermined form. In addition, any suitable temperature range and/or pressure range may be applied depending on the desired forms of the composite.

**1.5. Weight ratio of calcium phosphate to biodegradable polymer used in preparation method of biocompatible composite of present application**

A weight ratio of the calcium phosphate to the biodegradable polymer used in the preparation method of the biocompatible composite may be appropriately determined depending on the weight ratio of the calcium phosphate particles contained in the biocompatible composite to be prepared. Alternatively, the weight ratio of the calcium phosphate to the biodegradable polymer, used in the preparation method of the biocompatible composite, may be appropriately determined depending on the purpose of using the biocompatible composite to be prepared. Here, the weight of the calcium phosphate used herein means the weight of the calcium phosphate contained in the second calcium phosphate dispersion. In addition, the weight of the biodegradable polymer used herein is the weight of the biodegradable polymer dispersed (or dissolved) in the biodegradable polymer dispersion.

In one embodiment, the weight of the calcium phosphate used in the method for producing the biocompatible composite may be about 1%, 2%, 3%, 4%, 5%, 6%, 7%, 8%, 9%, 10%, 11%, 12%, 13%, 14%, 15%, 16%, 17%, 18%, 19%, 20%, 22%, 24%, 26%, 28%, 30%, 32%, 34%, 36%, 38%, 40%, 42%, 44%, 46%, 48%, 50%, 55%, 60%, 65%, or 70% of the weight of the biodegradable polymer used in the method for producing the biocompatible composite. In a specific embodiment, the weight of the calcium phosphate used in the method for producing the biocompatible composite may be about 20%, 22%, 24%, 26%, 28%, 30%, 32%, 34%, 36%, 38%, 40%, 42%, 44%, 46%, 48%, or 50% of the biodegradable polymer used in the method for producing the biocompatible composite. In a specific embodiment, the weight of the calcium phosphate used herein may be about 40%, 42%, or 44% of the weight of the biodegradable polymer used herein.

## 2. Biocompatible composite

In terms of the composite, uniformity in the distribution of the calcium phosphate particles in the biodegradable polymer matrix is one of the important properties related to the quality of the biocompatible composite. In particular, the uniformity of dispersion of calcium phosphate in each region in the biodegradable polymer matrix is a variable directly related to the quality of the composite, such as mechanical strength and osteogenesis ability. In addition, uniformity is also a property that affects the profile of the calcium phosphate particles released over time when applied in vivo and is thus important.

The uniformity used herein may mean that the ratio of the calcium phosphate dispersed in each region in the biodegradable polymer matrix is relatively even. The ratio of the calcium phosphate dispersed in each region in the biodegradable polymer matrix may be quantified in various ways. For example, the area where the calcium phosphate particles are present in a reference area may be measured or calculated for quantification. In this case, the standard deviation of the values quantified for each region closer to 0, which is small, may mean that the calcium phosphate particles are more uniformly dispersed in each region in the biodegradable polymer matrix.

The ratio of the calcium phosphate dispersed in each region in the biodegradable polymer matrix disclosed herein may be quantified through a suitable method. For example, the ratio of the calcium phosphate dispersed in each region in the biodegradable polymer matrix may be quantified by a method including: dividing a cross section of the composite into a plurality of regions having a reference area, measuring a ratio of each atom in each region using energy-dispersive spectroscopy (EDS) which is a method of analyzing atoms on the surface, and/or Image J program, and analyzing the measurement results.

In one embodiment, the uniformity in the distribution of the calcium phosphate particles in the biocompatible composite may be calculated, measured, and/or quantified by the following method:

One or two or more cross sections of the biocompatible composite are divided into a plurality of regions having a predetermined reference area.

A ratio of an area occupied by a first atom in each region is measured. In this case, a plurality of ratios of areas occupied by the first atom measured from each region are collected to form a first data group (for example, the first data group includes the following: a ratio of an area occupied by the first atom measured from a first region, a ratio of an area occupied by the first atom measured from a second region, ..., a ratio of an area occupied by the first atom measured from an n-th region, where n is an integer of 2 or more). In this case, the Image J program may be used to measure each ratio of the area occupied by the first atom.

A ratio of an area occupied by a second atom in each region is measured. In this case, a plurality of ratios of areas occupied by the second atom measured from each region are collected to form a second data group (for example, the second data group includes the following: a ratio of an area occupied by the second atom measured from a first region, a ratio of an area occupied by the second atom measured from a second region, ..., a ratio of an area occupied by the second atom measured from an n-th region, where n is an integer of 2 or more). The ratio of the area occupied by the second atom may be selectively measured as needed. In this case, the Image J program may be used to measure each ratio of the area occupied by the first atom.

When there is a third atom, a ratio of an area occupied by the third atom in each region is measured. In this case, a plurality of ratios of areas occupied by the third atom measured from each region are collected to form a third data group (for example, the third data group includes the following: a ratio of an area occupied by the third atom measured from a first region, a ratio of an area occupied by the third atom measured from a second region, ..., a ratio of an area occupied by the third atom measured from an n-th region, where n is an integer of 2 or more). The ratio of the area occupied by the third atom may be selectively measured as needed. In this case, the Image J program may be used to measure each ratio of the area occupied by the first atom.

In this case, the first, second, and/or third atoms may be each independently selected from among atoms contained in the calcium phosphate particle. For example, when the calcium phosphate particles contained in the biocompatible composite are hydroxyapatite particles, the first atom may be Ca, and the second atom may be P. For another example, when the calcium phosphate particles contained in the biocompatible composite are whitlockite particles, the first atom may be Ca, the second atom may be Mg, and the third atom may be P. For a further example, when the calcium phosphate particles contained in the biocompatible composite material are $\beta$-TCP, the first atom may be Ca, and the second atom may be P.

On the basis of the ratio of the area occupied by the first atom measured from each region, an average of ratios of the area occupied by the first atom in the plurality of regions is calculated, and a standard deviation is calculated. In other words, the average of data included in the first data group is calculated, and the standard deviation is calculated.

On the basis of the ratio of the area occupied by the second atom measured from each region, an average of ratios of the area occupied by the second atom in the plurality of regions is calculated, and a standard deviation is calculated. In other words, the average of data included in the second data group is calculated, and the standard deviation is calculated. This process for the second atom is selectively performed as needed.

When there is the third atom, on the basis of the ratio of the area occupied by the third atom measured from each region, an average of ratios of the area occupied by the third atom in the plurality of regions is calculated, and a standard deviation is calculated. In other words, the average of data included in the third data group is calculated, and the standard deviation is calculated. This process for the third atom is selectively performed as needed.

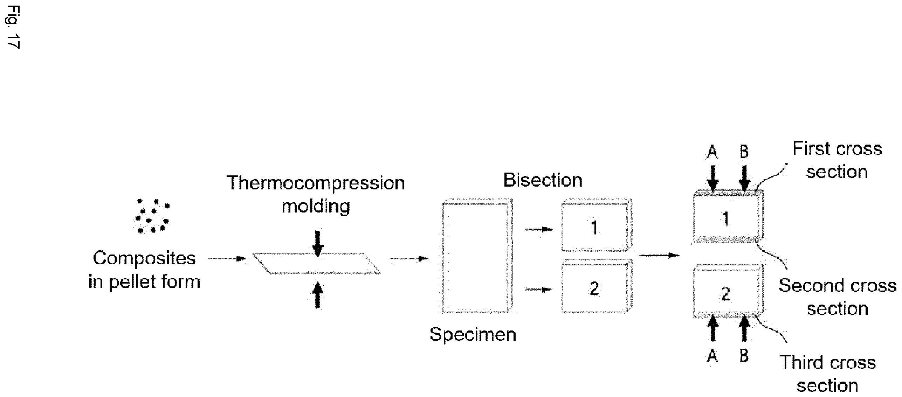

One embodiment of a method of measuring or quantifying the uniformity in the distribution of the calcium phosphate particles in the biocompatible composite is disclosed in detail with reference to FIGS. 17 to 20. However, any suitable quantification method other than the method described above may also be used.

In terms of the composite, the weight ratio of the calcium phosphate in the composite is one of the important variables related to the quality of the biocompatible composite. In particular, in terms of the composite, the weight ratio of the calcium phosphate in the composite directly affects the quality of the composite material, especially osteogenesis ability, and thus is important.

Therefore, in the process of preparing a composite, it is important to prepare the composite with a wide range of calcium phosphate weight ratios or to prepare the composite with a high calcium phosphate weight ratio.

The biocompatible composite, according to one embodiment of the present application, is characterized by uniform regional distribution of calcium phosphate particles in the biodegradable polymer matrix.

In the biocompatible composite according to one embodiment of the present application, the calcium phosphate particles may be distributed in the biodegradable polymer matrix in a relatively "higher weight ratio" than in a composite prepared by an existing preparation method of composites. In addition, in the biocompatible composite according to one embodiment of the present application, the calcium phosphate particles may be distributed in the biodegradable polymer matrix in a weight ratio falling within a relatively "wider range" than that in the existing preparation method of the composites.

The biocompatible composite of the present application may comprise the biodegradable polymer matrix and the calcium phosphate. In this case, the calcium phosphates are comprised in a dispersed form in the biodegradable polymer matrix. In this case, the calcium phosphate may contain calcium atom and phosphorus atom, or may contain calcium atom, magnesium atom, and phosphorus atom. For example, the calcium phosphate may be hydroxyapatite. In this case, the calcium phosphate may contain calcium and phosphorus atoms. For another example, the calcium phosphate may be β-TCP. In this case, the calcium phosphate may contain calcium and phosphorus atoms. For a further example, the calcium phosphate may be whitlockite. In this case, the calcium phosphate may contain calcium, phosphorus, and magnesium atoms.

In one embodiment of the present application, provided is a biocompatible composite comprising the following:

a biodegradable polymer matrix; and
calcium phosphate particles dispersed in the biodegradable polymer matrix with uniformity of a first degree of uniformity.

In one embodiment, the first degree of uniformity may be about 0.001, 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.6, 0.7, 0.8, 0.9, 1, 1.1, 1.2, 1.3, 1.4, 1.5, 1.6, 1.7, 1.8, 1.9, 2, 2.2, 2.4, 2.6, 2.8, 3, 3.2, 3.4, 3.6, 3.8, 4, 4.2, 4.4, 4.6, 4.8, 5, 5.5, 6, 6.5, 7, 7.5, 8, 8.5, 9, 9.5, 10, 11, 12, 13, 14, 15, 16, 17, 18, 19, or 20, or may fall within a range set by two values selected from among values described above.

In one embodiment, the first degree of uniformity may be 0.01, 0.02, 0.03, 0.04, 0.05, 0.06, 0.07, 0.08, 0.09, 0.1, 0.5, 1, 1.5, 2, 2.5, 3, 3.5, 4, 4.5, 5, 6, 7, 8, 9, 10, 12, 14, 16, 18, or 20 or less. In a specific embodiment, the first degree of uniformity may be 10 or less. In a specific embodiment, the first degree of uniformity may be 5 or less. In a specific embodiment, the first degree of uniformity may be 2 or less. In a specific embodiment, the first degree of uniformity may be 1 or less. In a specific embodiment, the first degree of uniformity may be 0.5 or less. In a specific embodiment, the first degree of uniformity may be 0.1 or less. In a specific embodiment, the first degree of uniformity may be 0.05 or less.

In one embodiment, the first degree of uniformity may be calculated as follows:

measuring first ratios (%) with respect to a plurality of regions in the biocompatible composite, wherein each of the first ratios is a ratio of an area occupied by the first atom in each of the plurality of regions;
calculating a standard deviation of the first ratios measured from the plurality of regions to obtain a first standard deviation; and
obtaining the first degree of uniformity on the basis of the first standard deviation.

In one embodiment, the respective regions may have the same reference area.

In one embodiment, the first atom may be any one selected from calcium atom, phosphorus atom, and magnesium atom. In a specific embodiment, the first atom may be a calcium atom. In a specific embodiment, the first atom may be a phosphorus atom. In a specific embodiment, the first atom may be a magnesium atom.

In one embodiment, the first degree of uniformity may be the first standard deviation, or may be a value calculated from the first standard deviation using a suitable function. In one embodiment, the first degree of uniformity may be a value derived by normalizing the first standard deviation.

In a specific embodiment, the first degree of uniformity may be a value obtained by dividing the first standard deviation by an average of the plurality of first ratios measured from the plurality of regions. When the first degree of uniformity is a value obtained by dividing the first standard deviation by the average of the plurality of first ratios measured from the plurality of regions, the first degree of uniformity may be 0.01, 0.02, 0.03, 0.04, 0.05, 0.06, 0.07, 0.08, 0.09, 0.1, or 0.2 or less. In a specific embodiment, the first degree of uniformity may be 0.05 or less.

In one embodiment, the first ratio may be measured using the Image J program.

In another embodiment, the first degree of uniformity may be a value calculated from the standard deviation with respect to a plurality of atoms rather than one type of atom. For example, the first degree of uniformity may be calculated as follows:

measuring first ratios and second ratios with respect to a plurality of regions in the biocompatible composite,
wherein each of the first ratios is a ratio of an area occupied by a first atom in each region, and each of the second ratios is a ratio of an area occupied by a second atom in each region,
wherein the first atom is any one selected from calcium atom, phosphorus atom, and magnesium atom, the second atom is any one selected from calcium atom, phosphorus atom, and magnesium atom, and the first atom and the second atom are different from each other;
calculating a standard deviation of the first ratios measured from the plurality of regions to obtain a first standard deviation, and calculating a standard deviation of the second ratios measured from to the plurality of regions to obtain a second standard deviation; and
obtaining the first degree of uniformity on the basis of the calculated first and second standard deviations.

In one embodiment, the first degree of uniformity may be calculated by the following equation: (first standard deviation + second standard deviation)/2.

In one embodiment, the first degree of uniformity may be normalized.

In one embodiment, the first degree of uniformity may be calculated by the following equation: (first standard deviation + second standard deviation)/(average of first ratios measured from plurality of regions + average of second ratios measured from plurality of regions).

In one embodiment, the first ratios and the second ratios may be measured using Image J.

In a further embodiment, the first degree of uniformity may be calculated as follows:

measuring first ratios, second ratios, and third ratios with respect to a plurality of regions in the biocompatible composite,
wherein each of the first ratios is a ratio of an area occupied by a first atom in each region, each of the second ratios is a ratio of an area occupied by a second atom in each region, and each of the third ratios is a ratio of an area occupied by a third atom in each region,
wherein the first atom is a calcium atom, the second atom is a phosphorus atom, and the third atom is a magnesium atom;
calculating a standard deviation of the first ratios measured from the plurality of regions to obtain a first standard deviation, calculating a standard deviation of the second ratios measured from the plurality of regions to obtain a second standard deviation, and calculating a standard deviation of the third ratios measured from the plurality of regions to obtain a third standard deviation; and
obtaining the first degree of uniformity on the basis of the calculated first standard deviation, second standard deviation, and third standard deviation.

In one embodiment, the first degree of uniformity may be calculated by the following equation: (first standard deviation + second standard deviation + third standard deviation)/3.

In one embodiment, the first degree of uniformity may be normalized.

In one embodiment, the first degree of uniformity may be calculated by the following equation: (first standard deviation + second standard deviation + third standard deviation)/(average of first ratios measured from plurality of regions + average of second ratios measured from plurality of regions + average of third ratios measured from plurality of areas).

In one embodiment, the first ratios, the second ratios, and the third ratios may be measured using Image J.

In one embodiment, the weight ratio of the calcium phosphate to the biocompatible composite may be in a range of 0.01 wt% or more and 60 wt% or less. In one embodiment, the weight ratio of the calcium phosphate to the biocompatible composite may be in a range of 0.1 wt% or more and 55 wt% or less. In a specific embodiment, the weight ratio of the calcium phosphate to the biocompatible composite may be in a range of 5 wt% or more and 50 wt% or less.

In a specific embodiment, the weight ratio of the calcium phosphate to the biocompatible composite may be in a range of 10 wt% or more and 40 wt% or less.

In one embodiment, the weight ratio of the calcium phosphate to the biocompatible composite may be about 5 wt%, 6 wt%, 7 wt%, 8 wt%, 9 wt%, 10 wt%, 11 wt%, 12 wt%, 13wt%, 14wt%, 15wt%, 16 wt%, 17 wt%, 18 wt%, 19 wt%, 20 wt%, 22 wt%, 24 wt%, 26 wt%, 28 wt%, 30 wt%, 32 wt%, 34 wt%, 36 wt%, 38 wt%, 40 wt%, 42 wt%, 44 wt%, 46 wt%, 48 wt%, or 50 wt%.

In one embodiment, provided is a biocompatible composite containing the following:

a biodegradable polymer matrix; and

calcium phosphate particles dispersed in the biodegradable polymer matrix.

Here, in a cross section of the biocompatible composite, an average of ratios of an area occupied by a first atom contained in calcium phosphate particles per unit area is a first average, and a standard deviation is a second standard deviation.

In one embodiment, the first atom may be any one selected from calcium atom, phosphorus atom, and magnesium atom. In a specific embodiment, the first atom may be a calcium atom. In a specific embodiment, the first atom may be a phosphorus atom. In a specific embodiment, the first atom may be a magnesium atom.

In one embodiment, the first atom may be a calcium atom. In addition, the first average, which is an average of ratios of area occupied by the calcium atoms contained in the calcium phosphate particles per unit area, in the cross section of the biocompatible composite may be about 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90%, or may fall within a range set by two values selected from among the % values described above. In a specific embodiment, the first average, which is the average of ratios of area occupied by the calcium atoms contained in the calcium phosphate particles per unit area, may be about 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, or 85%, or may fall within a range set by two values selected from among the % values described above. In a specific embodiment, the first average, which is the average of ratios of area occupied by the calcium atoms contained in the calcium phosphate particles per unit area, may be in a range of 50% to 85%.

In one embodiment, the first atom is calcium atoms. In addition, the second standard deviation may be about 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.6, 0.7, 0.8, 0.9, 1, 1.1, 1.2, 1.3, 1.4, 1.5, 1.6, 1.7, 1.8, 1.9, 2, 2.2, 2.4, 2.6, 2.8, 3, 3.2, 3.4, 3.6, 3.8, 4, 4.2, 4.4, 4.6, 4.8, 5, 5.5, 6, 6.5, 7, 7.5, 8, 8.5, 9, 9.5, 10, 11, 12, 13, 14, 15, 16, 17, 18, 19, or 20, or may fall within a range set by two values selected from among the values described above. In one embodiment, the second standard deviation may be 0.5, 1, 1.5, 2, 2.5, 3, 3.5, 4, 4.5, 5, 6, 7, 8, 9, 10, 12, 14, 16, 18, or 20 or less. In a specific embodiment, the second standard deviation may be 10 or less. In a specific embodiment, the second standard deviation may be 5 or less. In a specific embodiment, the second standard deviation may be 2 or less. In a specific embodiment, the second standard deviation may be 1 or less. In a specific embodiment, the second standard deviation may be 0.5 or less.

In one embodiment, the first atom is a phosphorus atom. In addition, the first average, which is the average of ratios of area occupied by the phosphorus atoms contained in the calcium phosphate particles per unit area, in the cross section of the biocompatible composite may be about 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90%, or may fall within a range set by two values selected from among the % values described above. In a specific embodiment, the first average, which is the average of ratios of area occupied by the phosphorus atoms contained in the calcium phosphate particles per unit area, may be about 45%, 46%, 47%, 48%, 49%, 50%, 51 %, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, or 85%, or may fall within a range set by two values selected from among the % values described above. In a specific embodiment, the first average, which is the average of ratios of area occupied by the phosphorus atoms contained in the calcium phosphate particles per unit area, may be in a range of 50% to 85%.

In one embodiment, the first atom may be a phosphorus atom. In addition, the second standard deviation may be about 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.6, 0.7, 0.8, 0.9, 1, 1.1, 1.2, 1.3, 1.4, 1.5, 1.6, 1.7, 1.8, 1.9, 2, 2.2, 2.4, 2.6, 2.8, 3, 3.2, 3.4, 3.6, 3.8, 4, 4.2, 4.4, 4.6, 4.8, 5, 5.5, 6, 6.5, 7, 7.5, 8, 8.5, 9, 9.5, 10, 11, 12, 13, 14, 15, 16, 17, 18, 19, or 20, or may fall within a range set by two values selected from among the values described above. In one embodiment, the second standard deviation may be 0.5, 1, 1.5, 2, 2.5, 3, 3.5, 4, 4.5, 5, 6, 7, 8, 9, 10, 12, 14, 16, 18, or 20 or less. In a specific embodiment, the second standard deviation may be 10 or less. In a specific embodiment, the second standard deviation may be 5 or less. In a specific embodiment, the second standard deviation may be 2 or less. In a specific embodiment, the second standard deviation may be 1 or less. In a specific embodiment, the second standard deviation may be 0.5 or less.

In one embodiment, the first atom may be a magnesium atom. In addition, the first average, which is the average of ratios of area occupied by the magnesium atoms contained in the calcium phosphate particles per unit area, in the cross section of the biocompatible composite may be about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, or 60%, or may fall within a range set by two values selected from among the % values described above. In a specific embodiment, the first average, which is the average of ratios of area of the magnesium atoms contained in the calcium phosphate particles per unit area may be about 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, or 40%, or may fall within a range set by two values selected from among the % values described above. In a specific embodiment, the first average, which is the average of ratios of the area occupied by the magnesium atoms contained in the calcium phosphate particles per unit area, may be in a range of 15% to 45%.

In one embodiment, the first atom may be a magnesium atom. In addition, the second standard deviation may be about 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.6, 0.7, 0.8, 0.9, 1, 1.1, 1.2, 1.3, 1.4, 1.5, 1.6, 1.7, 1.8, 1.9, 2, 2.2, 2.4, 2.6, 2.8, 3, 3.2, 3.4, 3.6, 3.8, 4, 4.2, 4.4, 4.6, 4.8, 5, 5.5, 6, 6.5, 7, 7.5, 8, 8.5, 9, 9.5, 10, 11, 12, 13, 14, 15, 16, 17, 18, 19, or 20, or may fall within a range set by two values selected from among the values described above. In one embodiment, the second standard deviation may be 0.5, 1, 1.5, 2, 2.5, 3, 3.5, 4, 4.5, 5, 6, 7, 8, 9, 10, 12, 14, 16, 18, or 20 or less. In a specific embodiment, the second standard deviation may be 10 or less. In a specific embodiment, the second standard deviation may be 5 or less. In a specific embodiment, the second standard deviation may be 2 or less. In a specific embodiment, the second standard deviation may be 1 or less. In a specific embodiment, the second standard deviation may be 0.5 or less.

In one embodiment, the weight ratio of the calcium phosphate particles to the biocompatible composite may be in a range of 0.01 wt% or more and 60 wt% or less. In one embodiment, the weight ratio of the calcium phosphate particles to the biocompatible composite may be in a range of 0.1 wt% or more and 55 wt% or less. In a specific embodiment, the weight ratio of the calcium phosphate particles to the biocompatible composite may be in a range of 5 wt% or more and 50 wt% or less. In a specific embodiment, the weight ratio of the calcium phosphate particles to the biocompatible composite may be in a range of 10 wt% or more and 40 wt% or less.

In one embodiment, the weight ratio of the calcium phosphate particles to the biocompatible composite may be about 5 wt%, 6 wt%, 7 wt%, 8 wt%, 9 wt%, 10 wt%, 11 wt%, 12 wt%, 13 wt%, 14 wt%, 15 wt%, 16 wt%, 17 wt%, 18 wt%, 19 wt%, 20 wt%, 22 wt%, 24 wt%, 26 wt%, 28 wt%, 30 wt%, 32 wt%, 34 wt%, 36 wt%, 38 wt%, 40 wt%, 42 wt%, 44 wt%, 46 wt%, 48 wt%, or 50 wt%.

Hereinafter, the "biocompatible composite", according to one embodiment of the present application, will be described in a manner of comparing the same with a "composite" prepared by an existing technique.

The biocompatible composite, according to one embodiment of the present application, is a biocompatible composite, which may contain: the biodegradable polymer matrix; and the calcium phosphate dispersed in the biodegradable polymer matrix.

In this case, in the biocompatible composite according to one embodiment of the present application, measured by an analysis method using Image J, which will be described later, the average of ratios of area occupied by the calcium atoms contained in the calcium phosphate particles per unit area in the cross section of the biocompatible composite may be in a range of 53% to 85%. In addition, the standard deviation may be in a range of 0.8 to 8.7. On the other hand, in the case of the composite prepared by existing technique, especially a biocompatible composite prepared by mixing whitlockite powder with a PCL polymer dispersion or the like, in which whitlockite particles are dispersed in a PCL polymer matrix as described above, the average value of ratios of area occupied by the calcium atoms contained in the whitlockite particles per unit area in a cross section of the biocompatible composite may be about 64.6%. In addition, the standard deviation may be about 15.64.

For another example, in the case of the biocompatible composite in which the whitlockite particles are dispersed in the PCL polymer matrix, the average of ratios of area occupied by the magnesium atoms contained in the whitlockite particles per unit area in the cross section of the biocompatible composite may be about 27.4%. In addition, the standard deviation may be about 1.68. On the other hand, in the case of the composite prepared by the existing technique, especially the biocompatible composite prepared by mixing the whitlockite powder with the PCL polymer dispersion or the like, in which the whitlockite particles are dispersed in the PCL polymer matrix as described above, the average value of ratios of area occupied by the magnesium atom s contained in the whitlockite particles per unit area in the cross section of the biocompatible composite may be about 24.1%. In addition, the standard deviation may be about 4.348.

For another example, in the case of the biocompatible composite in which the whitlockite particles are dispersed in the PCL polymer matrix, the average of ratios of area occupied by the phosphorous atoms contained in the whitlockite particles per unit area in the cross section of the biocompatible composite may be about 84.5%. In addition, the standard deviation may be about 2.78. On the other hand, in the case of the composite prepared by the existing technique, especially the biocompatible composite prepared by mixing the whitlockite powder with the PCL polymer dispersion or the like, in which the whitlockite particles are dispersed in the PCL polymer matrix as described above, the average value of the ratios of area occupied by the phosphorous atoms contained in the whitlockite particles per unit area in the cross section of the biocompatible composite may be about 71.66%. In addition, the standard deviation may be about 12.788.

For another example, in the case of the biocompatible composite in which the hydroxyapatite particles are dispersed in the PCL polymer matrix, the average of ratios of area occupied by the calcium atoms contained in the calcium phosphate particles per unit area in the cross section of the biocompatible composite may be about 53.6%. In addition, the standard deviation may be about 0.83. On the other hand, in the case of a composite prepared by the existing technique, especially a biocompatible composite prepared by mixing hydroxyapatite powder with the PCL polymer dispersion or the like, in which hydroxyapatite particles are dispersed in a PCL polymer matrix as described above, the average of ratios of area occupied by the calcium atoms contained in the hydroxyapatite particles per unit area in the cross section of the biocompatible composite may be about 43%. In addition, the standard deviation may be about 1.71.

For another example, in the case of the biocompatible composite in which the hydroxyapatite particles are dispersed in the PCL polymer matrix, the average of ratios of area occupied by the phosphorus atoms contained in the calcium phosphate particles per unit area in the cross section of the biocompatible composite may be about 53.32%. In addition, the standard deviation may be about 0.3. On the other hand, in the case of the composite prepared by the existing technique, especially the biocompatible composite prepared by mixing the hydroxyapatite powder with the PCL polymer dispersion or the like, in which the hydroxyapatite particles are dispersed in the PCL polymer matrix as described above, the average of ratios of area occupied by the phosphorus atom contained in the hydroxyapatite particles per unit area in the cross section of the biocompatible composite may be about 45.95%. In addition, the standard deviation may be about 0.95.

It is confirmed that the biocompatible composite, according to one embodiment of the present application, is characterized by having a relatively small standard deviation of the ratio of the area occupied by the calcium phosphate particles per unit area, compared to the composites prepared by the existing technique.

Unlike in the existing preparation method of the composites, the biocompatible composite, according to one embodiment of the present application, is prepared by a process in which the dispersion-form calcium phosphate particles are mixed with the biodegradable polymer dispersion or biodegradable polymer solution. Specifically, in the process of preparing the biocompatible composite according to one embodiment of the present application, the calcium phosphate particles are sufficiently dispersed in the continuous phase before mixing the calcium phosphate particles and the biodegradable polymer. Therefore, compared to the existing technique in which calcium phosphate powder is directly mixed with a polymer, random aggregation between the calcium phosphate powders may be prevented. In addition, since random aggregation between the calcium phosphate powders occurs less, the calcium phosphate particles may be characterized in that the size thereof is regular. Furthermore, the difference in the degree to which each of the calcium phosphate particles is affected by gravity or the like may decrease. Through this, the calcium phosphate particles may be dispersed uniformly in each region in the biodegradable polymer matrix.

As the calcium phosphate particles are dispersed uniformly in each region in the biodegradable polymer matrix, a composite that obtains consistent quality, such as mechanical strength, bioactivity, or the like, in each region may be prepared.

In addition, when the composite is applied in vivo, the degree to which the calcium phosphate particles are released over time may be uniform. Therefore, when the biocomposite, according to one embodiment of the present application, is applied in vivo, control of the calcium phosphate particles to be released at a target location in a desired amount may be further facilitated.

The biocompatible composite, according to one embodiment of the present application, is a biocompatible composite, which may contain: the biodegradable polymer matrix; and the calcium phosphate dispersed in the biodegradable polymer matrix.

In this case, the biocompatible composite, according to one embodiment of the present application, may be characterized in that the weight ratio of the calcium phosphate particles to the biocompatible composite is in a range of 0.01 wt% or more and 55 wt% or less. In the biocompatible composite prepared according to a preferred embodiment of the present application, the weight ratio of the calcium phosphate particles to the biocompatible composite may be in a range of 5 wt% or more and 50 wt% or less.

For example, the biocompatible composite according to one embodiment, in which the whitlockite particles are dispersed in the PCL polymer matrix may be characterized in that the weight ratio of whitlockite to the biocompatible composite is in a range of 0.01 wt% or more and 55 wt% or less. On the other hand, the composite prepared by existing technique, especially the biocompatible composite prepared by mixing the whitlockite powder with the PCL polymer dispersion or the like as described above, is characterized in that the weight ratio of the whitlockite to the biocompatible composite is in a range of 10 wt% to 40 wt%.

For example, the biocompatible composite according to one embodiment, in which the hydroxyapatite particles are dispersed in the PCL polymer matrix, may be characterized in that the weight ratio of the hydroxyapatite to the biocompatible composite is 0.01 wt% or more and 55 wt% or less. On the other hand, composites prepared by existing techniques, especially biocompatible composites prepared by mixing hydroxyapatite powder with the PCL polymer dispersion or the like as described above, are characterized in that the weight ratio of the hydroxyapatite to the biocompatible composite is in a range of 10 wt% to 40 wt%.

The biocompatible composite, according to one embodiment of the present application, may be characterized in that the maximum value of the weight ratio of the calcium phosphate particles is greater than that of the composite prepared by the existing technique.

In addition, according to one embodiment of the present application, the biocompatible composite with varying weight ratios of calcium phosphate may be provided. In other words, according to one embodiment of the present application, the biocompatible composite having the weight ratio of calcium phosphate that has failed to be obtained by existing techniques may be provided.

In the case of the existing composites, calcium phosphate powder is mixed with a biodegradable polymer dispersion or the like to prepare the composites. In this case, as described above, there is a strong force with which the powder-form calcium phosphate particles aggregate to each other. Therefore, the calcium phosphate particles, serving as the dispersed phase, may be insufficiently dispersed in the liquid continuous phase of the calcium phosphate-biodegradable polymer dispersion. In addition, due to the strong force with which the calcium phosphate powders aggregate to each other, the calcium phosphate particles may be insufficiently mixed with the biodegradable polymer matrix in the calcium phosphate-biodegradable polymer dispersion phase.

In other words, to facilitate the calcium phosphate particles to be well-mixed with the calcium phosphate-biodegradable polymer dispersion, the attraction between the calcium phosphate particles and the liquid continuous phase of the calcium phosphate-biodegradable polymer dispersion is required to reach a predetermined level or the above. However, when directly mixing the powder-form calcium phosphate particles with the polymer dispersion, the force with which the calcium phosphate particles aggregate to each other is more dominant than the attraction between the calcium phosphate particles and the liquid continuous phase of the calcium phosphate-biodegradable polymer dispersion. For this reason, it is assumed that the mixing is insufficiently performed.

Similarly, to facilitate the calcium phosphate particles to be well-dispersed in the biodegradable polymer matrix on the calcium phosphate-biodegradable polymer dispersion, the attraction between the calcium phosphate particles and the biodegradable polymer particles is required to reach a predetermined level or the above. However, when preparing the composite by directly mixing the powder-form calcium phosphate particles with the polymer dispersion, the force with which the calcium phosphate particles aggregate to each other is more dominant than the attraction between the calcium phosphate particles and the biodegradable polymer. For this reason, it is assumed that the calcium phosphate particles are insufficiently mixed with the biodegradable polymer matrix.

On the other hand, as described above, the biocompatible composite of the present application is prepared by the process of mixing the dispersion-form calcium phosphate particles with the biodegradable polymer dispersion or biodegradable polymer solution. In other words, the calcium phosphate particles in the dispersion form, not in the powder form, are mixed with the biodegradable polymer dispersion or the like. Therefore, the calcium phosphate particles are mixed with the biodegradable polymer dispersion while the force with which the calcium phosphate particles aggregate to each other is relatively weaker than that in the case of the existing technique. In this case, the attraction (or affinity) between the calcium phosphate particles and the liquid continuous phase of the calcium phosphate-biodegradable polymer dispersion or the attraction (or affinity) between the calcium phosphate particles and the biodegradable polymer on the calcium phosphate-biodegradable polymer dispersion is relatively stronger than that in the case of the existing technique. For this reason, it is assumed that the mixing or dispersion is well facilitated.

As described above, the biocompatible composite, according to one embodiment of the present application, may be characterized in that the calcium phosphate is contained in a relatively higher weight ratio than that of the existing biocompatible composite. Therefore, the biocompatible composite, according to one embodiment of the present application, is capable of delivering calcium phosphate particles as a high content into the body and thus may have excellent performance, such as osteogenesis ability, osteoconductivity, and the like.

In addition, according to one embodiment of the present application, the biocompatible composite with varying weight ratios of calcium phosphate may be provided. In other words, the biocompatible composite with varying weight ratios of calcium phosphate may be prepared by the method according to one embodiment provided herein. This enables a biocompatible composite with an appropriate weight ratio of calcium phosphate to be selected for use depending on the purposes. For example, when there is a target release profile of calcium phosphate in vivo, a biocompatible composite material with an appropriate weight ratio of calcium phosphate suitable for the target release profile may be provided.

The biocompatible composite, according to one embodiment of the present application, may be prepared in various forms (for example, a plate-like form, a screw-like form, and the like) related to "bones", which may be applicable in vivo, to be usable in any appropriate area.

For example, the biocompatible composite, according to one embodiment of the present application, may be applied to bone graft materials. Specifically, the biocompatible composite, according to one embodiment of the present application, may be applied to supplements for bone loss.

For another example, the biocompatible composite, according to one embodiment of the present application, may be used to manufacture products, such as bones for transplantation, artificial teeth, periodontal tissue regeneration materials, artificial joints, or the like. In this case, the biocompatible composite may be molded to be shaped into an appropriate form depending on the areas to which the product manufactured using the biocompatible composite is applied.

For another example, the biocompatible composite, according to one embodiment of the present application, may be applied to screws for fixation.

In addition, the biocompatible composite, according to one embodiment of the present application, may be usable as a raw material for 3D printing to manufacture bone graft materials, supplements for bone loss, and the like that are applicable in vivo.

## 3. Preparation example of biocompatible composite

### (1) Preparation Example 1 (preparation example of specimen No. 1)

1) Preparation of PCL polymer dispersion (preparation of biodegradable polymer dispersion)

A PCL polymer dispersion in which PCL polymer particles were dispersed in an ethanol phase was prepared by putting 200 ml of 99.9% ethanol (EtOH) and 21 g of a bead-type PCL polymer into a reaction tank and then heating the resulting product to a temperature of 80°C for about 1 hour while operating an impeller. In this case, the temperature of 80°C was selected as a temperature for partially melting the PCL polymer by transferring heat to the PCL polymer using ethanol as a heat medium.

2) Preparation of second whitlockite dispersion (preparation of second calcium phosphate dispersion)

A phosphate solution was added to a mixed solution containing calcium ions and magnesium ions using water as a liquid continuous phase. The resulting solution was allowed to react and be aged while adjusting the pH to precipitate 9 g of whitlockite, which was washed with 0.225 L of D.W to prepare a first whitlockite dispersion (preparation of first calcium phosphate dispersion).

Specifically, the first whitlockite dispersion was prepared according to a preparation method of whitlockite by spatial separation and/or temporal separation disclosed in Korean Patent Nos. 2116206 and 2084896.

Water which is the continuous phase of the first whitlockite dispersion was mixed with 0.075L of ethanol (EtOH) that is different from water, and water was partially removed from ethanol by centrifugation with 500 relative centrifugal force (RCF) for 3.3 min. The centrifugation was repeated 3 times. In addition, excess ethanol was partially removed to prepare a second whitlockite dispersion in which a ratio of whitlockite to ethanol was 1 g/10 ml.

3) Mixing of second whitlockite dispersion and PCL polymer dispersion (preparation of calcium phosphate-biodegradable polymer dispersion)

The second whitlockite dispersion was added to a reaction tank containing the PCL polymer dispersion. Then, whitlockite particles and the PCL polymer dispersion were mixed for 30 minutes while operating an impeller to prepare a whitlockite-PCL polymer dispersion in which the whitlockite particles and the PCL polymer particles were dispersed in the ethanol phase. In addition, a portion of ethanol contained in the reaction tank was evaporated by being heated to 80°C while operating the impeller.

In this case, the mixing time was set to 30 minutes when the volume of ethanol contained in the reaction tank was based on 100 ml with respect to 30 g of a composite to be prepared.

4) Drying of whitlockite-PCL polymer dispersion

The whitlockite-PCL polymer dispersion was dried for 12 hours while applying heat thereto to 80°C using a dry oven to prepare a whitlockite-PCL polymer composite in a cubic form by a casting method. Then, the whitlockite-PCL polymer composite in the cubic form was cut to prepare a biocompatible composite in a pellet form.

5) Compression molding

3.5g of the biocompatible composites in the pellet form were poured into a mold and then the mold was heated at about 200°C. And then thermal compression molding in which a pressure of about 30 MPa was applied to the biocompatible composite in the pellet form. Through the thermal compression molding, specimen No. 1 in a plate form with a size of 40 * 25 (mm), to be used for SEM analysis, was prepared.

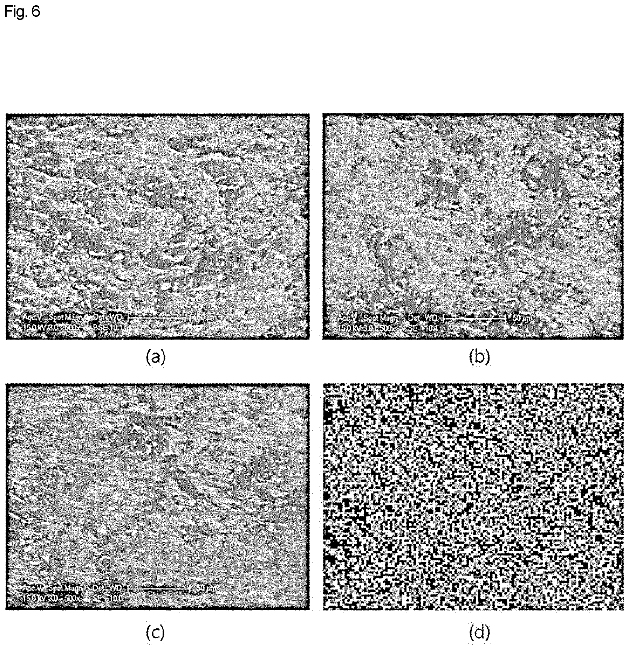

Referring to FIG. 6,

FIGS. 6A, 6B, and 6C show SEM images of specimen No. 1, the images obtained by an analysis method to be described later. Specifically, FIGS. 6A, 6B, and 6C show SEM images taken at 500x magnification for each cross section of synthesized specimen No. 1.

FIG. 6D shows an image displaying the portion occupied by calcium atom s in specimen No. 1, the image obtained by the analysis method to be described later. Specifically, Image J analysis was performed on the calcium atoms using FIG. 6D.

Referring to FIGS. 6A, 6B, 6C, and 6D, it is confirmed that the biocompatible composite in which the whitlockite particles are dispersed in a PCL polymer matrix is enabled to be synthesized by the preparation method of the biocompatible composite of the present application, which includes separately preparing the second whitlockite dispersion (using EtOH as the continuous phase) and the PCL polymer dispersion and then mixing the two.

**(2) Preparation Example 2 (preparation example of specimen No. 2)**

A phosphate solution was added to a solution containing calcium ions using water as a liquid continuous phase. The pH of the solution was adjusted and the resulting solution was allowed to react and be aged to precipitate calcium phosphate particles, thereby preparing a first hydroxyapatite dispersion (preparation of first calcium phosphate dispersion).

Specifically, 10 mmol of calcium nitrate $Ca(NO_3)_2*4H_2O$ (about 1.5601 g), 10 mmol of sodium phosphate ($NaH_2PO_4*2H_2O$) (about 1.5601 g), and 20 mmol of urea (about 1.2012 g) were added to 500 ml of water.

The pH was adjusted to fall within a pH range of about 12 to 13 by sequentially adding 2.8 mL of sodium hydroxide (NaOH) dropwise.

Then, the resulting product was allowed to be aged at room temperature for 24 hours to prepare 500 ml of a first hydroxyapatite dispersion containing 10 g of hydroxyapatite particles.

Next, the first hydroxyapatite dispersion was prepared into a second hydroxyapatite dispersion (second calcium phosphate dispersion) by substituting the water, serving as the continuous phase of the first hydroxyapatite dispersion, with ethanol in the same manner as in Preparation Example 1.

In addition, the second hydroxyapatite dispersion was mixed with a PCL polymer dispersion prepared in the same manner as in Preparation Example 1 (preparation of calcium phosphate-biodegradable polymer dispersion).

Thereafter, specimen No. 2 in a plate form with a size of 40 * 25 (mm), to be used for SEM analysis, was prepared in the same manner as in Preparation Example 1 through the drying and compression molding processes.

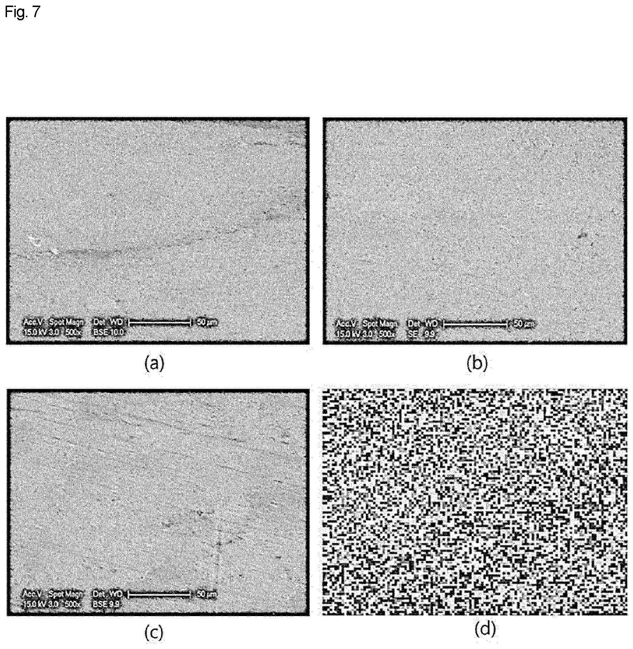

Referring to FIG. 7,

FIGS. 7A, 7B, and 7C show SEM images of specimen No. 2, the images obtained by an analysis method to be described later. Specifically, FIGS. 7A, 7B, and 7C show SEM images taken at 500x magnification for each cross section of synthesized specimen No. 2.

FIG. 7D shows an image displaying the portion occupied by calcium atoms in the cross section of specimen No. 2. Specifically, Image J analysis was performed on the calcium atoms using FIG. 7D.

Referring to FIGS. 7A, 7B, 7C, and 7D, it is confirmed that the biocompatible composite in which the hydroxyapatite particles are dispersed in a PCL polymer matrix is enabled to be synthesized by the preparation method of the biocompatible composite of the present application, which includes separately preparing the second hydroxyapatite dispersion (using EtOH as the continuous phase) and the PCL polymer dispersion and then mixing the same.

**(3) Preparation Example 3 (preparation example of specimen No. 3)**

1) Preparation of PCL polymer solution (preparation of biodegradable polymer dispersion)

A PCL polymer and chloroform were ground by putting 500 ml of 99.9% chloroform ($CHCl_3$) and a bead-type PCL polymer into a Duran bottle and then firstly stirring the resulting product for 30 minutes using an impeller at 200 rpm while using an ultrasonicator under the condition of 750 watts. Secondly, additional stirring was performed for 2 hours using an impeller at 200 rpm to prepare a PCL polymer solution in which PCL polymer particles were dissolved in chloroform.

2) Preparation of second whitlockite dispersion (preparation of second calcium phosphate dispersion)

A first whitlockite dispersion was prepared in the same manner as described above in Preparation Example 1.

Water which is continuous phase of the first whitlockite dispersion was mixed with 5L of ethanol that is different from water, and water was partially removed from ethanol by centrifugation with 500RCF for 10 min. The centrifugation was repeated 3 times. In addition, excess ethanol was partially removed. Furthermore, sonication was additionally performed on the second whitlockite dispersion for 30 minutes at 120 kHz to completely disperse the whitlockite particles in the ethanol continuous phase to prepare the second whitlockite dispersion in which a ratio of whitlockite to ethanol was 1 g/10 ml.

3) Mixing of second whitlockite dispersion and PCL polymer solution (preparation of calcium phosphate-biodegradable polymer dispersion)

The second whitlockite dispersion was added to the Duran bottle containing the PCL polymer solution. Then, the resulting product in the Duran bottle was stirred for 30 minutes at 200 rpm using a magnetic bar to prepare a whitlockite-PCL polymer dispersion in which the whitlockite particles and the PCL polymer particles were dispersed in the chloroform phase. In addition, the whitlockite-PCL polymer mixture was mixed at 200 rpm while simultaneously applying heat thereto at 60°C.

In this case, the stirring time was set to 30 minutes when the volume of chloroform contained in the Duran bottle was based on 500 ml with respect to 70 g of a composite to be prepared.

4) Drying of whitlockite-PCL polymer dispersion

The whitlockite-PCL polymer dispersion was thinly spread on a die and dried at a temperature of 80°C for 24 hours to prepare a whitlockite-PCL polymer composite in a sheet form. Then, the whitlockite-PCL polymer composite in the sheet form was cut to prepare a biocompatible composite in a pellet-like form.

5) Compression molding

About 3g to 3.5g of the biocompatible composites in the pellet form were poured into a mold, and then the mold was heated at about 200°C. And then thermal compression molding in which a pressure of about 30 MPa was applied to the biocompatible composite in the pellet form. Through the thermal compression molding, specimen No. 3 in a plate form with a size of 40 * 25 (mm), to be used for SEM analysis, was prepared.

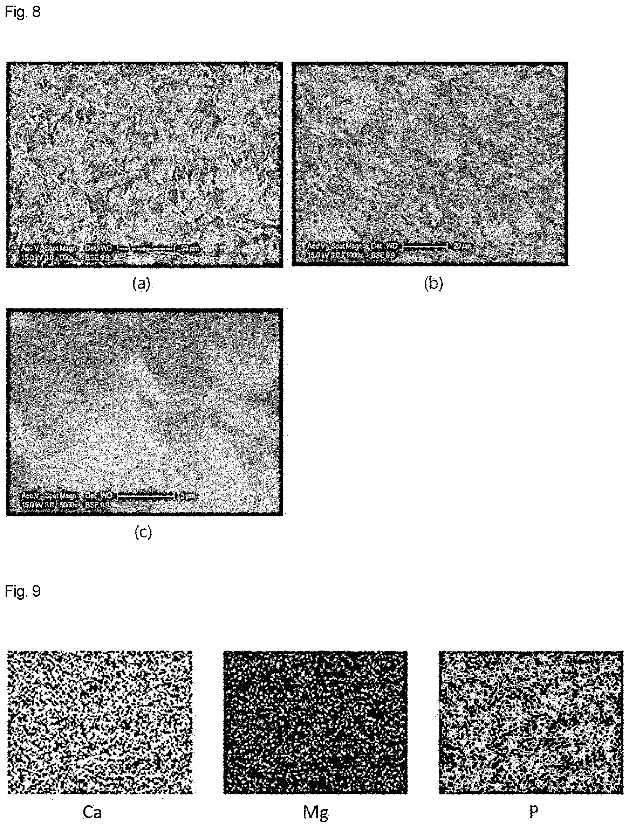

Referring to FIG. 8, FIGS. 8A, 8B, and 8C show SEM images of specimen No. 3, the images obtained by an analysis method to be described later. Specifically, FIGS. 8A, 8B, and 8C show SEM images taken at 500x, 1000x, and 5000x magnifications, respectively, for cross section of the synthesized biocompatible composite.

FIG. 9 shows images displaying the portions occupied by calcium, magnesium, and phosphorus atoms in specimen No. 3, the image obtained by an analysis method to be described later. Specifically, Image J analysis was performed on each of the calcium, magnesium, and phosphorus atoms using FIG. 9.

Referring to FIGS. 8A, 8B, 8C, and 9, it is confirmed that the biocompatible composite in which the whitlockite particles are dispersed in a PCL polymer matrix is enabled to be synthesized by the preparation method, which includes separately preparing the second whitlockite dispersion and the PCL polymer solution containing chloroform as the solvent, and then mixing the two. In other words, it was confirmed that the whitlockite-PCL polymer composite was enabled to be prepared even when using the PCL polymer solution containing chloroform as the solvent.

(4) Preparation Example 4 (preparation example of specimen No. 4)

A phosphate solution was added to a solution containing calcium ions using water as a liquid continuous phase. The pH of the solution was adjusted. The resulting solution was allowed to react and be aged to precipitate calcium phosphate particles, thereby preparing a first hydroxyapatite dispersion.

Specifically, 10 mmol of calcium nitrate $Ca(NO_3)_2 \cdot 4H_2O$ (about 1.5601 g), 10 mmol of sodium phosphate $(NaH_2PO_4 \cdot 2H_2O)$ (about 1.5601 g), and 20 mmol of urea (about 1.2012 g) were added to 500 ml of water.

The pH was adjusted to fall within a range of about 12 to 13 by adding 2.8 mL of sodium hydroxide (NaOH) dropwise.

Then, the resulting product was allowed to be aged at room temperature for 24 hours to prepare 500 ml of a first hydroxyapatite dispersion containing 10 g of hydroxyapatite particles (preparation of first calcium phosphate dispersion).

Next, the first hydroxyapatite dispersion was prepared into a second hydroxyapatite dispersion by substituting the water, serving as the continuous phase of the first hydroxyapatite dispersion, with ethanol in the same manner as described above in Preparation Example 3 (preparation of second calcium phosphate dispersion).

In addition, the second hydroxyapatite dispersion was mixed with the PCL polymer solution prepared in the same manner as in Preparation Example 3. Thereafter, specimen No. 4 in a plate form with a size of 40 * 25 (mm), to be used for SEM analysis, was prepared in the same manner as in Preparation Example 3 through the drying and compression molding processes (preparation of calcium phosphate-biodegradable polymer dispersion).

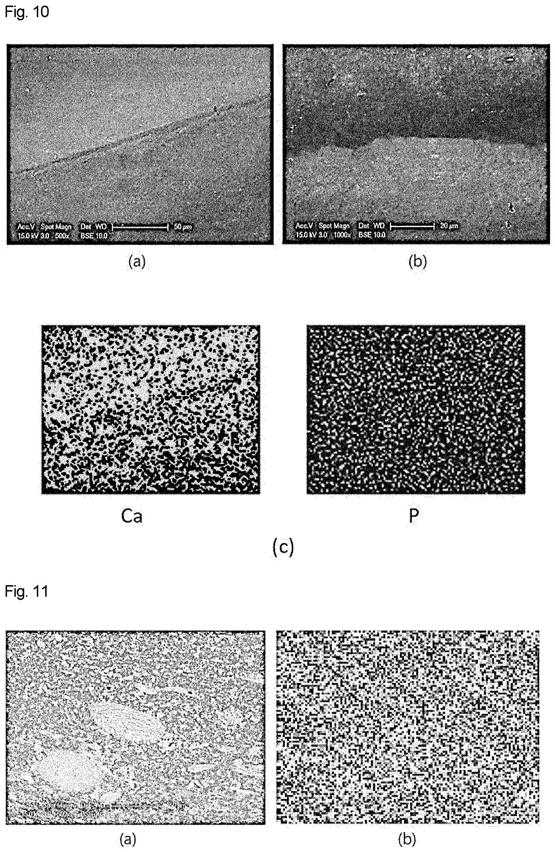

Referring to FIG. 10,
FIGS. 10A and 10B show SEM images of specimen No. 4, the images obtained by an analysis method to be described later. Specifically, FIGS. 10A and 10B show SEM data taken at 500x and 1000x magnifications, respectively, for each cross section of synthesized specimen No. 4.

FIG. 10C shows an image displaying the portions occupied by calcium and phosphorus atoms in specimen No. 4. Specifically, Image J analysis was performed on each of the calcium and phosphorus atoms in specimen No. 4 using FIG. 10C.

Referring to FIGS. 10A, 10B, and 10C, it is confirmed that the biocompatible composite in which the hydroxyapatite particles are dispersed in a PCL polymer matrix is enabled to be synthesized by the preparation method, which includes separately preparing the second hydroxyapatite dispersion and the PCL polymer solution containing "chloroform" as the solvent, and then mixing the two. In other words, it was confirmed that the hydroxyapatite-PCL polymer composite was enabled to be prepared even when using the PCL polymer solution containing "chloroform" as the solvent.

**(5) Preparation Example 5 (preparation example of specimen No. 5)**

A PLLA polymer and a chloroform solvent were put into a reaction tank and then mixed to completely dissolve the PLLA polymer in the chloroform solvent, thereby preparing a PLLA polymer solution (preparation of biodegradable polymer dispersion).

Then, ultrasonication was performed for 30 minutes on a second whitlockite dispersion prepared in the same manner as described above in Preparation Example 3.

Next, the PLLA polymer solution and the sonicated second whitlockite dispersion were mixed. Then, the resulting product was mixed at 200 rpm for 30 minutes to prepare a whitlockite-PLLA polymer dispersion (preparation of calcium phosphate-biodegradable polymer dispersion).

Subsequently, the whitlockite-PLLA polymer dispersion was dried in the same manner as described above in Preparation Example 3 to prepare a whitlockite-PLLA polymer composite in a sheet form. Then, the whitlockite-PLLA polymer composite in the sheet form was cut to prepare a biocompatible composite in a pellet form.

Thereafter, the prepared pellet formed biocompatible composites are poured into a mold, and the mold was heated to about 200°C. Thermal compression molding in which a pressure of about 30 MPa was applied to the biocompatible composites in the pellet form as described above in Preparation Example 3. Through the thermal compression molding, specimen No. 5 in a plate form with a size of 40 * 25 (mm), to be used for SEM analysis, was prepared.

Referring to FIGS. 11A and 11B,
FIG. 11A shows an SEM image of specimen No. 5, the image obtained by an analysis method to be described later. Specifically, FIG. 11A shows an SEM image taken at 500x magnification for the cross section of the synthesized biocompatible composite.

FIG. 11B shows an image displaying the portion occupied by calcium atoms in specimen No. 5. Image J analysis was performed on the calcium atoms using FIG. 11B.

Referring to FIGS. 11A and 11B, it is confirmed that the biocompatible composite in which the whitlockite particles are dispersed in a PLLA polymer matrix is enabled to be synthesized by the preparation method, which includes separately preparing the second whitlockite dispersion and the "PLLA polymer solution" containing chloroform as the solvent, and then mixing the two. Even when using the "PLLA" polymer solution, it was confirmed that the preparation method of the biocompatible composite disclosed herein was applied, thereby preparing the whitlockite-PLLA polymer composite.

In addition, considering that the preparation method disclosed herein is applied not only to the PCL polymer, which is a biodegradable polymer, but also to the PLLA polymer, biocompatible composites may be enabled to be prepared even when using various biodegradable polymers, including a PLGA polymer as well as the PLLA polymer and the PCL polymer.

**(6) Preparation Example 6 (preparation example of specimens Nos. 6-1 and 6-2)**

1) Preparation of specimen No. 6-1

A bead-type PCL polymer and a dichloromethane (DCM) solvent were added to a reaction tank, where a ratio of the PCL polymer to the DCM solvent was 10%, and then mixed, thereby preparing a PCL polymer solution in which the PCL polymer was completely dissolved in the DCM solvent (preparation of biodegradable polymer dispersion).

Then, ultrasonication was performed on a second whitlockite dispersion prepared in the same manner as described above in Preparation Example 3 for 30 minutes.

Next, the PCL polymer solution and the sonicated second whitlockite dispersion were mixed. Then, the resulting product was mixed at 200 rpm for 30 minutes to prepare a whitlockite-PCL polymer dispersion (preparation of calcium phosphate-biodegradable polymer dispersion).

Subsequently, the whitlockite-PCL polymer dispersion was dried in the same manner as described above in Preparation Example 3 to prepare a whitlockite-PCL polymer composite in a sheet form. Then, the whitlockite-PCL polymer composite in the sheet form was cut to prepare a biocompatible composite in a pellet form.

Thereafter, the pellet formed biocompatible composites are poured into a mold, and the mold was heated to about 200°C. Thermal compression molding in which a pressure of about 30 MPa was applied to the biocompatible composite in the pellet form as described above in Preparation Example 3. Through the thermal compression molding, specimen No. 6-1 in a plate form with a size of 40 * 25 (mm), to be used for SEM analysis, was prepared.

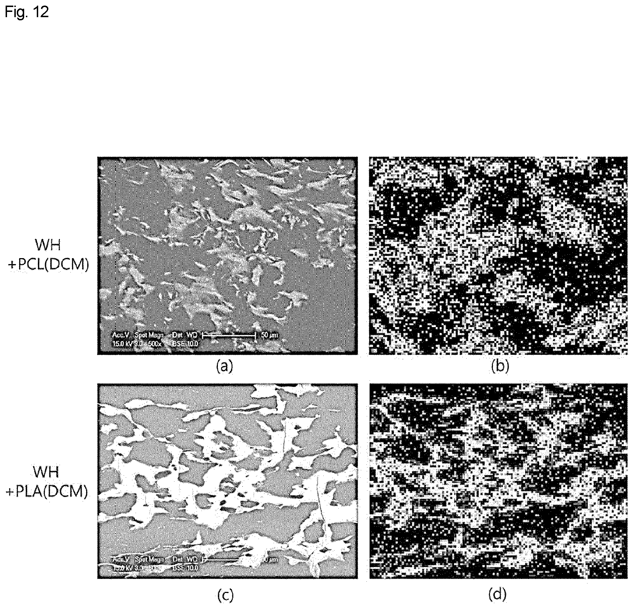

Referring to FIGS. 12A and 12B,
FIG. 12A shows an SEM image of specimen No. 6-1, the image obtained by an analysis method to be described later. Specifically, FIG. 12A shows an SEM image taken at 500x magnification for the cross section of the synthesized biocompatible composite.

FIG. 12B shows an image displaying the portion occupied by calcium atoms in specimen No. 6-1. Specifically, FIG. 12B was used to perform Image J analysis on the calcium atoms.

Referring to FIGS. 12A and 12B, it is confirmed that the biocompatible composite in which the whitlockite particles are dispersed in a PCL polymer matrix is enabled to be synthesized by the preparation method, which includes separately preparing the second whitlockite dispersion and the PCL polymer solution containing "DCM" as the solvent, and then mixing the two. In other words, it was confirmed that the whitlockite-PCL polymer composite was enabled to be prepared even when using the PCL polymer solution containing DCM as the solvent.

In addition, considering that chloroform and DCM are organic solvents with similar properties, whitlockite-PCL polymer composites may be enabled to be prepared even when using organic solvents (for example, tetrahydrofuran (THF), dimethylformamide (DMF), dimethyl sulfoxide (DMSO), and acetone) other than chloroform and DCM, as PCL polymer solvents or PCL polymer dispersion.

However, in the case of dissolving the PCL polymer using the DCM solvent, it was confirmed that the whitlockite particles were relatively less homogeneously dispersed in the PCL polymer matrix than in the case of dissolving the PCL polymer using chloroform, as described with reference to FIG. 11. This is presumed to be because DCM has a relatively lower solubility than chloroform in ethanol which is the liquid continuous phase contained in the second whitlockite dispersion. Through these results, it was confirmed that the solubility between the liquid continuous phase (or solvent) of the biodegradable polymer dispersion (or biodegradable polymer solution) and the liquid continuous phase of the second calcium phosphate dispersion had a significant effect on the uniformity with which the calcium phosphate particles are dispersed in the biodegradable polymer matrix.

2) Preparation of specimen No. 6-2

A PLLA polymer and a dichloromethane (DCM) solvent were added to a reaction tank, where a ratio of the PLLA polymer to the DCM solvent was 10%, and then mixed, thereby preparing a PLLA polymer solution in which the PCL polymer was completely dissolved in the DCM solvent (preparation of biodegradable polymer dispersion).

Then, ultrasonication was performed for 30 minutes on a second whitlockite dispersion prepared in the same manner as described above in Preparation Example 3.

Next, the PLLA polymer solution and the sonicated second whitlockite dispersion were mixed. Then, the resulting mixture was mixed at 200 rpm for 30 minutes to prepare a whitlockite-PLLA polymer dispersion (preparation of calcium phosphate-biodegradable polymer dispersion).

Subsequently, the whitlockite-PLLA polymer dispersion was dried in the same manner as described above in Preparation Example 3 to prepare a whitlockite-PLLA polymer composite in a sheet form. Then, the whitlockite-PLLA polymer composite in the sheet form was cut to prepare a biocompatible composite in a pellet form.

Thereafter, the pellet formed biocompatible composites are poured into a mold, and the mold was heated to about 200°C. Thermal compression molding in which a pressure of about 30 MPa was applied to the biocompatible composite in the pellet form as described above in Preparation Example 3. Through the thermal compression molding, specimen No. 6-2 in a plate form with a size of 40 * 25 (mm), to be used for SEM analysis, was prepared.

Referring to FIGS. 12C and 12D,

FIG. 12C shows an SEM image of specimen No. 6-2, the image obtained by an analysis method to be described later. Specifically, FIG. 12C shows an SEM image taken at 500x magnification for the cross section of the synthesized biocompatible composite.

FIG. 12D shows an image displaying the portion occupied by calcium atoms in specimen No. 6-2. Specifically, FIG. 12D was used to perform Image J analysis on the calcium atoms.

Referring to FIGS. 12C and 12D, it is confirmed that the biocompatible composite in which the whitlockite particles are dispersed in a PLLA polymer matrix is enabled to be synthesized by the preparation method, which includes separately preparing the second whitlockite dispersion and the PLLA polymer dispersion (or solution) containing "DCM" as the liquid continuous phase (or solvent), and then mixing the two. In other words, it was confirmed that the whitlockite-PLLA polymer composite was enabled to be prepared even when using the "PLLA" polymer dispersion containing "DCM" as the liquid continuous phase.

In addition, considering that the preparation method disclosed herein is applied not only to the PCL polymer but also to the PLLA polymer, biocompatible composites may be enabled to be prepared even when using biodegradable polymers including a PLGA polymer as well as the PLLA polymer and the PCL polymer.

(7) Preparation Example 7 (preparation example of specimens Nos. 7-1 and 7-2)

A total of 30 g of a biocompatible composite was prepared in the same manner as described above in Preparation Example 1. However, experimental conditions were adjusted so that the amount of whitlockite crystals contained in the first whitlockite dispersion was set to 3 g, thereby preparing specimen No. 7-1.

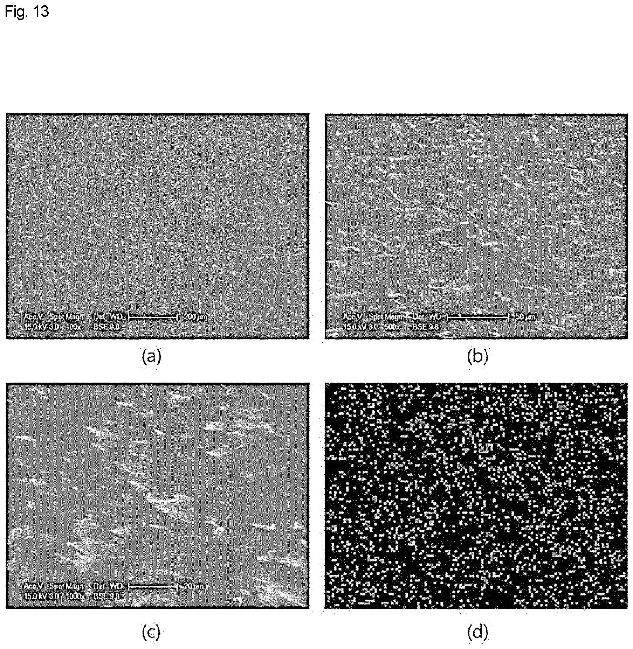

Referring to FIG. 13,

FIGS. 13A, 13B, and 13C show SEM images of specimen No. 7-1, the images obtained by an analysis method to be described later. Specifically, FIGS. 13A, 13B, and 13C show SEM images taken at 100x, 500x, and 1000x magnifications, respectively, for each cross section of synthesized specimen No. 7-1.

FIG. 13D shows an image displaying the portion occupied by magnesium atoms in specimen No. 7-1. Specifically, FIG. 13D was used to perform Image J analysis on the magnesium atoms.

Referring to FIGS. 13A, 13B, 13C, and 13D, even when the weight ratio of the whitlockite particles to the entire biocompatible composite is 10 wt%, the biocompatible composite in which the whitlockite particles are dispersed in a PCL polymer matrix is enabled to be prepared by the preparation method of the biocompatible composite according to one embodiment of the present application.

In addition, a total of 30 g of the biocompatible composite was prepared in the same manner as described above in Preparation 1. However, experimental conditions were adjusted so that the amount of whitlockite crystals contained in the first whitlockite dispersion was set to 15 g, thereby preparing specimen No. 7-2.

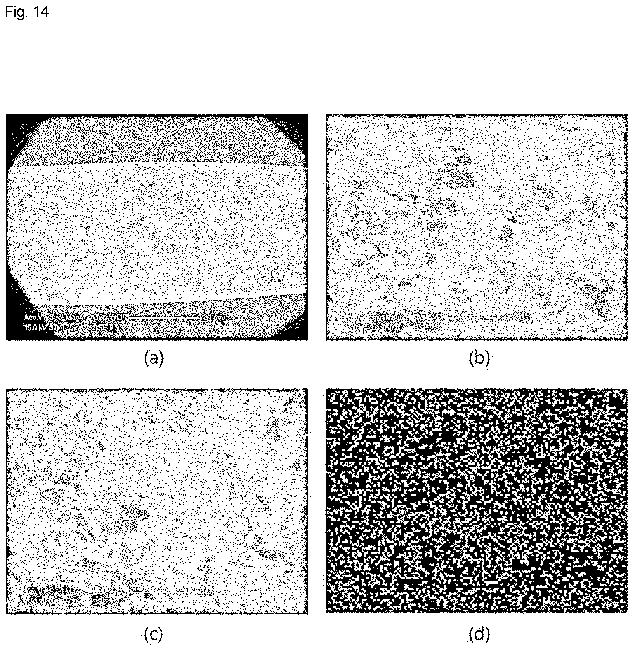

Referring to FIG. 14,

FIGS. 14A, 14B, and 14C show SEM images of specimen No. 7-2, the images obtained by an analysis method to be described later. Specifically, 14A, 14B, and 14C show SEM images taken at 100x, 500x, and 1000x magnifications, respectively, for each cross section of synthesized specimen No. 7-2.

FIG. 14D shows an image displaying the portion occupied by magnesium atoms in specimen No. 7-2. Specifically, FIG. 14D was used to perform Image J analysis on the magnesium atoms.

Referring to FIGS. 14A, 14B, 14C, and 14D, even when the weight ratio of the whitlockite particles to the entire biocompatible composite is 50 wt%, the biocompatible composite in which the whitlockite particles are dispersed in a PCL polymer matrix is enabled to be prepared by the preparation method of the biocompatible composite according to one embodiment of the present application.

Referring to Preparation Example 1 and Preparation Example 7, the biocompatible composite, according to one embodiment of the present application, may be prepared such that the weight ratio of the whitlockite particles contained in the biocompatible composite is characterized by falling within a range of 0.01 wt% or more and less than 55 wt% and preferably, 5 wt% or more and less than 55 wt%.

**(8) Preparation Example 8 (preparation example of specimens Nos. 8-1 to 8-4)**

A total of 33.3 g of a biocompatible composite was prepared in the same manner as described above in Preparation Example 2. However, experimental conditions were adjusted so that the amount of hydroxyapatite crystals contained in the first hydroxyapatite dispersion was set to 6.67 g, thereby preparing specimen No. 8-1.

In addition, a total of 33.3 g of a biocompatible composite was prepared in the same manner as described above in Preparation Example 2. In this case, experimental conditions were adjusted so that the amount of hydroxyapatite crystals contained in the first hydroxyapatite dispersion was set to 10 g, thereby preparing specimen No. 8-2.

In addition, a total of 33.3 g of a biocompatible composite was prepared in the same manner as described above in Preparation Example 2. However, experimental conditions were adjusted so that the amount of hydroxyapatite crystals contained in the first hydroxyapatite dispersion was set to 13.33 g, thereby preparing specimen No. 8-3.

In addition, a total of 33.3 g of a biocompatible composite was prepared in the same manner as described above in Preparation Example 2. However, experimental conditions were adjusted so that the amount of hydroxyapatite crystals contained in the first hydroxyapatite dispersion was 16.67 g, thereby preparing specimen No. 8-4.

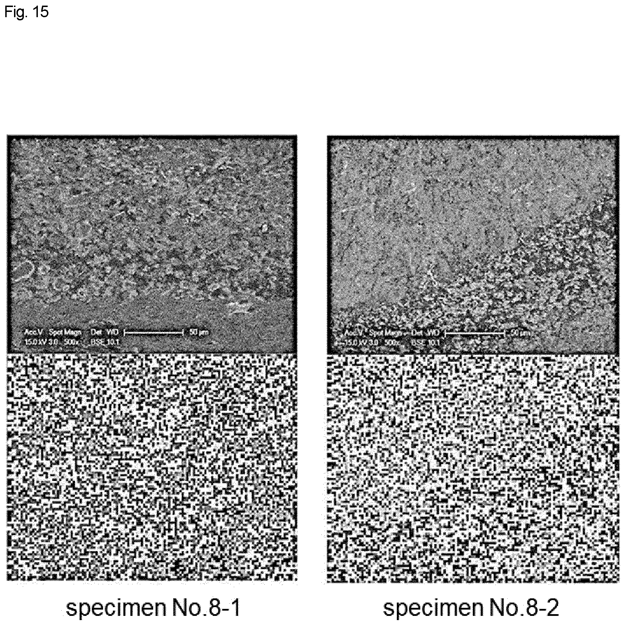

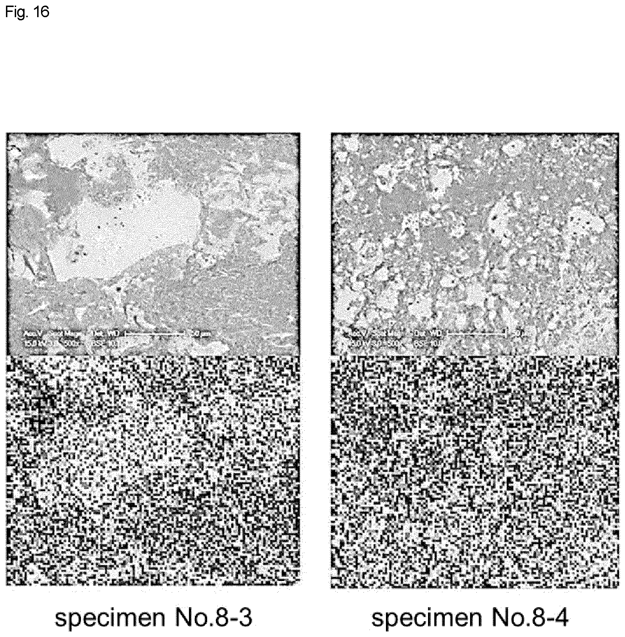

Referring to FIGS. 15 and 16,

FIG. 15 shows SEM images of specimens Nos. 8-1 and 8-2 prepared in Preparation Example 8, the images obtained by an analysis method to be described later, and images displaying the portions occupied by calcium atom s in each of specimens Nos. 8-1 and 8-2. These images were used to perform Image J analysis of the calcium atoms contained in each specimen.

FIG. 16 shows SEM images of specimens Nos. 8-3 and 8-4 prepared in Preparation Example 8, the images obtained by an analysis method to be described later, and images displaying the portions occupied by calcium atom s in each of specimens Nos. 8-3 and 8-4. These images were used to perform Image J analysis of the calcium atoms contained in each specimen.

Referring to FIGS. 15 and 16, even when the weight ratio of the hydroxyapatite particles to the entire biocompatible composite is either one of 20 wt%, 30 wt%, 40 wt%, or 50 wt%, it is confirmed that the biocompatible composite in which the hydroxyapatite particles are dispersed in a PCL polymer matrix is enabled to be prepared by the preparation method of the biocompatible composite according to one embodiment of the present application.

Therefore, it was proved by Preparation Example 8 that the biocompatible composite, according to one embodiment of the present application, was enabled to be prepared such that the weight ratio of the hydroxyapatite particles contained in the biocompatible composite was characterized by falling within a range of 0.01 wt% or more and less than 55 wt% and preferably, 5 wt% or more and less than 55 wt%.

**(9) Preparation Example 9 (preparation example of specimen No. 9)**

1) Preparation of PCL polymer dispersion (preparation of biodegradable polymer dispersion)

After putting 5 L of 99.9% chloroform ($CHCl_3$) and 700 g of a particle-type PCL polymer into a 10-L beaker, the resulting product was stirred at room temperature using an impeller at 200 RPM for 2 hours and then completely dissolved.

2) Preparation of second whitlockite dispersion (preparation of second calcium phosphate dispersion)

After putting 300 g of whitlockite powder and 2 L of 99.9% ethanol (EtOH) in a 10-L beaker, the resulting product was stirred for 30 minutes by setting an impeller and a sonicator under conditions of 200 RPM and 750 Watt, respectively, at room temperature to prepare the second whitlockite dispersion.

3) Mixing of second whitlockite dispersion and PCL polymer dispersion (preparation of calcium phosphate-biodegradable polymer dispersion)

The second whitlockite dispersion and the PCL polymer dispersion were put into a 10-L beaker. Then, the resulting product was stirred at room temperature for 30 minutes under conditions of an impeller and a sonicator at 200 RPM and 750 Watt, respectively, and then thoroughly mixed.

4) Drying of whitlockite-PCL polymer dispersion

The whitlockite-PCL polymer dispersion was thinly spread on a die, dried at 150°C for 2 hours and 80°C for 24 hours using a hot plate, and then left at room temperature for 24 hours.

Next, the dried whitlockite-PCL was ground using a grinder and then molded into a specific form.

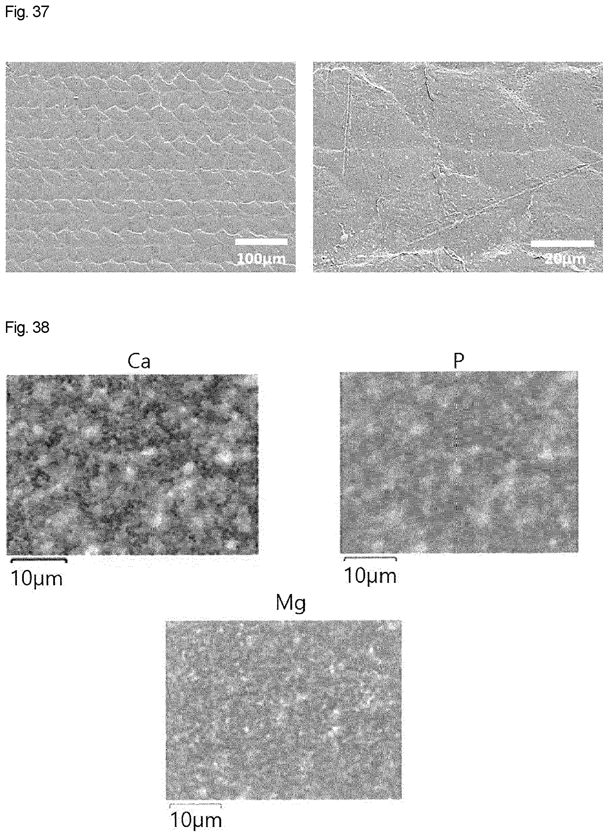

Referring to FIG. 37, FIG. 37 shows SEM images for the surface of the composite after preparing the biocompatible composite. FIG. 38 shows images obtained by EDS analysis performed on the surface of the composite. Specifically, FIG. 38 displays the portions occupied by each atom (calcium, phosphorus, and magnesium atoms) contained in the calcium phosphate particles.

Referring to FIGS. 37 and 38, it is confirmed that the biocompatible composite in which the whitlockite particles are dispersed in a PCL polymer matrix is enabled to be synthesized by the preparation method of the biocompatible composite according to one embodiment of the present application, which includes separately preparing the second whitlockite dispersion and the PCL polymer dispersion.

Hereinafter, comparative examples of biocompatible composites prepared by existing methods are to be described in detail.

**4. Comparative Example**

**(1) Comparative Example 1 (preparation example of comparative specimen No. 1)**

1) Preparation of PCL polymer dispersion

A PCL polymer dispersion in which PCL polymer particles were dispersed in an ethanol phase was prepared by putting 200 ml of 99.9% ethanol (EtOH) and 21 g of a bead-type PCL polymer into a reaction tank and then heating the resulting product to 80°C for about 1 hour while operating an impeller. In this case, the temperature of 80°C was selected as a temperature for partially melting the PCL polymer by transferring heat to the PCL polymer using ethanol as a heat medium.

2) Preparation of whitlockite powder

The first whitlockite dispersion prepared in Preparation Example 1 described above was dispensed into a weighing dish and dried at a temperature in a range of 60°C to 80°C for 24 hours. Then, the whitlockite particles were ground and sieved using a mortar and a 75-um sieve to prepare whitlockite powder.

3) Mixing of whitlockite powder and PCL polymer dispersion

After adding 9 g of the whitlockite powder to the reaction tank containing the PCL polymer dispersion, the whitlockite particles and the PCL polymer dispersion were mixed for 30 minutes while operating an impeller. As a result, a whitlockite-PCL polymer dispersion in which the whitlockite particles and the PCL polymer particles were dispersed in the ethanol phase was prepared. In addition, a portion of ethanol contained in the reaction tank was evaporated by being heated to 80°C while operating the impeller.
In this case, the mixing time was set to 30 minutes when the volume of ethanol contained in the reaction tank was based on 100 ml with respect to 30 g of a composite to be prepared.

4) Drying of whitlockite-PCL polymer dispersion

The whitlockite-PCL polymer dispersion was dried for 12 hours while applying heat thereto to 80°C using a dry oven to prepare a whitlockite-PCL polymer composite in a cubic form by a casting method. Then, the whitlockite-PCL polymer composite in the cubic form was cut to prepare a biocompatible composite in a pellet form.

5) Compression molding

The pellet formed biocompatible composites are poured into a mold, and the mold was heated to about 200°C. Thermal compression molding in which a pressure of about 30 MPa was applied to the biocompatible composite in the pellet form. Through the thermal compression molding, comparative specimen No. 1 in the plate form with a size of 40 * 25 (mm), to be used for SEM analysis, was prepared.

**(2) Comparative Example 2 (preparation example of comparative specimen No. 2)**

A 200-nm hydroxyapatite powder product purchased from Sigma Aldrich was prepared, and 10 g of 200-nm hydroxyapatite purchased from Sigma Aldrich was mixed with a PCL polymer dispersion prepared in the same manner as described above in Comparative Example 1. Then, comparative specimen No. 2 in a plate form with a size of 40 * 25 (mm), to be used for SEM analysis, was prepared through the drying and compression molding processes in the same manner as described above in Comparative Example 1.

**(3) Comparative Example 3 (preparation example of comparative specimen No. 3)**

1) Preparation of PCL polymer solution

A PCL polymer and chloroform were ground by putting 500 ml of 99.9% chloroform ($CHCl_3$) and a bead-type PCL polymer into a Duran bottle and then firstly stirring the resulting mixture for 30 minutes using an impeller at 200 rpm while using an ultrasonicator under the condition of 750 watts. Secondly, additional stirring was performed for 2 hours using an impeller at 200 rpm to prepare a PCL polymer solution in which PCL polymer particles were dissolved in chloroform.

2) Preparation of whitlockite powder

The first whitlockite dispersion prepared in Preparation Example 1 described above was dispensed into a weighing dish and dried at a temperature in a range of 60°C to 80°C for 24 hours. Then, the whitlockite particles were ground and sieved using a mortar and a 75-um sieve to prepare whitlockite powder. In other words, the whitlockite powder was prepared in the same manner as in Comparative Example 1.

3) Mixing of whitlockite powder and PCL polymer solution

The whitlockite powder was added to the Duran bottle containing the PCL polymer solution. Then, the resulting product in the Duran bottle was stirred for 30 minutes at 200 rpm using a magnetic bar to prepare a whitlockite-PCL polymer dispersion in which the whitlockite particles and the PCL polymer particles were dispersed in the chloroform phase. In addition, the whitlockite-PCL polymer mixture was mixed at 200 rpm while simultaneously applying heat thereto to 60°C.
In this case, the mixing time was set to 30 minutes when the volume of chloroform contained in the Duran bottle was based on 500 ml with respect to 70 g of a composite to be prepared.

4) Drying of whitlockite-PCL polymer dispersion

The whitlockite-PCL polymer dispersion was thinly spread on a die and dried at a temperature of 80°C for 24 hours to prepare a whitlockite-PCL polymer composite in a sheet form. Then, the whitlockite-PCL polymer composite in the sheet form was cut to prepare a biocompatible composite in a pellet form.

5) Compression molding

The pellet formed biocompatible composites are poured into a mold, and the mold was heated to about 200°C. Thermal compression molding in which a pressure of about 30 MPa was applied to the biocompatible composite in the pellet form. Through the thermal compression molding, comparative specimen No. 3 in a plate form with a size of 40 * 25 (mm), to be used for SEM analysis, was prepared.

**(4) Comparative Example 4 (preparation example of comparative specimen No. 4)**

A 200-nm hydroxyapatite powder product purchased from Sigma Aldrich was prepared, and 10 g of 200-nm hydroxyapatite purchased from Sigma Aldrich was mixed with a PCL polymer dispersion containing chloroform as the solvent, prepared in the same manner as in Comparative Example 3. Then, comparative specimen No. 4 in a plate form with a size of 40 * 25 (mm), to be used for SEM analysis, was prepared through the drying and compression molding processes in the same manner as in Comparative Example 3.

**5. Analysis method**

Hereinafter, an analysis method for quantitatively quantifying the degree of dispersion of calcium phosphate particles in a biodegradable polymer matrix will be described in detail with reference to FIGS. 17 to 20.
The degree of dispersion of the calcium phosphate particles of specimens Nos. 1 to 8 (hereinafter referred to as test specimens), prepared according to preparation examples described above, and comparative specimens Nos. 1 to 4 (hereinafter referred to as control specimens), prepared according to comparative examples, in each region in the biodegradable polymer matrix was quantified by analyzing the EDS images taken using Image J software.
Image J software, a Java-based image processing program developed at the National Institute of Health and the Laboratory for Optical and Computational Instrumentation, is being widely used for calculating area fractions in SEM images, EDS images, and the like.
However, the present disclosure is not limited thereto, and various methods may be used to quantify the degree of the dispersion of the calcium phosphate particles in each region in the biodegradable polymer matrix.
FIG. 17 illustrates one aspect of defining a cross section to be analyzed, using Image J software, for the test specimens prepared according to preparation examples described above and the control specimens prepared according to comparative examples described above.

Referring to FIG. 17, the thermal compression molding was performed by heating each of specimens Nos. 1 to 8 in the pellet form, prepared according to preparation examples described above, to about 200°C and applying a pressure of about 30 MPa, thereby preparing the test specimens. In addition, the thermal compression molding was performed by heating each of comparative specimens Nos. 1 to 4 in the pellet form, prepared according to comparative examples described above, to about 200°C and applying a pressure of about 30 MPa, thereby preparing the control specimens.

The test specimens and the control specimens in the plate form were cut into two halves by being cut in a cross section perpendicular to the longitudinal direction.

A polishing process to smooth each cross section was sequentially performed on the upper cross section (hereinafter referred to as a first cross section), the lower cross section (hereinafter referred to as a third cross section), and the intermediate cross section positioned between the first and third cross sections (hereinafter referred to as a second cross section) of the specimens in the plate form of the test specimens and the control specimens.

Then, SEM images were taken with respect to the first, second, and third cross sections of the test specimens and the control specimens.

In particular, for the convenience of SEM image analysis, each of the first, second, and third cross sections of the test specimens and the control specimens were divided into two areas, and SEM images were taken.

Referring back to FIG. 17, SEM images were taken from locations A and B in the first cross section, locations A and B in the second cross section, and locations A and B in the third cross section.

In addition, each cross section was taken at various magnifications to verify the accuracy and reproducibility of the experiment. Specifically, SEM images were taken by varying magnifications to 500x, 1000x, and 5000x, with respect to the test specimens and the control specimens.

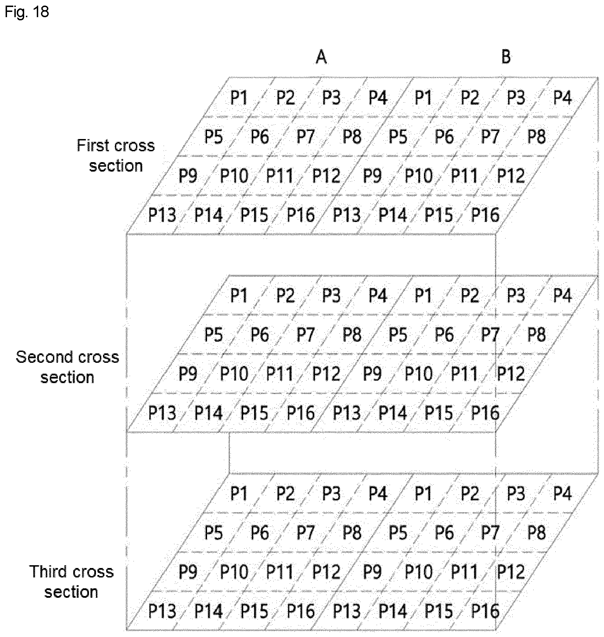

Referring to FIG. 18, FIG. 18 is a view illustrating one aspect of dividing an SEM image (EDS image) corresponding to each cross section into a plurality of regions using Image J software, for quantitative analysis of the test specimens and the control specimens.

For each of the test specimens and the control specimens, regions A and B of the first cross section, regions A and B of the second cross section, and regions A and B of the third cross section were each independently divided into 16 regions to perform quantitative analysis using Image J software.

Specifically, the quantitative analysis was performed on regions P1 to P16 of the SEM image (EDS image) for region A of the first cross section using Image J software. In addition, the quantitative analysis was performed on regions P1 to P16 of the SEM image (EDS image) for region B of the first cross section using Image J software.

In addition, the quantitative analysis was performed on regions P1 to P16 of the SEM image (EDS image) for region A of the second cross section using Image J software. In addition, the quantitative analysis was performed on regions P1 to P16 of the SEM image (EDS image) for region B of the second cross section using Image J software.

In addition, the quantitative analysis was performed on regions P1 to P16 of the SEM image (EDS image) for region A of the third cross section using Image J software. In addition, the quantitative analysis was performed on regions P1 to P16 of the SEM image (EDS image) for region B of the third cross section using Image J software.

In this case, for the accuracy and reproducibility of the analysis, each of specimens Nos. 1 to 8, according to preparation examples described above, and comparative specimens Nos. 1 to 4, according to comparative examples described above, was repeatedly prepared three times. Then, the quantitative analysis was performed.

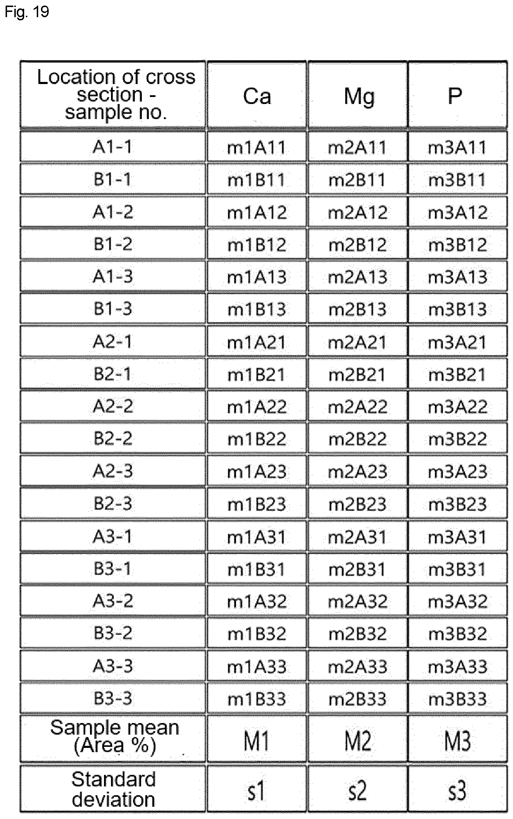

FIG. 19 is a table for explaining one aspect of calculating an average and a standard deviation of quantitative values measured by the analysis method using Image J software.

First, SEM images and EDS data were obtained for each cross section. The average and standard deviation of the ratio of the area occupied by each atom were calculated on the basis of the SEM images and EDS data for each cross section through the following processes.

Using Image J software, a ratio of an area occupied by each of the calcium, magnesium, and/or phosphorus atoms distributed in region P1 in region A of the first cross section was calculated. In other words, the ratio of the area occupied by each atom in region P1 with respect to the area of region P1 was calculated. In addition, for regions P2 to P16 in region A of the first cross section, ratios of areas occupied by the calcium, magnesium, and/or phosphorus atoms were calculated in the same manner.

Referring to FIG. 19, the average value (m1A11) of the quantitative values regarding the "calcium atoms" distributed in region A of the first cross section was calculated on the basis of the quantitative values of areas P1 to P16 related to region A of the first cross section. Specifically, the m1A11 value is an average value of the ratios of the areas occupied by the calcium atoms in region Pi (i = 1 to 16) with respect to the area of region Pi included in region A of the first cross section.

In addition, the average value (m2A1 1) of the quantitative values regarding the "magnesium atoms" distributed in region A of the first cross section was calculated on the basis of the quantitative values of areas P1 to P16 in region A of the first cross section. Specifically, the m2A11 value is an average value of the ratios (i = 1 to 16) of the areas occupied by the magnesium atoms in region Pi with respect to the area of region Pi in region A of the first cross section.

In addition, the average value (m3A1 1) of the quantitative values regarding the "phosphorus atoms" distributed in region A of the first cross section was calculated on the basis of the quantitative values of areas P1 to P16 in region A of the first cross section. Specifically, the m3A11 value is an average value of the ratios (i = 1 to 16) of the areas occupied by the phosphorus atoms in region Pi with respect to the area of region Pi in region A of the first cross section.

Similarly, the average value (m1B11) of the quantitative values regarding the "calcium atoms" distributed in "region B" of the first cross section was calculated. In addition, the average value (m2B11) of the quantitative values regarding the "magnesium atoms" distributed in "region B" of the first cross section was calculated. Furthermore, the average value (m3B11) of the quantitative values regarding the "phosphorus atoms" distributed in the "region B" of the first cross section was calculated.

Similarly, for regions A and B of the "second cross section", the average values (m1A21 and m1B21) of the quantitative values regarding the calcium atoms, the average values (m2A21 and m2B21) of the quantitative values regarding the magnesium atoms, or the average values (m3A21 and m3B21) of the quantitative values regarding the phosphorus atoms were calculated.

Similarly, for regions A and B of the "third cross section", the average values (m1A31 and m1B31) of the quantitative values regarding the calcium atoms, the average values (m2A31 and m2B31) of the quantitative values regarding the magnesium atoms, or the average values (m3A31 and m3B31) of the quantitative values regarding the phosphorus atoms were calculated.

In this case, for the accuracy and reproducibility of the analysis, each of specimens Nos. 1 to 8, according to preparation examples described above, and comparative specimens Nos. 1 to 4, according to comparative examples described above, was repeatedly prepared three times, as described above. Then, the calculation process described above was performed. Tests Nos. 1, 2, and 3 in the table of FIG. 19 indicate the first, second, and third measurements among three measurements, respectively. Through this, each of the average values of the quantitative values regarding the calcium atom (m1A11, m1B11, m1A21, m1B21, m1A31, m1B31, m1A12, m1B12, m1A22, m1B22, m1A32, m1B32, m1A13, m1B13, m1A23, m1B23, m1A33, and m1B33), the average values of the quantitative values regarding the magnesium atom (m2A11, m2B11, m2A21, m2B21, m2A31, m2B31, m2A12, m2B12, m2A22, m2B22, m2A32, m2B32, m2A13, m2B13, m2A23, m2B23, m2A33, m2B33), and the average values of the quantitative values regarding the phosphorus (m3A11, m3B11, m3A21, m3B21, m3A31, m3B31, m3A12, m3B12, m3A22, m3B22, m3A32, m3B32, m3A13, m3B13, m3A23, m3B23, m3A33, m3B33) were calculated.

In addition, for each of the test specimens and the control specimens, sample means and standard deviations of the average values of the quantitative values of the calcium, magnesium, and/or phosphorus atoms (that is, the ratios of the areas occupied by each elemental atom with respect to the total area of each region Pi) were calculated.

Specifically, regarding the calcium atoms, the sample mean (M1) of the average values of the quantitative values of the calcium atoms distributed in the first, second, and third cross sections was calculated by the following equation.

$$M1 = \frac{\sum_{i=1,2,3}^{j=1,2,3}(m1Aij + m1Bij)}{18}$$

In addition, regarding the calcium atoms, the standard deviation (s1) was calculated based on the sample mean (M1) of the average values of the quantitative values of the calcium atoms distributed in the first, second, and third cross sections.

Similarly, regarding the magnesium atom, the sample mean (M2) of the average values of quantitative values of the magnesium atom distributed in the first, second, and third cross sections was calculated.

$$M2 = \frac{\sum_{i=1,2,3}^{j=1,2,3}(m2Aij + m2Bij)}{18}$$

In addition, regarding the magnesium atoms, the standard deviation (s2) was calculated based on the sample mean (M2) of the average values of the quantitative values of the magnesium atoms distributed in the first, second, and third cross sections.

Regarding the phosphorus atoms, the sample mean (M3) of the average values of quantitative values of the phosphorus atoms distributed in the first, second, and third cross sections was calculated in the same manner as above.

$$M3 = \frac{\sum_{i=1,2,3}^{j=1,2,3}(m3Aij + m3Bij)}{18}$$

In addition, regarding the phosphorus atoms, the standard deviation (s3) was calculated based on the sample mean (M3) of the average values of the quantitative values of the phosphorus atoms distributed in the first, second, and third cross sections.

In this case, it may mean that the smaller the calculated standard deviation is, the more uniformly the calcium phosphate particles are distributed in the biodegradable polymer matrix including the first, second, and third cross sections.

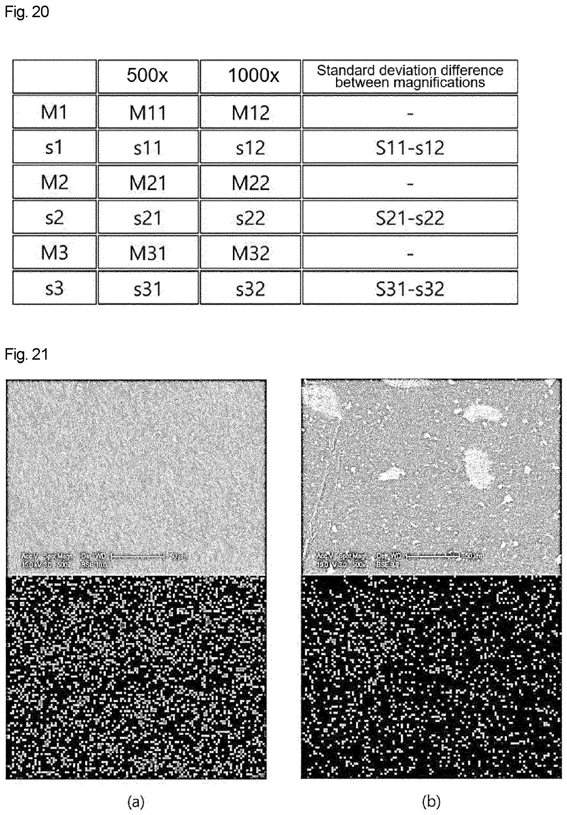

FIG. 20 is a table for explaining one aspect of calculating the average and standard deviation of the quantitative values measured according to the analysis method using Image J software.

As described above, SEM images were taken while varying magnifications to 500x, 1000x, and 5000x, with respect to each of the test specimens and the control specimens. In this case, quantitative data were calculated as a result of performing the analysis method described above on the SEM images with varying magnifications (50x and 1000x) for each of the test specimens and the control specimens.

Specifically, referring to FIG. 20, the SEM images taken at 500x magnification for each cross section were analyzed to obtain the average value (M11) and the standard deviation (s11) related to the ratio of the area occupied by the calcium atoms in each cross section. In addition, the SEM images taken at 1000x for each cross section in the same specimens were analyzed to obtain the average value (M12) and the standard deviation (s12) related to the ratio of the area occupied by the calcium atoms in each cross section.

The SEM images taken at 500x magnification for each cross section were similarly analyzed to obtain the average value (M21) and the standard deviation (s21) related to the ratio of the area occupied by the magnesium atoms in each cross section. In addition, the SEM images taken at 1000x for each cross section in the same specimens were analyzed to obtain the average value (M22) and the standard deviation (s22) related to the ratio of the area occupied by the magnesium atoms in each cross section.

The SEM images taken at 500x magnification for each cross section were similarly analyzed to obtain the average value (M31) and the standard deviation (s31) related to the ratio of the area occupied by the phosphorus atoms in each cross section. In addition, the SEM images taken at 1000x for each cross section in the same specimens were analyzed to obtain the average value (M32) and the standard deviation (s32) related to the ratio of the area occupied by the phosphorus atoms in each cross section.

In addition, a difference between the standard deviation calculated from the SEM image taken at 500x magnification for each cross section and the standard deviation calculated from the SEM image taken at 1 000x magnification for each cross section was calculated.

Specifically, regarding the calcium atoms, a difference (s11 - s12) between the standard deviation (s11), calculated from the SEM image taken at 500x magnification for each cross section, and the standard deviation (s12), calculated from the SEM image taken at 1000x magnification for each cross section, was calculated.

Regarding the magnesium atoms, a difference (s21 - s22) between the standard deviation (s21), calculated from the SEM image taken at 500x magnification for each cross section, and the standard deviation (s22), calculated from the SEM image taken at 1000x magnification for each cross section, was similarly calculated.

Similarly, regarding the phosphorus atoms, a difference (s31 - s32) between the standard deviation (s31), calculated from the SEM image taken at 500x magnification for each cross section, and the standard deviation (s32), calculated from the SEM image taken at 1000x magnification for each cross section, was calculated.

In this case, it may mean that the closer the difference in the standard deviation, calculated at each magnification, is to 0, the more uniformly the calcium phosphate is distributed in the biodegradable polymer matrix.

Hereinafter, referring to FIGS. 21 to 36, the quality of the biocompatible composites prepared by preparation examples, and the biocompatible composites, prepared by comparative examples are compared and analyzed (for example, for an uniformity in the degree of the dispersion of the calcium phosphate particles dispersed in each region in the biodegradable polymer). Specifically, the biocompatible composites, prepared according to preparation examples, and the biocompatible composite, prepared according to comparative examples, are compared and analyzed on the basis of the quantitative data on each of the specimens and the comparative specimens, calculated using the analysis method described above.

## 6. Comparative analysis result

FIGS. 21 to 23 are referred,
FIG. 21A shows an SEM image of specimen No. 1 prepared according to Preparation Example 1 and an image displaying the portion occupied by the magnesium atoms in specimen No. 1. FIG. 21B shows an SEM image of comparative specimen No. 1 prepared according to Comparative Example 1 and an image displaying the portion occupied by the magnesium atoms in comparative specimen No. 1.

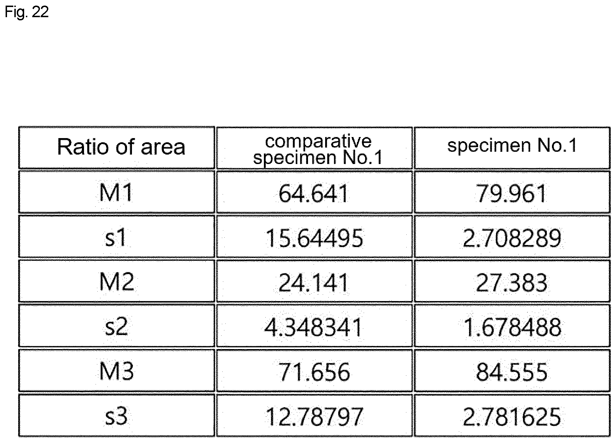

FIG. 22 is a comparison table of the quantitative data on specimen No. 1 and the quantitative data on comparative specimen No. 1, according to the analysis method described above.

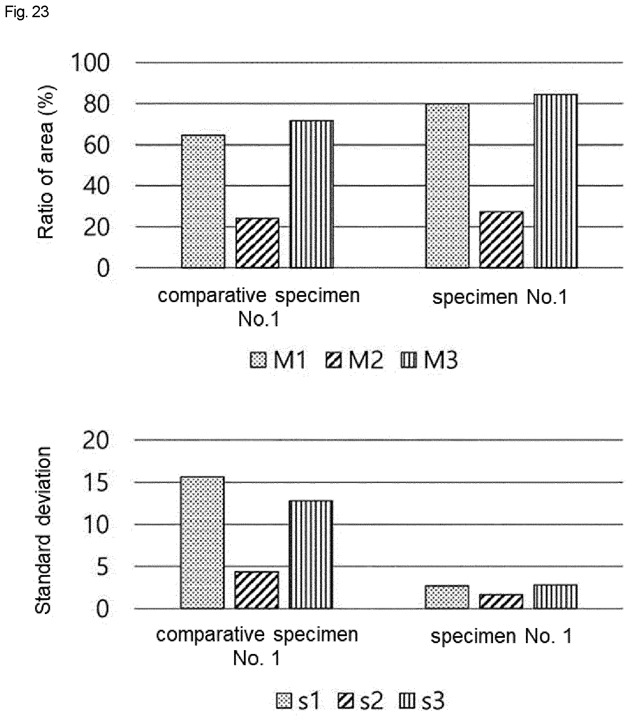

FIG. 23 shows graphs comparing the quantitative data on specimen No. 1 and the quantitative data on comparative specimen No. 1, according to the analysis method described above.

As described above, specimen No. 1 was prepared by selecting the method of mixing the whitlockite dispersion, containing ethanol as the liquid continuous phase with the PCL polymer dispersion, containing ethanol as the liquid continuous phase. Comparative specimen No. 1 was prepared by the method of mixing the whitlockite "powder" with the PCL polymer dispersion, containing ethanol as the liquid continuous phase.

In this case, for the specimen No. 1 and the comparative specimen No. 1, the SEM images (and EDS images for the respective atoms) were measured according to the analysis method described above. Then, the quantitative data were calculated using Image J software.

Referring to FIGS. 21A and 21B, it is visually confirmed that the degree of the dispersion of the whitlockite particles in the PCL polymer in the case of specimen No. 1 is more uniform than that in the case of comparative specimen No. 1. In particular, in the case of comparative specimen No. 1, it is confirmed that the size of the whitlockite particles is irregular and the size of empty spaces between the whitlockite particles is relatively large. On the other hand, in the case of specimen No. 1, it is confirmed that the size of the whitlockite particles is regular and the size of empty spaces between the whitlockite particles is relatively small.

Referring to FIGS. 22 and 23, the quantitative data for the calcium, magnesium, and phosphorus atoms were calculated using Image J software. Both specimen No. 1 and comparative specimen No. 1 are the composites containing the whitlockite particles, which contain calcium, magnesium, and phosphorus atoms. For this reason, quantitative data for all the calcium, magnesium, and phosphorus atoms were measured.

In the case of specimen No. 1, the average (M1) of the degree of the dispersion of the calcium atoms in the cross section of the PCL polymer matrix was calculated to be 79.961. In addition, the average (M2) of the degree of the dispersion of the magnesium atoms in the cross section of the PCL polymer matrix was calculated to be 27.383. Furthermore, the average (M3) of the degree of the dispersion of the phosphorus atoms in the cross section of the PCL polymer matrix was calculated to be 84.555. In addition, in the case of specimen No. 1, the standard deviation (s1) of the calcium atoms was calculated to be 2.708289, the standard deviation (s2) of the magnesium atoms was calculated to be 1.678488, and the standard deviation (s3) of the phosphorus atoms was calculated to be 2.781625.

On the other hand, in the case of comparative specimen No. 1, the average (M1) of the degree of the dispersion of the calcium atoms in the cross section of the PCL polymer matrix was calculated to be 64.641. In addition, the average (M2) of the degree of the dispersion of the magnesium atoms in the cross section of the PCL polymer matrix was calculated to be 24.141. Furthermore, the average (M3) of the degree of the dispersion of the phosphorus atoms in the cross section of the PCL polymer matrix was calculated to be 71.656. In addition, in the case of comparative specimen No. 1, the standard deviation (s1) of the calcium atom was calculated to be 15.64495, the standard deviation (s2) of the magnesium atom was calculated to be 4.348341, and the standard deviation (s3) of the phosphorus atom was calculated to be 12.78797.

In this case, it is confirmed that the average values of the calcium, magnesium, and phosphorus atoms are relatively larger in the case of specimen No. 1 than in the case of comparative specimen No. 1. This may be because, in the case of comparative specimen No. 1, aggregation occurred among the whitlockite particles, resulting in the dispersion of whitlockite particles with irregular sizes in the PCL polymer matrix. In other words, the whitlockite particles are locally dispersed while being relatively more aggregated, thereby forming relatively larger empty spaces between the whitlockite particles. For this reason, the average values of the calcium, magnesium, and phosphorus atoms may be calculated to be relatively small in the case of comparative specimen No. 1.

In addition, it is confirmed that the standard deviations of the calcium, magnesium, and phosphorus atoms are relatively smaller in the case of specimen No. 1 than in the case of comparative specimen No. 1. Thus, in the case of specimen No. 1, that is, in the case of the biocompatible composite according to the present disclosure, it was proved that the degree of the dispersion of the whitlockite in the PCL polymer matrix was relatively uniform.

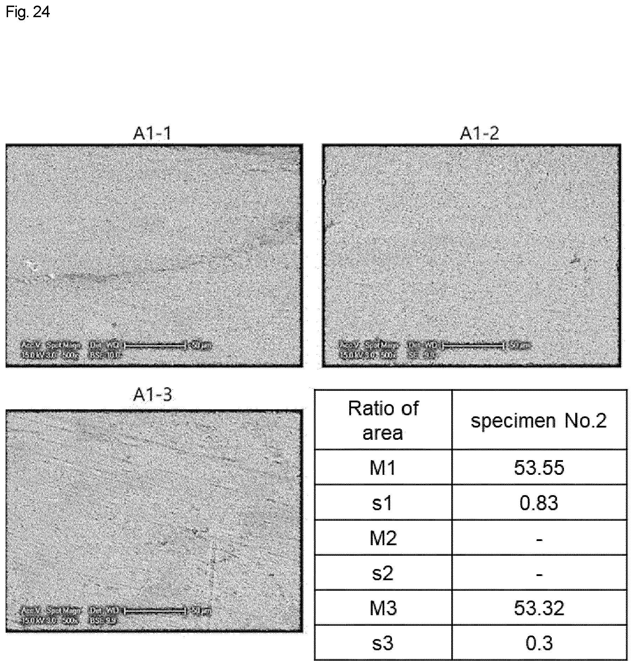

FIGS. 24 to 26 are referred.

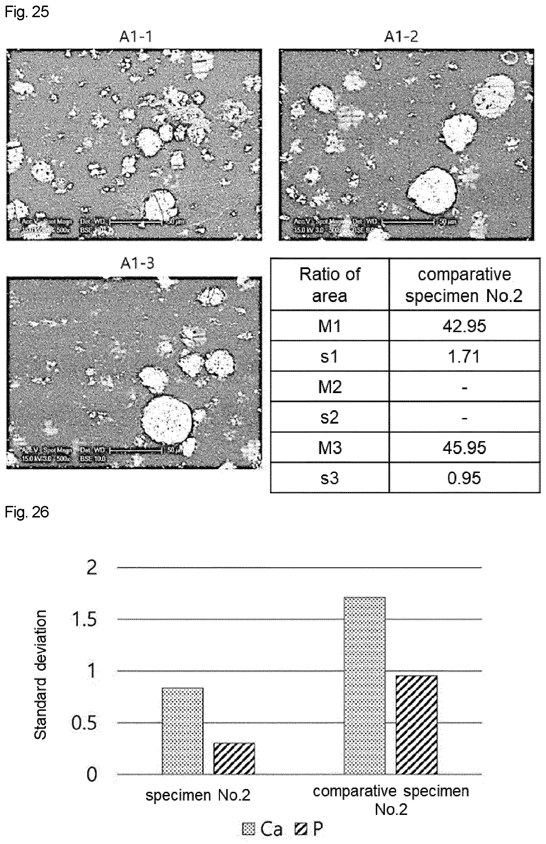

FIG. 24 shows SEM images of specimen No. 2 prepared according to Preparation Example 2 and the quantitative data for each atom, the data derived by analyzing EDS data for each atom with the Image J program. FIG. 25 shows SEM images of comparative specimen No. 2 prepared according to Comparative Example 2 and the quantitative data for each atom, the data derived by analyzing EDS data for each atom with the Image J program.

FIG. 26 shows graphs comparing the quantitative data on specimen No. 2 and the quantitative data on comparative specimen No. 2, according to the analysis method described above.

As described above, specimen No. 2 was prepared by selecting the method of mixing the hydroxyapatite dispersion, containing ethanol as the liquid continuous phase, with the PCL polymer dispersion, containing ethanol as the liquid continuous phase. Comparative specimen No. 2 was prepared by the method of mixing the hydroxyapatite powder with the PCL polymer dispersion containing ethanol as the liquid continuous phase.

In this case, the SEM images were measured according to the analysis method described above for specimen No. 2 and comparative specimen No. 2. Then, the quantitative data were calculated using Image J software.

Referring to the SEM images of FIGS. 24 and 25, it is visually confirmed that the hydroxyapatite particles are relatively more uniformly distributed in the PCL polymer matrix in the case of specimen No. 2 than in the case of comparative specimen No. 2. In particular, in the case of comparative specimen No. 2, it is confirmed that the size of the hydroxyapatite particles is irregular and the size of empty spaces between the hydroxyapatite particles is relatively large. On the other hand, in the case of specimen No. 2, it is confirmed that the size of the hydroxyapatite particles is regular and the size of empty spaces between the hydroxyapatite particles is relatively small.

Referring to FIGS. 24 to 26, the quantitative data for the calcium and phosphorus atoms were calculated using Image J software. Both specimen No. 2 and comparative specimen No. 2 are the composites containing the hydroxyapatite particles, which contain calcium and phosphorus atoms but do not contain magnesium atoms. For this reason, quantitative data for magnesium atoms were not measured.

In the case of specimen No. 2, the average (M1) of the degree of the dispersion of the calcium atoms in the cross section of the PCL polymer matrix was calculated to be 53.55. In addition, the average (M3) of the degree of the dispersion of the phosphorus atoms in the cross section of the PCL polymer matrix was calculated to be 53.32. Furthermore, in the case of specimen No. 2, the standard deviation (s1) of the calcium atoms was calculated to be 0.83, and the standard deviation (s3) of the phosphorus atoms was calculated to be 0.3.

On the other hand, in the case of comparative specimen No. 2, the average (M1) of the degree of the dispersion of the calcium atoms in the cross section of the PCL polymer matrix was calculated to be 42.95. In addition, the average (M3) of the degree of the dispersion of the phosphorus atoms in the cross section of the PCL polymer matrix was calculated to be 45.95. Furthermore, in the case of comparative specimen No. 2, the standard deviation (s1) of the calcium atoms was calculated to be 1.71, and the standard deviation (s3) of the phosphorus atoms was calculated to be 0.95.

In this case, it is confirmed that the average values of the calcium and phosphorus atoms are relatively larger in the case of specimen No. 2 than in the case of comparative specimen No. 2. This may be because, in the case of comparative specimen No. 2, aggregation occurred between the hydroxyapatite particles, resulting in the dispersion of the hydroxyapatite particles with irregular size in the PCL polymer matrix. In other words, the hydroxyapatite particles are locally dispersed while being relatively more aggregated, thereby forming relatively larger empty spaces between the hydroxyapatite particles. As a result, the average values of the calcium and phosphorus atoms may be calculated to be relatively small in the case of comparative specimen No. 2.

In addition, it is confirmed that standard deviations of calcium and phosphorus atoms are relatively small in the case of specimen No. 2 compared to the case of comparative specimen No. 2. Thus, in the case of specimen No. 2, that is, in the case of the biocompatible composite according to the present disclosure, it was proved that the degree of the dispersion of the hydroxyapatite particles in the PCL polymer matrix was relatively uniform.

In addition, through the comparative analysis, it is confirmed that both the whitlockite particles and hydroxyapatite particles described above have similar tendencies.

Through this, it was proved that the preparation method of the biocompatible composite, according to one embodiment of the present application, was enabled to be applied to both the whitlockite particles and hydroxyapatite particles. In addition, it is presumed that the preparation method of the biocompatible composite, according to one embodiment of the present application, may be applied to calcium phosphate particles having similar properties (for example, beta-TCP), in addition to the whitlockite particles and hydroxyapatite particles, thereby preparing biocompatible composites.

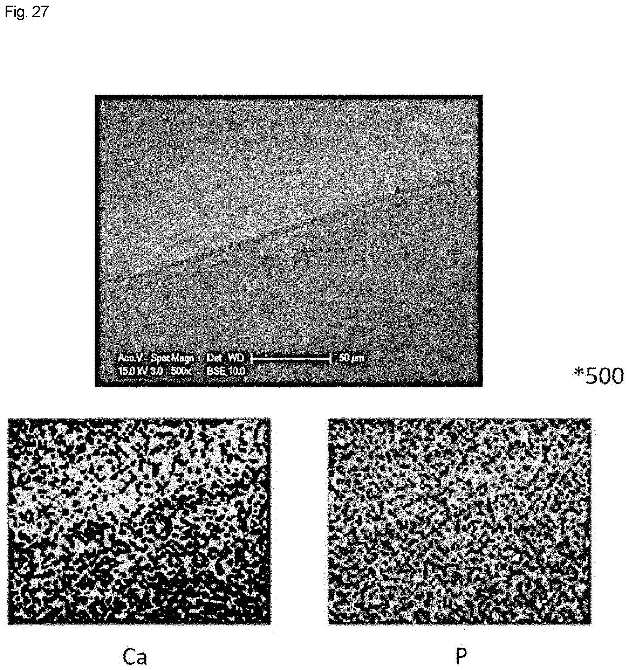

FIGS. 27 to 32 are referred.

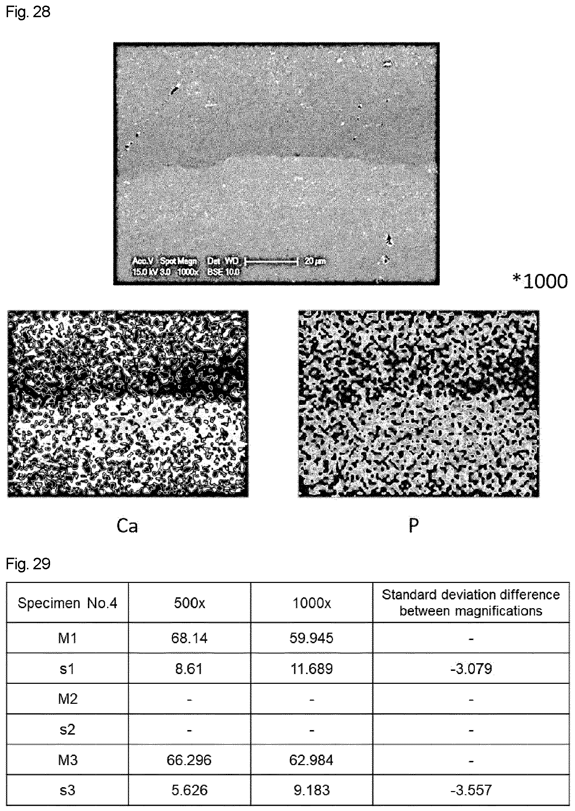

FIGS. 27 and 28 show SEM images of specimen No. 4 prepared according to Preparation Example 4 and EDS data for each atom in specimen No. 4. Specifically, FIG. 27 shows data taken at 500x magnification, and FIG. 28 shows data taken at 1000x magnification. FIG. 29 shows quantitative data for each atom, which is derived by analyzing the EDS data for each atom with the Image J program.

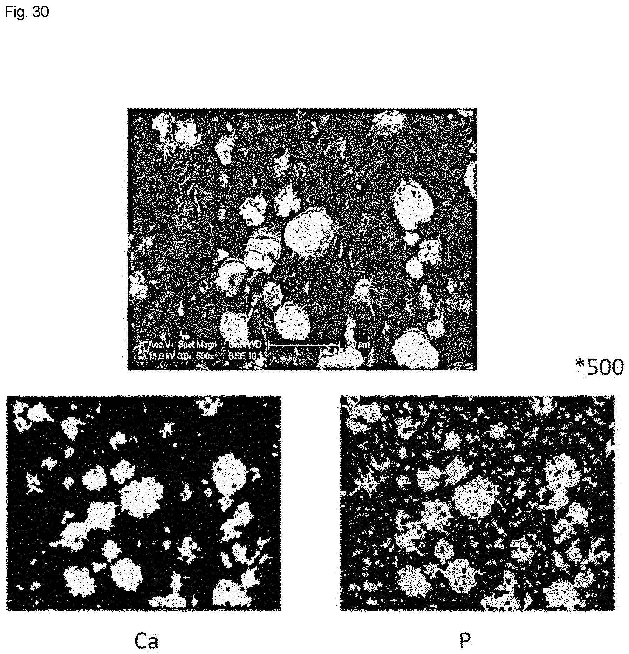

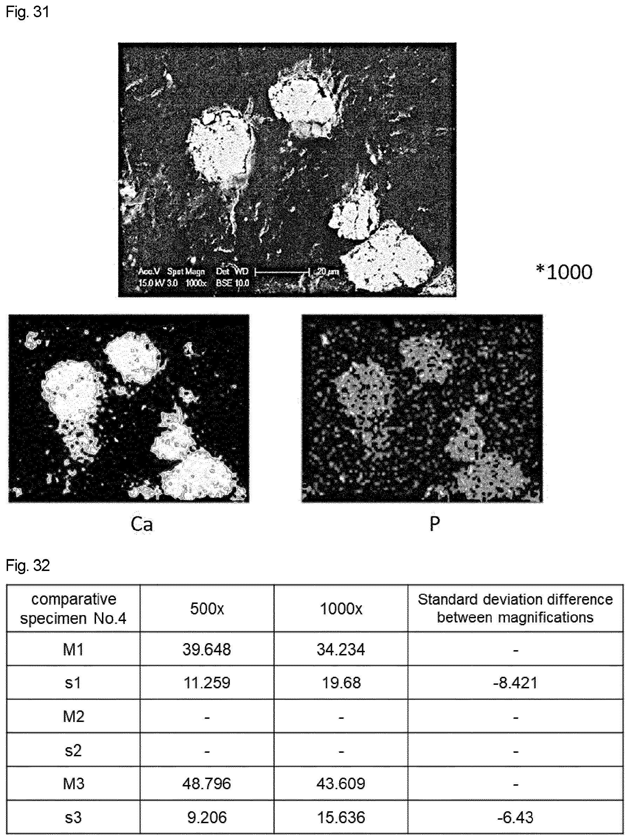

FIGS. 30 and 31 show SEM images of comparative specimen No. 4 prepared according to Comparative Example 4 and EDS data for each atom in comparative specimen No. 4. Specifically, FIG. 30 shows data taken at 500x magnification, and FIG. 31 shows data taken at 1000x magnification. FIG. 32 shows quantitative data for each atom, which is derived by analyzing the EDS data for each atom with the Image J program.

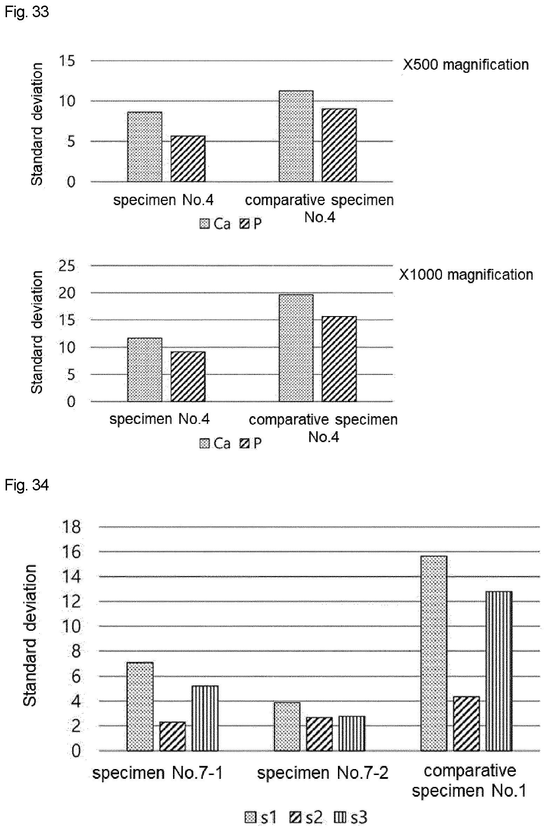

FIG. 33 shows graphs comparing the quantitative data on specimen No. 4 and the quantitative data on comparative specimen No. 4, according to the analysis method described above.

As described above, specimen No. 4 was prepared by the method of mixing the hydroxyapatite dispersion, containing ethanol as the liquid continuous phase, with the PCL polymer solution, containing a chloroform as a solvent. On the other hand, comparative specimen No. 4 was prepared by the method of mixing the hydroxyapatite powder with the PCL polymer solution, containing chloroform as the solvent.

In this case, for specimen No. 4 and comparative specimen No. 4, the SEM images were measured according to the analysis method described above. Then, the quantitative data were calculated using Image J software.

Referring to the SEM images (and EDS images) of FIGS. 27 to 32, it is visually confirmed that the hydroxyapatite particles are relatively more uniformly distributed in the PCL polymer matrix in the case of specimen No. 4 than in the case of comparative specimen No. 4. In particular, in the case of comparative specimen No. 4, it is confirmed that the size of the hydroxyapatite particles is irregular and the size of empty spaces between the hydroxyapatite particles is relatively large. On the other hand, in the case of specimen No. 4, it is confirmed that the size of the hydroxyapatite particles is regular and the size of empty spaces between the hydroxyapatite particles is relatively small.

Referring to FIGS. 29 and 32, the quantitative data for the calcium and phosphorus atoms were calculated on the basis of the EDS data taken from each specimen using Image J software. Both specimen No. 4 and comparative specimen No. 4 are the composites containing the hydroxyapatite particles, which contain calcium and phosphorus atoms but do not contain magnesium atoms. For this reason, quantitative data for magnesium atoms were not measured.

On the other hand, regarding FIGS. 17 to 20, in the comparative analysis, quantitative analysis was performed by measuring the cross section of each specimen while varying magnifications to 500x and 1000x using the analysis method described above.

When analyzing each cross section of specimen No. 4 at 500x magnification, the average (M1) of the degree of the dispersion of the calcium atoms in the cross section of the PCL polymer matrix was calculated to be 68.14. In addition, the average (M3) of the degree of the dispersion of the phosphorus atoms in the cross section of the PCL polymer matrix was calculated to be 66.296. Furthermore, the standard deviation (s1) of the calcium atoms was calculated to be 8.61, and the standard deviation (s3) of the phosphorus atoms was calculated to be 5.626.

On the other hand, when analyzing each cross section of comparative specimen No. 4 at 500x magnification, the average (M1) of the degree of the dispersion of the calcium atoms in the cross section of the PCL polymer matrix was calculated to be 39.648. In addition, the average (M3) of the degree of the dispersion of the phosphorus atoms in the cross section of the PCL polymer matrix was calculated to be 48.796. Furthermore, the standard deviation (s1) of the calcium atoms was calculated to be 11.259, and the standard deviation (s3) of the phosphorus atoms was calculated to be 9.206.

On the other hand, when analyzing each cross section of specimen No. 4 at 1000x magnification, the average (M1) of the degree of the dispersion of the calcium atoms in the cross section of the PCL polymer matrix was calculated to be 59.945. In addition, the average (M3) of the degree of the dispersion of the phosphorus atoms in the cross section of the PCL polymer matrix was calculated to be 62.984. Furthermore, the standard deviation (s1) of the calcium atoms was calculated to be 11.689, and the standard deviation (s3) of the phosphorus atoms was calculated to be 9.183.

On the other hand, when analyzing each cross section of comparative specimen No. 4 at 1000x magnification, the average (M1) of the degree of the dispersion of the calcium atoms in the cross section of the PCL polymer matrix was calculated to be 34.234. In addition, the average (M3) of the degree of the dispersion of the phosphorus atoms in the cross section of the PCL polymer matrix was calculated to be 43.609. Furthermore, the standard deviation (s1) of the calcium atoms was calculated to be 19.68, and the standard deviation (s3) of the phosphorus atoms was calculated to be 15.636.

Accordingly, differences in the standard deviations of the calcium atoms and phosphate atoms, calculated from specimen No. 4 taken at 500x and 1000x magnifications for each cross section, were calculated to be -3.079 for the calcium atoms and -3.557 for the phosphate atoms.

On the other hand, differences in the standard deviations of the calcium atoms and phosphate atoms, calculated from comparative specimen No. 4 taken at 500x and 1 000x magnifications for each cross section, were calculated to be -8.421 for the calcium atoms and -6.43 for the phosphate atoms.

In this case, under the same analysis conditions (that is, the same magnification), it is confirmed that the average values of the calcium atoms and phosphorus atoms are relatively larger in specimen No. 4 than in comparative specimen No. 4. This may be because, in the case of comparative specimen No. 4, aggregation of the powder-form hydroxyapatite particles is occurred, resulting in dispersion of the hydroxyapatite particles with irregular size in the PCL polymer matrix. In other words, the hydroxyapatite particles are locally dispersed while being relatively more aggregated, thereby forming relatively larger empty spaces between the hydroxyapatite particles. As a result, the average values of the calcium and phosphorus atoms may be calculated to be relatively small in the case of comparative specimen No. 4.

In addition, it is confirmed that all the standard deviations of the calcium and phosphorus atoms calculated from the SEM images taken at 500x and 1000x magnifications are relatively small in the case of specimen No. 4, compared to the case of comparative specimen No. 4.

In addition, it was confirmed that the differences in the standard deviations of the calcium and phosphorus atoms calculated from specimen No. 4 taken at 500x and 1000x magnifications for each cross section were closer to 0 than those calculated from comparative specimen No. 4.

Thus, in the case of specimen No. 4, that is, in the case of the biocompatible composite according to the present disclosure, it was proved that the degree of the dispersion of the hydroxyapatite particles in the PCL polymer matrix was relatively uniform.

In addition, it is confirmed through the comparative analysis that the quantitative data have similar tendencies for both cases where the liquid continuous phase (or solvent) of the PCL polymer dispersion or the like is ethanol or chloroform.

Through this, it was proved that the preparation method of the biocompatible composite, according to one embodiment of the present application, was enabled to be applied not only to the case of using the PCL polymer dispersion containing ethanol as the liquid continuous phase, but also to the case of using the PCL polymer soluti on containing chloroform as the solvent. In addition, it was proved that the biocompatible composite, prepared by the preparation method, was also characterized in that the calcium phosphate particles were relatively more uniformly distributed in the PCL polymer matrix.

In addition, referring to Preparation Example 6 together, the biocompatible composite may be prepared by the preparation method, according to one embodiment, using the biodegradable polymer solution containing DCM as the solvent. In this case, chloroform and DCM are organic solvents having similar properties. Therefore, when using biodegradable polymer dispersions or biodegradable polymer solutions containing not only chloroform and DCM but also organic solvents having similar properties (for example, tetrahydrofuran (THF), dimethylformamide (DMF), dimethyl sulfoxide (DMSO), acetone, and the like) as the liquid continuous phase or solvent, the preparation method of the biocompatible composite, according to one embodiment of the present application, may be applied. As a result, it is presumed that a composite in which calcium phosphate particles are relatively uniformly distributed in a biodegradable polymer may be prepared.

FIG. 34 is a graph comparing quantitative data on specimen No. 7-1, specimen No. 7-2, and comparative specimen No. 1 analyzed using the analysis method described above. On the other hand, specimens Nos. 7-1 and 7-2 were prepared on the basis of Preparation Example 7 described above.

As described above, specimen No. 7-1 was prepared such that the weight ratio of the whitlockite particles to the entire biocompatible composite was 10 wt%. In addition, specimen No. 7-2 was prepared such that the weight ratio of the whitlockite particles to the entire biocompatible composite was 50 wt%.

In particular, specimens Nos. 7-1 and 7-2 were prepared by the method of mixing the whitlockite dispersion, containing ethanol as the liquid continuous phase, with the PCL polymer dispersion, containing ethanol as the liquid continuous phase.

On the other hand, comparative specimen No. 1 was prepared by the method of mixing the whitlockite powder with the PCL biodegradable polymer dispersion, containing ethanol as the liquid continuous phase.

In addition, quantitative data for the calcium, magnesium, and phosphorus atoms were calculated by the analysis method described above.

Referring to FIG. 34, it is confirmed that the standard deviations of the calcium, magnesium, and phosphorus atoms are relatively smaller in the case of both specimens Nos. 7-1 and 7-2 than in the case of comparative specimen No. 1.

Therefore, in the case of specimens Nos. 7-1 and 7-2, that is, even in the case of preparing the biocompatible composite by the preparation method according to one embodiment while varying the weight ratio, it was proved that the whitlockite particles tended to be uniformly distributed in the PCL polymer matrix.

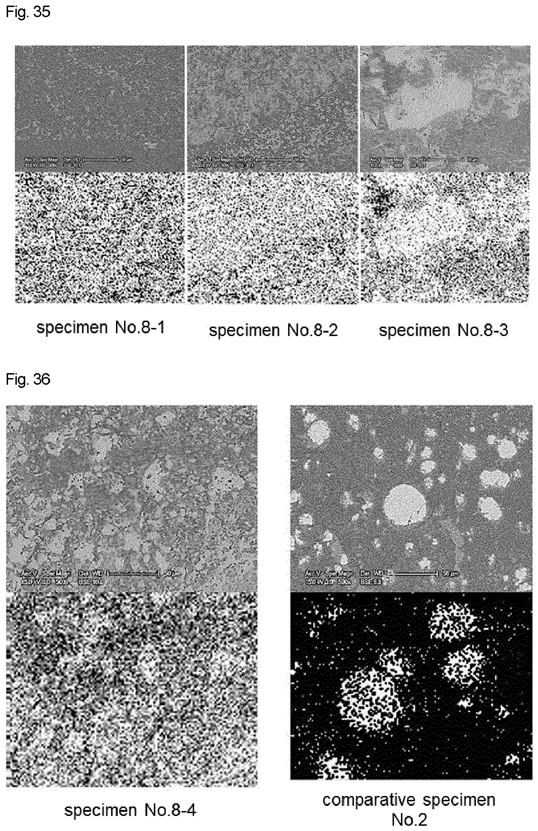

FIGS. 35 and 36 show SEM data and EDS data of specimens Nos. 8-1 to 8-4, prepared according to Preparation Example 8, and SEM images and EDS analysis images of comparative specimen No. 2 prepared according to Comparative Example 2.

As described above, specimen No. 8-1 was prepared such that the weight ratio of the hydroxyapatite particles to the entire biocompatible composite was 20 wt%, and specimen No. 8-2 was prepared such that the weight ratio of the hydroxyapatite particles to the entire biocompatible composite was 30 wt%. In addition, specimen No. 8-3 was prepared such that the weight ratio of the hydroxyapatite particles to the entire biocompatible composite was 40 wt%, and specimen No. 8-4 was prepared such that the weight ratio of the hydroxyapatite particles to the entire biocompatible composite was 50 wt%.

In particular, specimens Nos. 8-1 to 8-4 were prepared by the method of mixing the hydroxyapatite dispersion, containing ethanol as the liquid continuous phase, with the PCL polymer dispersion, containing ethanol as the liquid continuous phase.

On the other hand, comparative specimen No. 2 was prepared by the method of mixing the hydroxyapatite powder with the PCL polymer dispersion, containing ethanol as the liquid continuous phase.

Referring to FIGS. 35 and 36, in the case of comparative specimen No. 2, it is visually confirmed that there are relatively many empty spaces between the hydroxyapatite particles. In other words, it was confirmed that the hydroxyapatite particles were relatively non-uniformly distributed in the PCL polymer matrix.

On the other hand, although there are some empty spaces between the hydroxyapatite particles in the case of specimens Nos. 8-1 to 8-4, it is visually confirmed that the extent thereof is relatively less than that in the case of comparative specimen No. 2. In other words, in the case of specimens Nos. 8-1 to 8-4, it was confirmed that the hydroxyapatite particles were relatively uniformly distributed in the biodegradable polymer matrix, compared to the case of comparative specimen No. 2.

Through this, it was proved that the hydroxyapatite particles were enabled to be uniformly distributed in the PCL polymer matrix even when preparing the biocompatible composite by the preparation method according to one embodiment while varying the weight ratio.

In addition, when considering the comparative analysis results of the whitlockite particles with reference to FIG. 34 together, it is presumed that biocompatible composites exhibiting consistent uniformity may be prepared by the preparation method according to one embodiment disclosed herein even when varying the weight ratio of the calcium phosphate particles (for example, beta-TCP and the like) having similar properties, in addition to the whitlockite particles and hydroxyapatite particles.

According to the comparative analysis results described above, it is confirmed that, in the case of the test specimens, the calcium phosphate particles tend to be more uniformly distributed in the biodegradable polymer than in the case of the control specimens.

In particular, when analyzing the test specimens using Image J software, the ratio of the area occupied by the calcium atoms of the calcium phosphate particles per unit area fell within a range of 53% to 85%, and the standard deviation fell within a range of 0.8 to 8.7.

On the other hand, in the control specimens, for example, in the cross section of comparative specimen No. 1, the average value of the ratio of the area occupied by the calcium atoms of the calcium phosphate particles per unit area was calculated to be about 64.641%. In addition, the standard deviation of the average value thereof was calculated to be about 15.64495.

Through such comparative analysis results, it was also quantitatively proved that the calcium phosphate particles were more uniformly dispersed in the biodegradable polymer in the case of the test specimens than in the case of the control specimens.

As a result of such experiments, the test specimens and the biocompatible composite were prepared by mixing the calcium phosphate particles, contained in the dispersion rather than being present in the powder form, with the biodegradable polymer dispersion or the like, as described above. Therefore, there is no case of directly mixing the calcium phosphate particles with the biodegradable polymer in the powder form during the preparation process of the biocompatible composite. Accordingly, aggregation that occurs randomly between the calcium phosphate particles in the powder form may be prevented. As a result, a phenomenon in which the calcium phosphate particles have irregular sizes due to aggregation between the calcium phosphate particles may be minimized.

In addition, it is presumed that when mixing the calcium phosphate particles with the biodegradable polymer dispersion or the like, the effect of gravity, the interaction between the calcium phosphate particles, or the interaction between the calcium phosphate particles and the biodegradable polymer particles that randomly occur due to the irregular size of the calcium phosphate particles may be prevented.

Therefore, in the case of the test specimens, it is expected that the calcium phosphate particles are characterized by being uniformly distributed in the biodegradable polymer matrix due to the reasons described above.

On the other hand, in the control specimens prepared by the existing preparation method, the calcium phosphate powder was ground, and the ground calcium phosphate powder was then mixed with the biodegradable polymer dispersion. In this case, aggregation between the calcium phosphate particles inevitably and randomly occurs due to the nature of the powder. Accordingly, the irregularly sized calcium phosphate particles are dispersed in the biodegradable polymer matrix. When the irregularly sized calcium phosphate particles are dispersed in the biodegradable polymer matrix, the effect of gravity on the irregularly sized calcium phosphate particles may be different. Specifically, calcium phosphate particles having a large particle size due to the aggregation are relatively more affected by gravity and thus are more likely to be distributed in the downward direction of gravity in the biodegradable polymer matrix. On the other hand, calcium phosphate particles that have a relatively small particle size because aggregation does not occur are relatively less affected by gravity and thus are more likely to be dispersed on the opposite side of the gravitational direction of the biodegradable polymer matrix. Therefore, it is assumed that the control specimens prepared by the existing preparation method resulted in a relatively non-uniform degree of dispersion of the calcium phosphate in each region in the biodegradable polymer.

According to the comparative analysis results described above, it was proved that the biocompatible composite, prepared by the preparation method disclosed herein, was characterized in that the calcium phosphate particles were uniformly dispersed in the biodegradable polymer matrix.

Therefore, the biocompatible composite disclosed herein may obtain further consistent performance in each region in the biocompatible composite, such as mechanical strength and the like.

In addition, the biocompatible composite disclosed herein is characterized in that the calcium phosphate particles are uniformly dispersed in the biodegradable polymer matrix. For this reason, when applying the biocompatible composite material to a living tissue, the degree to which the calcium phosphate particles are released may tend to be uniform. In addition, the biocompatible composite disclosed herein may be characterized in that control of the calcium phosphate particles to be released at a targeted location in a desired amount is facilitated.

According to the comparative analysis results described above, it was confirmed that the test specimens were enabled to be prepared such that the calcium phosphate particles were contained in the biodegradable polymer in a higher weight ratio than that in the case of control specimens.

In addition, it was confirmed that the test specimens were enabled to be prepared such that the calcium phosphate particles were contained in the biodegradable polymer in a weight ratio in a wider range than that in the case of the control specimens.

In particular, according to the existing preparation method, when mixing the whitlockite powder with the PCL polymer in a weight ratio of about 50 wt%, the biocompatible polymer was not prepared because the whitlockite particles and the PCL polymer were insufficiently stirred.

On the other hand, when looking at the comparative analysis results of specimens Nos. 7-1 and 7-2 and comparative specimen No. 1, it was proved that the biocompatible composite, according to one embodiment of the present application, was prepared such that the calcium phosphate particles were contained in any weight ratio that falls within a range of 0.01 wt% or more and less than 55 wt% while being characterized in that the calcium phosphate particles were relatively uniform ly distributed in the biodegradable polymer matrix at the same time.

Therefore, the biocompatible composite disclosed herein is enabled to be prepared freely such that calcium phosphate is contained in an appropriate weight ratio depending on the use or properties of the biocompatible composite, which is advantageous.

According to the existing preparation method, calcium phosphate is mixed with the biodegradable polymer dispersion while being present in the "powder" form. In this case, the force with which the powder-form calcium phosphate aggregates to each other is relatively strong. For this reason, at least a portion of the calcium phosphate particles may be mixed with the biodegradable polymer dispersion while being aggregated. In addition, there is a tendency that the higher the weight ratio of the calcium phosphate particles, the stronger the force with which the calcium phosphate particles aggregate to each other. Therefore, it is presumed that the already aggregated calcium phosphate particles failed to be dispersed in the continuous phase (or solvent) of the dispersion or the like and were present while being aggregated to each other even in the dispersion or the like, so the calcium phosphate particles and the biodegradable polymer dispersion or the like were insufficiently mixed.

On the other hand, according to the preparation method of the present application, the calcium phosphate particles are mixed with the biodegradable polymer dispersion or the like while being present as the dispersed phase of the "dispersion". Therefore, the calcium phosphate particles are enabled to be mixed with the biodegradable polymer dispersion or the like while the force with which the powder-form calcium phosphate particles aggregate to each other is somewhat weakened. Therefore, according to the preparation method of the biocompatible composite disclosed herein, it is presumed that the calcium phosphate particles were smoothly mixed with the biodegradable polymer matrix even when preparing the biocompatible composite containing the calcium phosphate particles in a high weight ratio.

Referring to Preparation Examples 1 to 4, the preparation method of the biocompatible composite, according to one embodiment of the present application, may be applied to whitlockite and hydroxyapatite. In addition, it was confirmed that properties, such as weight ratio, uniformity in the degree of dispersion in the biodegradable polymer matrix, and the like showed similar tendencies in the case of using the whitlockite particles and the case of using the hydroxyapatite particles. Therefore, the preparation method of the biocompatible composite, according to one embodiment of the present application, may be applied to calcium phosphate particles, including $\beta$-tricalcium phosphate, having similar properties, other than whitlockite and hydroxyapatite.

Referring to Preparation Examples 5 and 6, the preparation method of the biocompatible composite, according to one embodiment of the present application, may be applied not only to the PCL polymer but also to the PLLA polymer. Therefore, the preparation method of the biocompatible composite, according to one embodiment of the present application, may be applied to biodegradable polymers, including PLGA polymers, having similar properties, other than the PCL polymer and the PLLA polymer.

Referring to Preparation Examples 1 to 8, in the preparation method of the biocompatible composite according to one embodiment of the present application, not only ethanol but also chloroform and DCM may be used as the continuous phase (or solvent) of the biodegradable polymer dispersion or the like.

Therefore, the preparation method of the biocompatible composite, according to one embodiment of the present application, may be implemented such that the biodegradable polymer dispersion is prepared using a material having properties similar to ethanol.

In particular, both chloroform and DCM, which are organic solvents, have similar properties. Therefore, the preparation method of the biocompatible composite, according to one embodiment of the present application, may be implemented such that the biodegradable polymer dispersion or the like is prepared using organic solvents having properties similar to chloroform and DCM (for example, tetrahydrofuran (THF), dimethylformamide (DMF), dimethyl sulfoxide (DMSO), acetone, and the like).

The features, structures, effects, and the like described in the embodiments above are included in at least one embodiment of the present disclosure and are not necessarily limited to one embodiment. Furthermore, the features, structures, effects, and the like provided in each embodiment may be combined or modified in other embodiments by those skilled in the art to which the embodiments belong. Therefore, contents related to the combination and modification should be construed to be included in the scope of the present disclosure.

Although the embodiments of the present disclosure were mainly described above, these embodiments were disclosed only for illustrative purposes and do not limit the present disclosure. Those skilled in the art to which the present disclosure belongs will appreciate that various modifications and applications are possible, without departing from the essential features of the present disclosure. That is, each component described in detail in the embodiments of the present disclosure may be modified. Furthermore, differences related to such modifications and applications should be construed as being included in the scope and spirit of the present disclosure as described in the appended claims.

## Claims

1. A method for preparing a biocompatible composite comprising:

    preparing a second calcium phosphate dispersion and a biodegradable polymer solution, respectively, wherein the biodegradable polymer solution is prepared by dissolving a biodegradable polymer in a solvent;
    preparing a calcium phosphate-biodegradable polymer dispersion by mixing the second calcium phosphate dispersion and the biodegradable polymer solution; and
    removing a liquid continuous phase of the calcium phosphate-biodegradable polymer dispersion.

2. The method of claim 1,
    wherein the second calcium phosphate dispersion is prepared by a method comprising:
    adding a calcium phosphate crystal to a second continuous phase.

3. The method of claim 2,
    wherein the calcium phosphate crystal is any one selected from a whitlockite crystal, a hydroxyapatite crystal, and a β-TCP crystal, or combination thereof.

4. The method of claim 2,
    wherein the calcium phosphate crystal is the whitlockite crystal.

5. The method of claim 2,
    wherein the calcium phosphate crystal is the hydroxyapatite crystal.

6. The method of claim 2,
    wherein the second continuous phase is selected through consideration of a solubility for the solvent of the biodegradable polymer solution.

7. The method of claim 2,
    wherein the second continuous phase is ethanol.

8. The method of claim 1,
    wherein the second calcium phosphate dispersion is prepared by a method comprising:

    adding a calcium phosphate crystal to a second continuous phase; and
    dispersing the calcium phosphate crystal added.

9. The method of claim 8,
    wherein dispersing the calcium phosphate crystal added to the second continuous phase is performed by one or more methods selected from sonication and stirring.

**10.** The method of claim 1,
wherein the second calcium phosphate dispersion is prepared by a method comprising:

preparing a first calcium phosphate dispersion; and
preparing the second calcium phosphate dispersion by substituting a continuous phase of the first calcium phosphate dispersion with a second continuous phase.

**11.** The method of claim 10,
wherein the first calcium phosphate dispersion is prepared by a method comprising:

preparing a calcium ion aqueous solution in which a calcium ion is dissolved; and
providing, while adding a phosphate ion to the calcium ion aqueous solution, a condition of pH and temperature at which a calcium phosphate particle can be generated, for a pre-determined time period,
whereby the first calcium phosphate dispersion in which water is a continuous phase and the calcium phosphate particle is a dispersed phase is prepared.

**12.** The method of claim 10,
wherein the substituting the continuous phase of the first calcium phosphate dispersion with the second continuous phase comprises:

adding the second continuous phase to the first calcium phosphate dispersion; and
removing the water by separating a layer of the water which is the continuous phase of the first calcium phosphate and a layer of the second continuous phase using centrifuge.

**13.** The method of claim 10,
wherein the second continuous phase is selected through consideration of solubility for the solvent of the biodegradable polymer solution.

**14.** The method of claim 10,
wherein the second continuous phase is ethanol.

**15.** The method of claim 1,
wherein the biodegradable polymer is at least one selected from PCL, PLA, and PLGA, or combination thereof.

**16.** The method of claim 1,
wherein the biodegradable polymer is PCL.

**17.** The method of claim 1,
wherein the solvent is at least one selected from chloroform and dichloromethane (DCM).

**18.** The method of claim 1,
wherein the solvent is chloroform.

**19.** The method of claim 1,
wherein a liquid continuous phase of the calcium phosphate-biodegradable polymer dispersion comprises a second continuous phase of the second calcium phosphate dispersion and the solvent of the polymer solution.

**20.** The method of claim 1,
wherein removing a liquid continuous phase of the calcium phosphate-biodegradable polymer dispersion comprises:
drying the calcium phosphate-biodegradable polymer dispersion at a drying temperature.

**21.** The method of claim 20,
wherein the drying temperature is determined by considering whether or not the biodegradable polymer is degraded and whether or not a calcium phosphate comprised in the calcium phosphate-biodegradable polymer dispersion is precipitated.

**22.** The method of claim 1,
wherein removing a liquid continuous phase of the calcium phosphate-biodegradable polymer dispersion comprises:

drying firstly the calcium phosphate-biodegradable polymer dispersion at a first temperature for a first time period; and

drying secondly the calcium phosphate-biodegradable polymer dispersion at a second temperature for a second time period.

23. The method of claim 22,
    wherein the first temperature and the first time period are determined by considering whether or not the biodegradable polymer is degraded and whether or not a calcium phosphate comprised in the calcium phosphate-biodegradable polymer dispersion is precipitated.

24. The method of claim 22,
    wherein the first temperature, the first time period, the second temperature, and the second time period are determined by considering whether or not the biodegradable polymer is degraded and whether or not a calcium phosphate comprised in the calcium phosphate-biodegradable polymer dispersion is precipitated.

25. The method of claim 1,
    wherein the method for preparing biocompatible composite further comprises forming the calcium phosphate-biodegradable polymer composite.

26. A biocompatible composite comprising:

    a biodegradable polymer matrix; and
    a calcium phosphate particle dispersed in the biodegradable polymer matrix with uniformity of a first degree of uniformity.

27. The biocompatible composite of claim 26,
    wherein the biodegradable polymer matrix is provided from any one or more of PCL, PLA, PLLA, and PLGA.

28. The biocompatible composite of claim 26,
    wherein the biodegradable polymer matrix is provided from PCL.

29. The biocompatible composite of claim 26,
    wherein the calcium phosphate particle is any one of a whitlockite particle, a hydroxyapatite particle, and a beta tricalcium phosphate particle, or a combination of two or more selected from the whitlockite particle, the hydroxyapatite particle, and the beta tricalcium phosphate particle.

30. The biocompatible composite of claim 26,
    wherein the calcium phosphate particle is a whitlockite particle.

31. The biocompatible composite of claim 26,
    wherein a weight ratio of the calcium phosphate particle to the biocompatible composite is 10 to 50wt%.

32. The biocompatible composite of claim 26,
    wherein the first degree of uniformity is measured by a method comprising:

    measuring first ratios with respect to a plurality of regions of the biocompatible composite,
    wherein each of the first ratios is a ratio of an area occupied by a first atom in each of the plurality of regions,
    wherein the first atom is any one selected from calcium atom, phosphorus atom, and magnesium atom;
    calculating standard deviation of the first ratios measured from the plurality of regions to obtain a first standard deviation; and
    obtaining the first uniformity based on the first standard deviation.

33. The biocompatible composite of claim 32,
    wherein the first atom is calcium atom.

34. The biocompatible composite of claim 32,
    wherein each region of the plurality of regions has a same size of reference area.

35. The biocompatible composite of claim 32,
wherein the first degree of uniformity is calculated by dividing the first standard deviation by an average of the plurality of first ratios measured from the plurality of regions.

36. The biocompatible composite of claim 26,
wherein the first degree of uniformity is 1 or less.

37. A biocompatible composite comprising:

a biodegradable polymer matrix; and
a calcium phosphate particle dispersed in the biodegradable polymer matrix,
wherein an average of a ratio of area occupied by a first atom included in the calcium phosphate particle per unit area is 53% to 85%, and the standard deviation is 0.8 to 8.7.

38. The biocompatible composite of claim 37,
wherein the first atom is any one selected from calcium atom, phosphorous atom, and magnesium atom.

39. The biocompatible composite of claim 37,
wherein the first atom is calcium atom.

40. The biocompatible composite of claim 37,
wherein a weight ratio of the calcium phosphate particle to the biocompatible composite is 10 to 50wt%.

41. The biocompatible composite of claim 37,
wherein the biodegradable polymer matrix is provided from any one or more of PCL, PLA, PLLA, and PLGA.

42. The biocompatible composite of claim 37,
wherein the biodegradable polymer matrix is provided from PCL.

43. The biocompatible composite of claim 37,
wherein the calcium phosphate particle is any one of a whitlockite particle, a hydroxyapatite particle, and a beta tricalcium phosphate particle, or a combination of two or more selected from the whitlockite particle, the hydroxyapatite particle, and the beta tricalcium phosphate particle.

44. The biocompatible composite of claim 37,
wherein the calcium phosphate particle is a whitlockite particle.

45. The biocompatible composite of claim 37,
wherein a weight ratio of the calcium phosphate particle to the biocompatible composite is 10 to 50wt%.

46. The biocompatible composite of claim 37,
wherein the ratio of area occupied by the first atom included in the calcium phosphate particle per unit area is calculated by using image J.

Fig. 1

```
┌─────────────────────────────────────────────────────────────┐
│          Milling calcium phosphate powders (S10)             │
└─────────────────────────────────────────────────────────────┘
                              │
                              ▼
┌─────────────────────────────────────────────────────────────┐
│   Preparing a calcium phosphate-biodegradable polymer         │
│   dispersion by mixing the calcium phosphate powders          │
│   and a biodegradable polymer dispersion (S20)                │
└─────────────────────────────────────────────────────────────┘
                              │
                              ▼
┌─────────────────────────────────────────────────────────────┐
│      Preparing a composite by drying the calcium              │
│   phosphate-biodegradable polymer dispersion and              │
│                  molding (S30)                                │
└─────────────────────────────────────────────────────────────┘
```

Fig. 2

(a)

(b)

Fig. 3

> Preparing separately a second calcium phosphate dispersion and a biodegradable polymer dispersion (S1000)

> Preparing a calcium phosphate-biodegradable polymer dispersion by mixing the second calcium phosphate dispersion and the biodegradable polymer dispersion (S2000)

> Removing a liquid continuous phase of the calcium phosphate-biodegradable polymer dispersion to prepare a biocompatible composite (S3000)

Fig. 4

Preparation of the second calcium phosphate dispersion

> Preparing a first calcium phosphate dispersion (S0101)

> Substituting a water which is a continuous phase of the first calcium phosphate dispersion with a second continuous phase (S0102)

Fig. 5

## Preparation of the second calcium phosphate dispersion

| Adding calcium phosphate crystals to a second continuous phase (S0201) |
|---|

Fig. 6

(a)

(b)

(c)

(d)

Fig. 7

(a)

(b)

(c)

(d)

Fig. 8

(a)

(b)

(c)

Fig. 9

Ca

Mg

P

Fig. 10

(a)

(b)

Ca

P

(c)

Fig. 11

(a)

(b)

Fig. 12

WH
+PCL(DCM)

(a)

(b)

WH
+PLA(DCM)

(c)

(d)

Fig. 13

(a)

(b)

(c)

(d)

Fig. 14

(a)

(b)

(c)

(d)

Fig. 15

specimen No.8-1          specimen No.8-2

Fig. 16

specimen No.8-3          specimen No.8-4

Fig. 17

Fig. 18

Fig. 19

| Location of cross section - sample no. | Ca | Mg | P |
|---|---|---|---|
| A1-1 | m1A11 | m2A11 | m3A11 |
| B1-1 | m1B11 | m2B11 | m3B11 |
| A1-2 | m1A12 | m2A12 | m3A12 |
| B1-2 | m1B12 | m2B12 | m3B12 |
| A1-3 | m1A13 | m2A13 | m3A13 |
| B1-3 | m1B13 | m2B13 | m3B13 |
| A2-1 | m1A21 | m2A21 | m3A21 |
| B2-1 | m1B21 | m2B21 | m3B21 |
| A2-2 | m1A22 | m2A22 | m3A22 |
| B2-2 | m1B22 | m2B22 | m3B22 |
| A2-3 | m1A23 | m2A23 | m3A23 |
| B2-3 | m1B23 | m2B23 | m3B23 |
| A3-1 | m1A31 | m2A31 | m3A31 |
| B3-1 | m1B31 | m2B31 | m3B31 |
| A3-2 | m1A32 | m2A32 | m3A32 |
| B3-2 | m1B32 | m2B32 | m3B32 |
| A3-3 | m1A33 | m2A33 | m3A33 |
| B3-3 | m1B33 | m2B33 | m3B33 |
| Sample mean (Area %) | M1 | M2 | M3 |
| Standard deviation | s1 | s2 | s3 |

Fig. 20

| | 500x | 1000x | Standard deviation difference between magnifications |
|---|---|---|---|
| M1 | M11 | M12 | - |
| s1 | s11 | s12 | S11-s12 |
| M2 | M21 | M22 | - |
| s2 | s21 | s22 | S21-s22 |
| M3 | M31 | M32 | - |
| s3 | s31 | s32 | S31-s32 |

Fig. 21

(a)                                    (b)

Fig. 22

| Ratio of area | comparative specimen No.1 | specimen No.1 |
|---|---|---|
| M1 | 64.641 | 79.961 |
| s1 | 15.64495 | 2.708289 |
| M2 | 24.141 | 27.383 |
| s2 | 4.348341 | 1.678488 |
| M3 | 71.656 | 84.555 |
| s3 | 12.78797 | 2.781625 |

Fig. 23

Fig. 24

A1-1

A1-2

A1-3

| Ratio of area | specimen No.2 |
|---|---|
| M1 | 53.55 |
| s1 | 0.83 |
| M2 | - |
| s2 | - |
| M3 | 53.32 |
| s3 | 0.3 |

Fig. 25

A1-1

A1-2

A1-3

| Ratio of area | comparative specimen No.2 |
|---|---|
| M1 | 42.95 |
| s1 | 1.71 |
| M2 | - |
| s2 | - |
| M3 | 45.95 |
| s3 | 0.95 |

Fig. 26

Fig. 27

*500

Ca                                    P

Fig. 28

*1000

Ca                              P

Fig. 29

| Specimen No.4 | 500x | 1000x | Standard deviation difference between magnifications |
|---|---|---|---|
| M1 | 68.14 | 59.945 | - |
| s1 | 8.61 | 11.689 | -3.079 |
| M2 | - | - | - |
| s2 | - | - | - |
| M3 | 66.296 | 62.984 | - |
| s3 | 5.626 | 9.183 | -3.557 |

Fig. 30

*500

Ca                                        P

Fig. 31

*1000

Ca          P

Fig. 32

| comparative specimen No.4 | 500x | 1000x | Standard deviation difference between magnifications |
|---|---|---|---|
| M1 | 39.648 | 34.234 | - |
| s1 | 11.259 | 19.68 | -8.421 |
| M2 | - | - | - |
| s2 | - | - | - |
| M3 | 48.796 | 43.609 | - |
| s3 | 9.206 | 15.636 | -6.43 |

Fig. 33

X500 magnification

X1000 magnification

Fig. 34

Fig. 35

specimen No.8-1       specimen No.8-2       specimen No.8-3

Fig. 36

specimen No.8-4              comparative specimen
No.2

Fig. 37

Fig. 38

Ca

10μm

P

10μm

Mg

10μm

# EP 4 309 685 A1

## INTERNATIONAL SEARCH REPORT

International application No.

**PCT/KR2022/003694**

### A. CLASSIFICATION OF SUBJECT MATTER

**A61L 27/46**(2006.01)i; **A61L 27/58**(2006.01)i; **A61F 2/28**(2006.01)i; **A61F 2/30**(2006.01)i

According to International Patent Classification (IPC) or to both national classification and IPC

### B. FIELDS SEARCHED

Minimum documentation searched (classification system followed by classification symbols)

A61L 27/46(2006.01); A61K 47/30(2006.01); A61K 9/34(2006.01); A61L 27/00(2006.01); A61L 27/12(2006.01);
A61L 27/40(2006.01); A61L 27/42(2006.01); A61L 27/50(2006.01); B01J 19/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인산칼슘(calcium phosphate), 하이드록시아파타이트(hydroxyapatite), 생분해성
고분자(biodegradable polymer), 폴리카프로락톤(polycaprolactone), 분산(dispersion), 에탄올(ethanol)

### C. DOCUMENTS CONSIDERED TO BE RELEVANT

| Category* | Citation of document, with indication, where appropriate, of the relevant passages | Relevant to claim No. |
|---|---|---|
| X | KR 10-2009-0104475 A (INJE UNIVERSITY INDUSTRY-ACADEMIC COOPERATION FOUNDATION et al.) 06 October 2009 (2009-10-06) See paragraphs [0053], [0055], [0061]-[0067], [0073], [0092]-[0095] and [0120]. | 1-9,15-45 |
| Y | | 10-14,46 |
| Y | JP 2005-103345 A (NEW RAIMU KENKYUSHA:KK) 21 April 2005 (2005-04-21) See paragraphs [0013], [0020], [0024] and [0031]; and claim 1. | 10-14 |
| Y | KR 10-2015-0119527 A (THE CATHOLIC UNIVERSITY OF KOREA INDUSTRY-ACADEMIC COOPERATION FOUNDATION) 26 October 2015 (2015-10-26) See paragraph [0062]. | 46 |
| X | KR 10-0429937 B1 (TAKIRON CO., LTD.) 02 August 2004 (2004-08-02) See paragraphs [0164], [0165], [0300], [0517]-[0519], [0630] and [0631]; and claims 20, 22 and 28. | 1-9,15-21,25-45 |

☑ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **20 June 2022** | **20 June 2022** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**EP 4 309 685 A1**

**C.     DOCUMENTS CONSIDERED TO BE RELEVANT**

| Category* | Citation of document, with indication, where appropriate, of the relevant passages | Relevant to claim No. |
|---|---|---|
| A | KR 10-2007-0010919 A (OSCOTEC INC.) 24 January 2007 (2007-01-24)<br>See entire document. | 1-46 |

Form PCT/ISA/210 (second sheet) (July 2019)

**INTERNATIONAL SEARCH REPORT**
**Information on patent family members**

International application No.

**PCT/KR2022/003694**

| Patent document cited in search report | | | Publication date (day/month/year) | Patent family member(s) | | | Publication date (day/month/year) |
|---|---|---|---|---|---|---|---|
| KR | 10-2009-0104475 | A | 06 October 2009 | None | | | |
| JP | 2005-103345 | A | 21 April 2005 | JP | 4439229 | B2 | 24 March 2010 |
| KR | 10-2015-0119527 | A | 26 October 2015 | KR | 10-1609047 | B1 | 05 April 2016 |
| KR | 10-0429937 | B1 | 02 August 2004 | AT | 242646 | T | 15 June 2003 |
| | | | | CA | 2205231 | A1 | 20 March 1997 |
| | | | | CA | 2205231 | C | 18 March 2008 |
| | | | | CN | 1168105 | A | 17 December 1997 |
| | | | | CN | 1301756 | C | 28 February 2007 |
| | | | | DE | 69628632 | T2 | 29 April 2004 |
| | | | | EP | 0795336 | A1 | 17 September 1997 |
| | | | | EP | 0795336 | A4 | 20 December 2000 |
| | | | | EP | 0795336 | B1 | 11 June 2003 |
| | | | | JP | 2002-325832 | A | 12 November 2002 |
| | | | | JP | 3482991 | B2 | 06 January 2004 |
| | | | | JP | 3633909 | B2 | 30 March 2005 |
| | | | | US | 5981619 | A | 09 November 1999 |
| | | | | WO | 97-10010 | A1 | 20 March 1997 |
| KR | 10-2007-0010919 | A | 24 January 2007 | KR | 10-0807108 | B1 | 26 February 2008 |
| | | | | WO | 2007-011172 | A1 | 25 January 2007 |

Form PCT/ISA/210 (patent family annex) (July 2019)

**REFERENCES CITED IN THE DESCRIPTION**

**Patent documents cited in the description**

- KR 2084896

- KR 2116206